# EUROPEAN PATENT SPECIFICATION

(11) **EP 1 760 087 B1**
(45) Date of publication and mention of the grant of the patent: **21.04.2010**
(21) Application number: 05748510.4
(22) Date of filing: 02.06.2005
(51) Int. Cl.: C07K 14/46, A61K 39/395, A61K 45/00, A61P 25/00, A61P 35/00, A61P 43/00, C07K 16/18, C07K 19/00, C12N 15/09, G01N 33/15, G01N 33/50, G01N 33/53

(54) **NOVEL PROTEIN COMPLEX AND USE THEREOF**
NEUER PROTEINKOMPLEX UND ANWENDUNGEN DAVON
NOUVEAU COMPLEXE PROTEINIQUE ET UTILISATION DE CE COMPLEXE

(30) Priority: 03.06.2004 JP 2004165407
(43) Date of publication of application: 07.03.2007
(73) Proprietor: Takeda Pharmaceutical Company Limited, Osaka-shi, Osaka 5408645 (JP)
(72) Inventor: SUNAHARA, Eiji, TAKEDA PHARMACEUTICAL COMPANY LTD., Osaka-shi, Osaka 5328686 (JP); ISHII, Takafumi, TAKEDA PHARMACEUTICAL CO. LTD., Osaka-shi, Osaka 5328686 (JP)
(74) Representative: Keller, Günter
(86) International application number: PCT/JP2005/010538
(87) International publication number: WO 2005/118631

(56) References cited:
- WO-A1-2004/058817
- WO-A2-02/46465
- SCHULTZE WIEBKE ET AL: "Semaphorin4F interacts with the synapse-associated protein SAP90/PSD-95" JOURNAL OF NEUROCHEMISTRY, vol. 78, no. 3, August 2001 (2001-08), pages 482-489, XP002434351 ISSN: 0022-3042
- DATABASE UniProt [Online] 27 April 2001 (2001-04-27), "Semaphorin-4B precursor." XP002434355 retrieved from EBI accession no. UNIPROT:Q9NPR2 Database accession no. Q9NPR2
- INAGAKI S. ET AL: 'Sema4c, a transmembrane semaphorin, interacts with a post-synaptic density protein, PSD-95.' J.BIOL CHEM. vol. 276, no. 12, 2001, pages 9174 - 9181, XP002991765
- LIM I.A. ET AL: 'Selectivity and promiscuity of the first and second PDZ domains of PSD-95 and synapse-associated protein 102.' J BIOL CHEM. vol. 277, no. 24, 2002, pages 21697 - 21698, XP002991766
- CLARK H.F. ET AL: 'The secreted protein discovery initiative (SPDI), a large-scale effort to identify novel human secreted and transmembrane proteins: a bioinformatics assessment.' GENOME RES. vol. 13, no. 10, 2003, pages 2265 - 2270, XP001189293
- BURKHARDT C. ET AL: 'Semaphorin 4B interacts with the post-synaptic density protein PSD-95/SAP90 and is recruited to synapses through a C-terminal PDZ-binding motif.' FEBS LETT. vol. 579, no. 17, 2005, pages 3821 - 3828, XP004961978

### SEQUENCE LISTING

<110> Takeda Pharmaceutical Company Limited
<120> Novel protein complex and use thereof
<130> G06-0071
<150> JP 2004-165407
   <151> 2004-06-03
<160> 30
<210> 1
   <211> 837
   <212> PRT
   <213> Homo sapiens
<400> 1
   Met Leu Arg Thr Ala Met Gly Leu Arg Ser Trp Leu Ala Ala Pro Trp
                    5                  10                  15
   Gly Ala Leu Pro Pro Arg Pro Pro Leu Leu Leu Leu Leu Leu Leu Leu
               20                  25                  30
   Leu Leu Leu Gln Pro Pro Pro Pro Thr Trp Ala Leu Ser Pro Arg Ile
           35                  40                  45
   Ser Leu Pro Leu Gly Ser Glu Glu Arg Pro Phe Leu Arg Phe Glu Ala
       50                  55                  60
   Glu His Ile Ser Asn Tyr Thr Ala Leu Leu Leu Ser Arg Asp Gly Arg
   65                  70                  75                  80
   Thr Leu Tyr Val Gly Ala Arg Glu Ala Leu Phe Ala Leu Ser Ser Asn
                   85                  90                  95
   Leu Ser Phe Leu Pro Gly Gly Glu Tyr Gln Glu Leu Leu Trp Gly Ala
               100                 105                 110
   Asp Ala Glu Lys Lys Gln Gln Cys Ser Phe Lys Gly Lys Asp Pro Gln
           115                 120                 125
   Arg Asp Cys Gln Asn Tyr Ile Lys Ile Leu Leu Pro Leu Ser Gly Ser
       130                 135                 140
   His Leu Phe Thr Cys Gly Thr Ala Ala Phe Ser Pro Met Cys Thr Tyr
   145                 150                 155                 160
   Ile Asn Met Glu Asn Phe Thr Leu Ala Arg Asp Glu Lys Gly Asn Val
                   165                 170                 175
   Leu Leu Glu Asp Gly Lys Gly Arg Cys Pro Phe Asp Pro Asn Phe Lys
               180                 185                 190
   Ser Thr Ala Leu Val Val Asp Gly Glu Leu Tyr Thr Gly Thr Val Ser
           195                 200                 205
   Ser Phe Gln Gly Asn Asp Pro Ala Ile Ser Arg Ser Gln Ser Leu Arg
       210                 215                 220
   Pro Thr Lys Thr Glu Ser Ser Leu Asn Trp Leu Gln Asp Pro Ala Phe
   225 230 235 240
   Val Ala Ser Ala Tyr Ile Pro Glu Ser Leu Gly Ser Leu Gln Gly Asp
   245 250 255
   Asp Asp Lys Ile Tyr Phe Phe Phe Ser Glu Thr Gly Gln Glu Phe Glu
   260 265 270
   Phe Phe Glu Asn Thr Ile Val Ser Arg Ile Ala Arg Ile Cys Lys Gly
   275 280 285
   Asp Glu Gly Gly Glu Arg Val Leu Gln Gln Arg Trp Thr Ser Phe Leu
   290 295 300
   Lys Ala Gln Leu Leu Cys Ser Arg Pro Asp Asp Gly Phe Pro Phe Asn
   305 310 315 320
   Val Leu Gln Asp Val Phe Thr Leu Ser Pro Ser Pro Gln Asp Trp Arg
   325 330 335
   Asp Thr Leu Phe Tyr Gly Val Phe Thr Ser Gln Trp His Arg Gly Thr
   340 345 350
   Thr Glu Gly Ser Ala Val Cys Val Phe Thr Met Lys Asp Val Gln Arg
   355 360 365
   Val Phe Ser Gly Leu Tyr Lys Glu Val Asn Arg Glu Thr Gln Gln Met
   370 375 380
   Val His Arg Asp Pro Pro Val Pro Thr Pro Arg Pro Gly Ala Cys Ile
   385 390 395 400
   Thr Asn Ser Ala Arg Glu Arg Lys Ile Asn Ser Ser Leu Gln Leu Pro
   405 410 415
   Asp Arg Val Leu Asn Phe Leu Lys Asp His Phe Leu Met Asp Gly Gln
   420 425 430
   Val Arg Ser Arg Met Leu Leu Leu Gln Pro Gln Ala Arg Tyr Gln Arg
   435 440 445
   Val Ala Val His Arg Val Pro Gly Leu His His Thr Tyr Asp Val Leu
   450 455 460
   Phe Leu Gly Thr Gly Asp Gly Arg Leu His Lys Ala Val Ser Val Gly
   465 470 475 480
   Pro Arg Val His Ile Ile Glu Glu Leu Gln Ile Phe Ser Ser Gly Gln
   485 490 495
   Pro Val Gln Asn Leu Leu Leu Asp Thr His Arg Gly Leu Leu Tyr Ala
   500 505 510
   Ala Ser His Ser Gly Val Val Gln Val Pro Met Ala Asn Cys Ser Leu
   515 520 525
   Tyr Arg Ser Cys Gly Asp Cys Leu Leu Ala Arg Asp Pro Tyr Cys Ala
   530 535 540
   Trp Ser Gly Ser Ser Cys Lys His Val Ser Leu Tyr Gln Pro Gln Leu
   545 550 555 560
   Ala Thr Arg Pro Trp Ile Gln Asp Ile Glu Gly Ala Ser Ala Lys Asp
   565 570 575
   Leu Cys Ser Ala Ser Ser Val Val Ser Pro Ser Phe Val Pro Thr Gly
   580 585 590
   Glu Lys Pro Cys Glu Gln Val Gln Phe Gln Pro Asn Thr Val Asn Thr
               595                 600                 605
   Leu Ala Cys Pro Leu Leu Ser Asn Leu Ala Thr Arg Leu Trp Leu Arg
           610                 615                 620
   Asn Gly Ala Pro Val Asn Ala Ser Ala Ser Cys His Val Leu Pro Thr
   625                 630                 635                 640
   Gly Asp Leu Leu Leu Val Gly Thr Gln Gln Leu Gly Glu Phe Gln Cys
                   645                 650                 655
   Trp Ser Leu Glu Glu Gly Phe Gln Gln Leu Val Ala Ser Tyr Cys Pro
               660                 665                 670
   Glu Val Val Glu Asp Gly Val Ala Asp Gln Thr Asp Glu Gly Gly Ser
           675                 680                 685
   Val Pro Val Ile Ile Ser Thr Ser Arg Val Ser Ala Pro Ala Gly Gly
       690                 695                 700
   Lys Ala Ser Trp Gly Ala Asp Arg Ser Tyr Trp Lys Glu Phe Leu Val
   705                 710                 715                 720
   Met Cys Thr Leu Phe Val Leu Ala Val Leu Leu Pro Val Leu Phe Leu
                   725                 730                 735
   Leu Tyr Arg His Arg Asn Ser Met Lys Val Phe Leu Lys Gln Gly Glu
               740                 745                 750
   Cys Ala Ser Val His Pro Lys Thr Cys Pro Val Val Leu Pro Pro Glu
           755                 760                 765
   Thr Arg Pro Leu Asn Gly Leu Gly Pro Pro Ser Thr Pro Leu Asp His
       770                 775                 780
   Arg Gly Tyr Gln Ser Leu Ser Asp Ser Pro Pro Gly Ser Arg Val Phe
   785                 790                 795                 800
   Thr Glu Ser Glu Lys Arg Pro Leu Ser Ile Gln Asp Ser Phe Val Glu
                   805                 810                 815
   Val Ser Pro Val Cys Pro Arg Pro Arg Val Arg Leu Gly Ser Glu Ile
               820                 825                 830
   Arg Asp Ser Val Val
           835
<210> 2
   <211> 2511
   <212> DNA
   <213> Homo sapiens
<400> 2
   atgctgcgca ccgcgatggg cctgaggagc tggctcgccg ccccatgggg cgcgctgccg   60
   cctcggccac cgctgctgct gctcctgctg ctgctgctcc tgctgcagcc gccgcctccg  120
   acctgggcgc tcagcccccg gatcagcctg cctctgggct ctgaagagcg gccattcctc  180
   agattcgaag ctgaacacat ctccaactac acagcccttc tgctgagcag ggatggcagg  240
   accctgtacg tgggtgctcg agaggccctc tttgcactca gtagcaacct cagcttcctg  300
   ccaggcgggg agtaccagga gctgctttgg ggtgcagacg cagagaagaa acagcagtgc  360
   agcttcaagg gcaaggaccc acagcgcgac tgtcaaaact acatcaagat cctcctgccg  420
   ctcagcggca gtcacctgtt cacctgtggc acagcagcct tcagccccat gtgtacctac  480
   atcaacatgg agaacttcac cctggcaagg gacgagaagg ggaatgtcct cctggaagat  540
   ggcaagggcc gttgtccctt cgacccgaat ttcaagtcca ctgccctggt ggttgatggc  600
   gagctctaca ctggaacagt cagcagcttc caagggaatg acccggccat ctcgcggagc  660
   caaagccttc gccccaccaa gaccgagagc tccctcaact ggctgcaaga cccagctttt  720
   gtggcctcag cctacattcc tgagagcctg ggcagcttgc aaggcgatga tgacaagatc  780
   tactttttct tcagcgagac tggccaggaa tttgagttct ttgagaacac cattgtgtcc  840
   cgcattgccc gcatctgcaa gggcgatgag ggtggagagc gggtgctaca gcagcgctgg  900
   acctccttcc tcaaggccca gctgctgtgc tcacggcccg acgatggctt ccccttcaac  960
   gtgctgcagg atgtcttcac gctgagcccc agcccccagg actggcgtga cacccttttc 1020
   tatggggtct tcacttccca gtggcacagg ggaactacag aaggctctgc cgtctgtgtc 1080
   ttcacaatga aggatgtgca gagagtcttc agcggcctct acaaggaggt gaaccgtgag 1140
   acacagcaga tggtacaccg tgacccaccc gtgcccacac cccggcctgg agcgtgcatc 1200
   accaacagtg cccgggaaag gaagatcaac tcatccctgc agctcccaga ccgcgtgctg 1260
   aactttctca aggaccactt cctgatggac gggcaggtcc gaagccgcat gctgctgctg 1320
   cagccccagg ctcgctacca gcgcgtggct gtacaccgcg tccctggcct gcaccacacc 1380
   tacgatgtcc tcttcctggg cactggtgac ggccggctcc acaaggcagt gagcgtgggc 1440
   ccccgggtgc acatcattga ggagctgcag atcttctcat cgggacagcc cgtgcagaat 1500
   ctgctcctgg acacccacag ggggctgctg tatgcggcct cacactcggg cgtagtccag 1560
   gtgcccatgg ccaactgcag cctgtaccgg agctgtgggg actgcctcct cgcccgggac 1620
   ccctactgtg cttggagcgg ctccagctgc aagcacgtca gcctctacca gcctcagctg 1680
   gccaccaggc cgtggatcca ggacatcgag ggagccagcg ccaaggacct ttgcagcgcg 1740
   tcttcggttg tgtccccgtc ttttgtacca acaggggaga agccatgtga gcaagtccag 1800
   ttccagccca acacagtgaa cactttggcc tgcccgctcc tctccaacct ggcgacccga 1860
   ctctggctac gcaacggggc ccccgtcaat gcctcggcct cctgccacgt gctacccact 1920
   ggggacctgc tgctggtggg cacccaacag ctgggggagt tccagtgctg gtcactagag 1980
   gagggcttcc agcagctggt agccagctac tgcccagagg tggtggagga cggggtggca 2040
   gaccaaacag atgagggtgg cagtgtaccc gtcattatca gcacatcgcg tgtgagtgca 2100
   ccagctggtg gcaaggccag ctggggtgca gacaggtcct actggaagga gttcctggtg 2160
   atgtgcacgc tctttgtgct ggccgtgctg ctcccagttt tattcttgct ctaccggcac 2220
   cggaacagca tgaaagtctt cctgaagcag ggggaatgtg ccagcgtgca ccccaagacc 2280
   tgccctgtgg tgctgccccc tgagacccgc ccactcaacg gcctagggcc ccctagcacc 2340
   ccactcgatc accgagggta ccagtccctg tcagacagcc ccccggggtc ccgagtcttc 2400
   actgagtcag agaagaggcc actcagcatc caagacagct tcgtggaggt atccccagtg 2460
   tgcccccggc cccgggtccg ccttggctcg gagatccgtg actctgtggt g          2511
<210> 3
   <211> 3766
   <212> DNA
   <213> Homo sapiens
<400> 3
<210> 4
   <211> 837
   <212> PRT
   <213> Homo sapiens
<400> 4
   Met Leu Arg Thr Ala Met Gly Leu Arg Ser Trp Leu Ala Ala Pro Trp
   5 10 15
   Gly Ala Leu Pro Pro Arg Pro Pro Leu Leu Leu Leu Leu Leu Leu Leu
   20 25 30
   Leu Leu Leu Gln Pro Pro Pro Pro Thr Trp Ala Leu Ser Pro Arg Ile
   35 40 45
   Ser Leu Pro Leu Gly Ser Glu Glu Arg Pro Phe Leu Arg Phe Glu Ala
   50 55 60
   Glu His Ile Ser Asn Tyr Thr Ala Leu Leu Leu Ser Arg Asp Gly Arg
   65 70 75 80
   Thr Leu Tyr Val Gly Ala Arg Glu Ala Leu Phe Ala Leu Ser Ser Asn
   85 90 95
   Leu Ser Phe Leu Pro Gly Gly Glu Tyr Gln Glu Leu Leu Trp Gly Ala
   100 105 110
   Asp Ala Glu Lys Lys Gln Gln Cys Ser Phe Lys Gly Lys Asp Pro Gln
   115 120 125
   Arg Asp Cys Gln Asn Tyr Ile Lys Ile Leu Leu Pro Leu Ser Gly Ser
   130 135 140
   His Leu Phe Thr Cys Gly Thr Ala Ala Phe Ser Pro Met Cys Thr Tyr
   145 150 155 160
   Ile Asn Met Glu Asn Phe Thr Leu Ala Arg Asp Glu Lys Gly Asn Val
   165 170 175
   Leu Leu Glu Asp Gly Lys Gly Arg Cys Pro Phe Asp Pro Asn Phe Lys
   180 185 190
   Ser Thr Ala Leu Val Val Asp Gly Glu Leu Tyr Thr Gly Thr Val Ile
   195 200 205
   Ser Phe Gln Gly Asn Asp Pro Ala Ile Ser Arg Ser Gln Ser Leu Arg
   210 215 220
   Pro Thr Lys Thr Glu Ser Ser Leu Asn Trp Leu Gln Asp Pro Ala Phe
   225 230 235 240
   Val Ala Ser Ala Tyr Ile Pro Glu Ser Leu Gly Ser Leu Gln Gly Asp
   245 250 255
   Asp Asp Lys Ile Tyr Phe Phe Phe Ser Glu Thr Gly Gln Glu Phe Glu
   260 265 270
   Phe Phe Glu Asn Thr Ile Val Ser Arg Ile Ala Arg Ile Cys Lys Gly
   275 280 285
   Asp Glu Gly Gly Glu Arg Val Leu Gln Gln Arg Trp Thr Ser Phe Leu
   290 295 300
   Lys Ala Gln Leu Leu Cys Ser Arg Pro Asp Asp Gly Phe Pro Phe Asn
   305 310 315 320
   Val Leu Gln Asp Val Phe Thr Leu Ser Pro Ser Pro Gln Asp Trp Arg
   325 330 335
   Asp Thr Leu Phe Tyr Gly Val Phe Thr Ser Gln Trp His Arg Gly Thr
   340 345 350
   Thr Glu Gly Ser Ala Val Cys Val Phe Thr Met Lys Asp Val Gln Arg
   355 360 365
   Val Phe Ser Gly Leu Tyr Lys Glu Val Asn Arg Glu Thr Gln Gln Met
   370 375 380
   Val His Arg Asp Pro Pro Val Pro Thr Pro Arg Pro Gly Ala Cys Ile
   385 390 395 400
   Thr Asn Ser Ala Arg Glu Arg Lys Ile Asn Ser Ser Leu Gln Leu Pro
   405 410 415
   Asp Arg Val Leu Asn Phe Leu Lys Asp His Phe Leu Met Asp Gly Gln
   420 425 430
   Val Arg Ser Arg Met Leu Leu Leu Gln Pro Gln Ala Arg Tyr Gln Arg
   435 440 445
   Val Ala Val His Arg Val Pro Gly Leu His His Thr Tyr Asp Val Leu
   450 455 460
   Phe Leu Gly Thr Gly Asp Gly Arg Leu His Lys Ala Val Ser Val Gly
   465 470 475 480
   Pro Arg Val His Ile Ile Glu Glu Leu Gln Ile Phe Ser Ser Gly Gln
   485 490 495
   Pro Val Gln Asn Leu Leu Leu Asp Thr His Arg Gly Leu Leu Tyr Ala
   500 505 510
   Ala Ser His Ser Gly Val Val Gln Val Pro Met Ala Asn Cys Ser Leu
   515 520 525
   Tyr Arg Ser Cys Gly Asp Cys Leu Leu Ala Arg Asp Pro Tyr Cys Ala
   530 535 540
   Trp Ser Gly Ser Ser Cys Lys His Val Ser Leu Tyr Gln Pro Gln Leu
   545 550 555 560
   Ala Thr Arg Pro Trp Ile Gln Asp Ile Glu Gly Ala Ser Ala Lys Asp
   565 570 575
   Leu Cys Ser Ala Ser Ser Val Val Ser Pro Ser Phe Val Pro Thr Gly
   580 585 590
   Glu Lys Pro Cys Glu Gln Val Gln Phe Gln Pro Asn Thr Val Asn Thr
   595 600 605
   Leu Ala Cys Pro Leu Leu Ser Asn Leu Ala Thr Arg Leu Trp Leu Arg
   610 615 620
   Asn Gly Ala Pro Val Asn Ala Ser Ala Ser Cys His Val Leu Pro Thr
   625 630 635 640
   Gly Asp Leu Leu Leu Val Gly Thr Gln Gln Leu Gly Glu Phe Gln Cys
   645 650 655
   Trp Ser Leu Glu Glu Gly Phe Gln Gln Leu Val Ala Ser Tyr Cys Pro
   660 665 670
   Glu Val Val Glu Asp Gly Val Ala Asp Gln Thr Asp Glu Gly Gly Ser
   675 680 685
   Val Pro Val Ile Ile Ser Thr Ser Arg Val Ser Ala Pro Ala Gly Gly
   690 695 700
   Lys Ala Ser Trp Gly Ala Asp Arg Ser Tyr Trp Lys Glu Phe Leu Val
   705 710 715 720
   Met Cys Thr Leu Phe Val Leu Ala Val Leu Leu Pro Val Leu Phe Leu
   725 730 735
   Leu Tyr Arg His Arg Asn Ser Met Lys Val Phe Leu Lys Gln Gly Glu
   740 745 750
   Cys Ala Ser Val His Pro Lys Thr Cys Pro Val Val Leu Pro Pro Glu
   755 760 765
   Thr Arg Pro Leu Asn Gly Leu Gly Pro Pro Ser Thr Pro Leu Asp His
   770 775 780
   Arg Gly Tyr Gln Ser Leu Ser Asp Ser Pro Pro Gly Ser Arg Val Phe
   785 790 795 800
   Thr Glu Ser Glu Lys Arg Pro Leu Ser Ile Gln Asp Ser Phe Val Glu
   805 810 815
   Val Ser Pro Val Cys Pro Arg Pro Arg Val Arg Leu Gly Ser Glu Ile
   820 825 830
   Arg Asp Ser Val Val
   835
<210> 5
   <211> 2511
   <212> DNA
   <213> Homo sapiens
<400> 5
   atgctgcgca ccgcgatggg cctgaggagc tggctcgccg ccccatgggg cgcgctgccg    60
   cctcggccac cgctgctgct gctcctgcta ctgctgctcc tgctgcagcc accgcctccg   120
   acctgggcgc tcagcccccg gatcagcctg cctctgggct ctgaagagcg gccattcctc   180
   agattcgaag ctgaacacat ctccaactac acagcccttc tgctgagcag ggatggcagg   240
   accctgtacg tgggtgctcg agaggccctc tttgcactca gtagcaacct cagcttcctg   300
   ccaggcgggg agtaccagga gctgctttgg ggtgcagacg cagagaagaa acagcagtgc   360
   agcttcaagg gcaaggaccc acagcgcgac tgtcaaaact acatcaagat cctcctgccg   420
   ctcagcggca gtcacctgtt cacctgtggc acagcagcct tcagccccat gtgtacctac   480
   atcaacatgg agaacttcac cctggcaagg gacgagaagg ggaatgtcct cctggaagat   540
   ggcaagggcc gttgtccctt cgacccgaat ttcaagtcca ctgccctggt ggttgatggc   600
   gagctctaca ctggaacagt catcagcttc caagggaatg acccggccat ctcgcggagc   660
   caaagccttc gccccaccaa gaccgagagc tccctcaact ggctgcaaga cccagctttt   720
   gtggcctcag cctacattcc tgagagcctg ggcagcttgc aaggcgatga tgacaagatc   780
   tactttttct tcagcgagac tggccaggaa tttgagttct ttgagaacac cattgtgtcc   840
   cgcattgccc gcatctgcaa gggcgatgag ggtggagagc gggtgctaca gcagcgctgg   900
   acctccttcc tcaaggccca gctgctgtgc tcacggcccg acgatggctt ccccttcaac   960
   gtgctgcagg atgtcttcac gctgagcccc agcccccagg actggcgtga cacccttttc  1020
   tatggggtct tcacttccca gtggcacagg ggaactacag aaggctctgc cgtctgtgtc  1080
   ttcacaatga aggatgtgca gagagtcttc agcggcctct acaaggaggt gaaccgtgag  1140
   acacagcaga tggtacaccg tgacccaccc gtgcccacac cccggcctgg agcgtgcatc  1200
   accaacagtg cccgggaaag gaagatcaac tcatccctgc agctcccaga ccgcgtgctg  1260
   aactttctca aggaccactt cctgatggac gggcaggtcc gaagccgcat gctgctgctg  1320
   cagccccagg ctcgctacca gcgcgtggct gtacaccgcg tccctggcct gcaccacacc  1380
   tacgatgtcc tcttcctggg cactggtgac ggccggctcc acaaggcagt gagcgtgggc  1440
   ccccgggtgc acatcattga ggagctgcag atcttctcat cgggacagcc cgtgcagaat  1500
   ctgctcctgg acacccacag ggggctgctg tatgcggcct cacactcggg cgtagtccag  1560
   gtgcccatgg ccaactgcag cctgtaccgg agctgtgggg actgcctcct cgcccgggac  1620
   ccctactgtg cttggagcgg ctccagctgc aagcacgtca gcctctacca gcctcagctg  1680
   gccaccaggc cgtggatcca ggacatcgag ggagccagcg ccaaggacct ttgcagcgcg  1740
   tcttcggttg tgtccccgtc ttttgtacca acaggggaga agccatgtga gcaagtccag  1800
   ttccagccca acacagtgaa cactttggcc tgcccgctcc tctccaacct ggcgacccga  1860
   ctctggctac gcaacggggc ccccgtcaat gcctcggcct cctgccacgt gctacccact  1920
   ggggacctgc tgctggtggg cacccaacag ctgggggagt tccagtgctg gtcactagag  1980
   gagggcttcc agcagctggt agccagctac tgcccagagg tggtggagga cggggtggca  2040
   gaccaaacag atgagggtgg cagtgtaccc gtcattatca gcacatcgcg tgtgagtgca  2100
   ccagctggtg gcaaggccag ctggggtgca gacaggtcct actggaagga gttcctggtg  2160
   atgtgcacgc tctttgtgct ggccgtgctg ctcccagttt tattcttgct ctaccggcac  2220
   cggaacagca tgaaagtctt cctgaagcag ggggaatgtg ccagcgtgca ccccaagacc  2280
   tgccctgtgg tgctgccccc tgagacccgc ccactcaacg gcctagggcc ccctagcacc  2340
   ccactcgatc accgagggta ccagtccctg tcagacagcc ccccggggtc ccgagtcttc  2400
   actgagtcag agaagaggcc actcagcatc caagacagct tcgtggaggt atccccagtg  2460
   tgcccccggc cccgggtccg ccttggctcg gagatccgtg actctgtggt g            2511
<210> 6
   <211> 3766
   <212> DNA
   <213> Homo sapiens
<400> 6
   gctctgccca agccgaggct gcggggccgg cgccggcggg aggactgcgg tgccccgcgg    60
   aggggctgag tttgccaggg cccacttgac cctgtttccc acctcccgcc ccccaggtcc   120
   ggaggcgggg gcccccgggg cgactcgggg gcggaccgcg gggcggagct gccgcccgtg   180
   agtccggccg agccacctga gcccgagccg cgggacaccg tcgctcctgc tctccgaatg   240
   ctgcgcaccg cgatgggcct gaggagctgg ctcgccgccc catggggcgc gctgccgcct   300
   cggccaccgc tgctgctgct cctgctactg ctgctcctgc tgcagccacc gcctccgacc   360
   tgggcgctca gcccccggat cagcctgcct ctgggctctg aagagcggcc attcctcaga   420
   ttcgaagctg aacacatctc caactacaca gcccttctgc tgagcaggga tggcaggacc   480
   ctgtacgtgg gtgctcgaga ggccctcttt gcactcagta gcaacctcag cttcctgcca   540
   ggcggggagt accaggagct gctttggggt gcagacgcag agaagaaaca gcagtgcagc   600
   ttcaagggca aggacccaca gcgcgactgt caaaactaca tcaagatcct cctgccgctc    660
   agcggcagtc acctgttcac ctgtggcaca gcagccttca gccccatgtg tacctacatc    720
   aacatggaga acttcaccct ggcaagggac gagaagggga atgtcctcct ggaagatggc    780
   aagggccgtt gtcccttcga cccgaatttc aagtccactg ccctggtggt tgatggcgag    840
   ctctacactg gaacagtcat cagcttccaa gggaatgacc cggccatctc gcggagccaa    900
   agccttcgcc ccaccaagac cgagagctcc ctcaactggc tgcaagaccc agcttttgtg    960
   gcctcagcct acattcctga gagcctgggc agcttgcaag gcgatgatga caagatctac   1020
   tttttcttca gcgagactgg ccaggaattt gagttctttg agaacaccat tgtgtcccgc   1080
   attgcccgca tctgcaaggg cgatgagggt ggagagcggg tgctacagca gcgctggacc   1140
   tccttcctca aggcccagct gctgtgctca cggcccgacg atggcttccc cttcaacgtg   1200
   ctgcaggatg tcttcacgct gagccccagc ccccaggact ggcgtgacac ccttttctat   1260
   ggggtcttca cttcccagtg gcacagggga actacagaag gctctgccgt ctgtgtcttc   1320
   acaatgaagg atgtgcagag agtcttcagc ggcctctaca aggaggtgaa ccgtgagaca   1380
   cagcagatgg tacaccgtga cccacccgtg cccacacccc ggcctggagc gtgcatcacc   1440
   aacagtgccc gggaaaggaa gatcaactca tccctgcagc tcccagaccg cgtgctgaac   1500
   tttctcaagg accacttcct gatggacggg caggtccgaa gccgcatgct gctgctgcag   1560
   ccccaggctc gctaccagcg cgtggctgta caccgcgtcc ctggcctgca ccacacctac   1620
   gatgtcctct tcctgggcac tggtgacggc cggctccaca aggcagtgag cgtgggcccc   1680
   cgggtgcaca tcattgagga gctgcagatc ttctcatcgg gacagcccgt gcagaatctg   1740
   ctcctggaca cccacagggg gctgctgtat gcggcctcac actcgggcgt agtccaggtg   1800
   cccatggcca actgcagcct gtaccggagc tgtggggact gcctcctcgc ccgggacccc   1860
   tactgtgctt ggagcggctc cagctgcaag cacgtcagcc tctaccagcc tcagctggcc   1920
   accaggccgt ggatccagga catcgaggga gccagcgcca aggacctttg cagcgcgtct   1980
   tcggttgtgt ccccgtcttt tgtaccaaca ggggagaagc catgtgagca agtccagttc   2040
   cagcccaaca cagtgaacac tttggcctgc ccgctcctct ccaacctggc gacccgactc   2100
   tggctacgca acggggcccc cgtcaatgcc tcggcctcct gccacgtgct acccactggg   2160
   gacctgctgc tggtgggcac ccaacagctg ggggagttcc agtgctggtc actagaggag   2220
   ggcttccagc agctggtagc cagctactgc ccagaggtgg tggaggacgg ggtggcagac   2280
   caaacagatg agggtggcag tgtacccgtc attatcagca catcgcgtgt gagtgcacca   2340
   gctggtggca aggccagctg gggtgcagac aggtcctact ggaaggagtt cctggtgatg   2400
   tgcacgctct ttgtgctggc cgtgctgctc ccagttttat tcttgctcta ccggcaccgg   2460
   aacagcatga aagtcttcct gaagcagggg gaatgtgcca gcgtgcaccc caagacctgc   2520
   cctgtggtgc tgccccctga gacccgccca ctcaacggcc tagggccccc tagcacccca   2580
   ctcgatcacc gagggtacca gtccctgtca gacagccccc cggggtcccg agtcttcact   2640
   gagtcagaga agaggccact cagcatccaa gacagcttcg tggaggtatc cccagtgtgc   2700
   ccccggcccc gggtccgcct tggctcggag atccgtgact ctgtggtgtg agagctgact   2760
   tccagaggac gctgccctgg cttcaggggc tgtgaatgct cggagagggt caactggacc   2820
   tcccctccgc tctgctcttc gtggaacacg accgtggtgc ccggcccttg ggagccttgg   2880
   ggccagctgg cctgctgctc tccagtcaag tagcgaagct cctaccaccc agacacccaa   2940
   acagccgtgg ccccagaggt cctggccaaa tatgggggcc tgcctaggtt ggtggaacag   3000
   tgctccttat gtaaactgag ccctttgttt aaaaaacaat tccaaatgtg aaactagaat   3060
   gagagggaag agatagcatg gcatgcagca cacacggctg ctccagttca tggcctccca   3120
   ggggtgctgg ggatgcatcc aaagtggttg tctgagacag agttggaaac cctcaccaac   3180
   tggcctcttc accttccaca ttatcccgct gccaccggct gccctgtctc actgcagatt   3240
   caggaccagc ttgggctgcg tgcgttctgc cttgccagtc agccgaggat gtagttgttg   3300
   ctgccgtcgt cccaccacct cagggaccag agggctaggt tggcactgcg gccctcacca   3360
   ggtcctgggc tcggacccaa ctcctggacc tttccagcct gtatcaggct gtggccacac   3420
   gagaggacag cgcgagctca ggagagattt cgtgacaatg tacgcctttc cctcagaatt  3480
   cagggaagag actgtcgcct gccttcctcc gttgttgcgt gagaacccgt gtgccccttc  3540
   ccaccatatc caccctcgct ccatctttga actcaaacac gaggaactaa ctgcaccctg 3600
   gtcctctccc cagtccccag ttcaccctcc atccctcacc ttcctccact ctaagggata 3660
   tcaacactgc ccagcacagg ggccctgaat ttatgtggtt tttatacatt ttttaataag 3720
   atgcacttta tgtcattttt taataaagtc tgaagaatta ctgttt 3766
<210> 7
   <211> 837
   <212> PRT
   <213> Homo sapiens
<400> 7
   Met Leu Arg Thr Ala Met Gly Leu Arg Ser Trp Leu Ala Ala Pro Trp
   5 10 15
   Gly Ala Leu Pro Pro Arg Pro Pro Leu Leu Leu Leu Leu Leu Leu Leu
   20 25 30
   Leu Leu Leu Gln Pro Pro Pro Pro Thr Trp Ala Leu Ser Pro Arg Ile
   35 40 45
   Ser Leu Pro Leu Gly Ser Glu Glu Arg Pro Phe Leu Arg Phe Glu Ala
   50 55 60
   Glu His Ile Ser Asn Tyr Thr Ala Leu Leu Leu Ser Arg Asp Gly Arg
   65 70 75 80
   Thr Leu Tyr Val Gly Ala Arg Glu Ala Leu Phe Ala Leu Ser Ser Asn
   85 90 95
   Leu Ser Phe Leu Pro Gly Gly Glu Tyr Gln Glu Leu Leu Trp Gly Ala
   100 105 110
   Asp Ala Glu Lys Lys Gln Gln Cys Ser Phe Lys Gly Lys Asp Pro Gln
   115 120 125
   Arg Asp Cys Gln Asn Tyr Ile Lys Ile Leu Leu Pro Leu Ser Gly Ser
   130 135 140
   His Leu Phe Thr Cys Gly Thr Ala Ala Phe Ser Pro Met Cys Thr Tyr
   145 150 155 160
   Ile Asn Ile Glu Asn Phe Thr Leu Ala Arg Asp Glu Lys Gly Asn Val
   165 170 175
   Leu Leu Glu Asp Gly Lys Gly Arg Cys Pro Phe Asp Pro Asn Phe Lys
   180 185 190
   Ser Thr Ala Leu Val Val Asp Gly Glu Leu Tyr Thr Gly Thr Val Ser
   195 200 205
   Ser Phe Gln Gly Asn Asp Pro Ala Ile Ser Arg Ser Gln Ser Leu Arg
   210 215 220
   Pro Thr Lys Thr Glu Ser Ser Leu Asn Trp Leu Gln Asp Pro Ala Phe
   225 230 235 240
   Val Ala Ser Ala Tyr Ile Pro Glu Ser Leu Gly Ser Leu Gln Gly Asp
   245 250 255
   Asp Asp Lys Ile Tyr Phe Phe Phe Ser Glu Thr Gly Gln Glu Phe Glu
   260 265 270
   Phe Phe Glu Asn Thr Ile Val Ser Arg Ile Ala Arg Ile Cys Lys Gly
   275 280 285
   Asp Glu Gly Gly Glu Arg Val Leu Gln Gln Arg Trp Thr Ser Phe Leu
   290 295 300
   Lys Ala Gln Leu Leu Cys Ser Arg Pro Asp Asp Gly Phe Pro Phe Asn
   305 310 315 320
   Val Leu Gln Asp Val Phe Thr Leu Ser Pro Ser Pro Gln Asp Trp Arg
   325 330 335
   Asp Thr Leu Phe Tyr Gly Val Phe Thr Ser Gln Trp His Arg Gly Thr
   340 345 350
   Thr Glu Gly Ser Ala Val Cys Val Phe Thr Met Lys Asp Val Gln Arg
   355 360 365
   Val Phe Ser Gly Leu Tyr Lys Glu Val Asn Arg Glu Thr Gln Gln Met
   370 375 380
   Val His Arg Asp Pro Pro Val Pro Thr Pro Arg Pro Gly Ala Cys Ile
   385 390 395 400
   Thr Asn Ser Ala Arg Glu Arg Lys Ile Asn Ser Ser Leu Gln Leu Pro
   405 410 415
   Asp Arg Val Leu Asn Phe Leu Lys Asp His Phe Leu Met Asp Gly Gln
   420 425 430
   Val Arg Ser Arg Met Leu Leu Leu Gln Pro Gln Ala Arg Tyr Gln Arg
   435 440 445
   Val Ala Val His Arg Val Pro Gly Leu His His Thr Tyr Asp Val Leu
   450 455 460
   Phe Leu Gly Thr Gly Asp Gly Arg Leu His Lys Ala Val Ser Val Gly
   465 470 475 480
   Pro Arg Val His Ile Ile Glu Glu Leu Gln Ile Phe Ser Ser Gly Gln
   485 490 495
   Pro Val Gln Asn Leu Leu Leu Asp Thr His Arg Gly Leu Leu Tyr Ala
   500 505 510
   Ala Ser His Ser Gly Val Val Gln Val Pro Met Ala Asn Cys Ser Leu
   515 520 525
   Tyr Arg Ser Cys Gly Asp Cys Leu Leu Ala Arg Asp Pro Tyr Cys Ala
   530 535 540
   Trp Ser Gly Ser Ser Cys Lys His Val Ser Leu Tyr Gln Pro Gln Leu
   545 550 555 560
   Ala Thr Arg Pro Trp Ile Gln Asp Ile Glu Gly Ala Ser Ala Lys Asp
   565 570 575
   Leu Cys Ser Ala Ser Ser Val Val Ser Pro Ser Phe Val Pro Thr Gly
   580 585 590
   Glu Lys Pro Cys Glu Gln Val Gln Phe Gln Pro Asn Thr Val Asn Thr
   595 600 605
   Leu Ala Cys Pro Leu Leu Ser Asn Leu Ala Thr Arg Leu Trp Leu Arg
   610 615 620
   Asn Gly Ala Pro Val Asn Ala Ser Ala Ser Cys His Val Leu Pro Thr
   625 630 635 640
   Gly Asp Leu Leu Leu Val Gly Thr Gln Gln Leu Gly Glu Phe Gln Cys
                           645                     650                     655
   Trp Ser Leu Glu Glu Gly Phe Gln Gln Leu Val Ala Ser Tyr Cys Pro
                   660                     665                     670
   Glu Val Val Glu Asp Gly Val Ala Asp Gln Thr Asp Glu Gly Gly Ser
           675                     680                     685
   Val Pro Val Ile Ile Ser Thr Ser Arg Val Ser Ala Pro Ala Gly Gly
       690                     695                     700
   Lys Ala Ser Trp Gly Ala Asp Arg Ser Tyr Trp Lys Glu Phe Leu Val
   705                     710                     715                     720
   Met Cys Thr Leu Phe Val Leu Ala Val Leu Leu Pro Val Leu Phe Leu
                   725                     730                     735
   Leu Tyr Arg His Arg Asn Ser Met Lys Val Phe Leu Lys Gln Gly Glu
               740                     745                     750
   Cys Ala Ser Val His Pro Lys Thr Cys Pro Val Val Leu Pro Pro Glu
           755                     760                     765
   Thr Arg Pro Leu Asn Gly Leu Gly Pro Pro Ser Thr Pro Leu Asp His
       770                     775                     780
   Arg Gly Tyr Gln Ser Leu Ser Asp Ser Pro Pro Gly Ser Arg Val Phe
   785                     790                     795                     800
   Thr Glu Ser Glu Lys Arg Pro Leu Ser Ile Gln Asp Ser Phe Val Glu
                   805                     810                     815
   Val Ser Pro Val Cys Pro Arg Pro Arg Val Arg Leu Gly Ser Glu Ile
               820                     825                     830
   Arg Asp Ser Val Val
           835
<210> 8
   <211> 2511
   <212> DNA
   <213> Homo sapiens
<400> 8
   atgctgcgca ccgcgatggg cctgaggagc tggctcgccg ccccatgggg cgcgctgccg     60
   cctcggccac cgctgctgct gctcctgctg ctgctgctcc tgctgcagcc gccgcctccg    120
   acctgggcgc tcagcccccg gatcagccta cctctgggct ctgaagagcg gccattcctc    180
   agattcgaag ctgaacacat ctccaactac acagcccttc tgctgagcag ggatggcagg    240
   accctgtacg tgggtgctcg agaggccctc tttgcactca gtagcaacct cagcttcctg    300
   ccaggcgggg agtaccagga gctgctttgg ggtgcagacg cagagaagaa acagcagtgc    360
   agcttcaagg gcaaggaccc acagcgcgac tgtcaaaact acatcaagat cctcctgccg    420
   ctcagcggca gtcacctgtt cacctgtggc acagcagcct tcagccccat gtgtacctac    480
   atcaacatag agaacttcac cctggcaagg gacgagaagg ggaatgttct cctggaagat    540
   ggcaagggcc gttgtccctt cgacccgaat ttcaagtcca ctgccctggt ggttgatggc    600
   gagctctaca ctggaacagt cagcagcttc caagggaatg acccggccat ctcgcggagc    660
   caaagccttc gccccaccaa gaccgagagc tccctcaact ggctgcaaga cccagctttt    720
   gtggcctcag cctacattcc tgagagcctg ggcagcttgc aaggcgatga tgacaagatc    780
   tactttttct tcagcgagac tggccaggaa tttgagttct ttgagaacac cattgtgtcc    840
   cgcattgccc gcatctgcaa gggcgatgag ggtggagagc gggtgctaca gcagcgctgg    900
   acctccttcc tcaaggccca gctgctgtgc tcacggcccg acgatggctt ccccttcaac    960
   gtgctgcagg atgtcttcac gctgagcccc agcccccagg actggcgtga cacccttttc    1020
   tatggggtct tcacttccca gtggcacagg ggaactacag aaggctctgc cgtctgtgtc   1080
   ttcacaatga aggatgtgca gagagtcttc agcggcctct acaaggaggt gaaccgtgag   1140
   acacagcaga tggtacaccg tgacccaccc gtgcccacac cccggcctgg agcgtgcatc   1200
   accaacagtg cccgggaaag gaagatcaac tcatccctgc agctcccaga ccgcgtgctg   1260
   aacttcctca aggaccactt cctgatggac gggcaggtcc gaagccgcat gctgctgctg   1320
   cagccccagg ctcgctacca gcgcgtggct gtacaccgcg tccctggcct gcaccacacc   1380
   tacgatgtcc tcttcctggg cactggtgac ggccggctcc acaaggcagt gagcgtgggc   1440
   ccccgggtgc acatcattga ggagctgcag atcttctcat cgggacagcc cgtgcagaat   1500
   ctgctcctgg acacccacag ggggctgctg tatgcggcct cacactcggg cgtagtccag    1560
   gtgcccatgg ccaactgcag cctgtacagg agctgtgggg actgcctcct cgcccgggac   1620
   ccctactgtg cttggagcgg ctccagctgc aagcacgtca gcctctacca gcctcagctg   1680
   gccaccaggc cgtggatcca ggacatcgag ggagccagcg ccaaggacct ttgcagcgcg   1740
   tcttcggttg tgtccccgtc ttttgtacca acaggggaga agccatgtga gcaagtccag   1800
   ttccagccca acacagtgaa cactttggcc tgcccgctcc tctccaacct ggcgacccga   1860
   ctctggctac gcaacggggc ccccgtcaat gcctcggcct cctgccacgt gctacccact   1920
   ggggacctgc tgctggtggg cacccaacag ctgggggagt tccagtgctg gtcactagag   1980
   gagggcttcc agcagctggt agccagctac tgcccagagg tggtggagga cggggtggca   2040
   gaccaaacag atgagggtgg cagtgtaccc gtcattatca gcacatcgcg tgtgagtgca   2100
   ccagctggtg gcaaggccag ctggggtgca gacaggtcct actggaagga gttcctggtg   2160
   atgtgcacgc tctttgtgct ggccgtgctg ctcccagttt tattcttgct ctaccggcac   2220
   cggaacagca tgaaagtctt cctgaagcag ggggaatgtg ccagcgtgca ccccaagacc   2280
   tgccctgtgg tgctgccccc tgagacccgc ccactcaacg gcctagggcc ccctagcacc   2340
   ccgctcgatc accgagggta ccagtccctg tcagacagcc ccccggggtc ccgagtcttc   2400
   actgagtcag agaagaggcc actcagcatc caagacagct tcgtggaggt atccccagtg   2460
   tgcccccggc cccgggtccg ccttggctcg gagatccgtg actctgtggt g            2511
<210> 9
   <211> 3766
   <212> DNA
   <213> Homo sapiens
<400> 9
   gctctgccca agccgaggct gcggggccgg cgccggcggg aggactgcgg tgccccgcgg    60
   aggggctgag tttgccaggg cccacttgac cctgtttccc acctcccgcc ccccaggtcc   120
   ggaggcgggg gcccccgggg cgactcgggg gcggaccgcg gggcggagct gccgcccgtg   180
   agtccggccg agccacctga gcccgagccg cgggacaccg tcgctcctgc tctccgaatg   240
   ctgcgcaccg cgatgggcct gaggagctgg ctcgccgccc catggggcgc gctgccgcct   300
   cggccaccgc tgctgctgct cctgctgctg ctgctcctgc tgcagccgcc gcctccgacc   360
   tgggcgctca gcccccggat cagcctacct ctgggctctg aagagcggcc attcctcaga   420
   ttcgaagctg aacacatctc caactacaca gcccttctgc tgagcaggga tggcaggacc   480
   ctgtacgtgg gtgctcgaga ggccctcttt gcactcagta gcaacctcag cttcctgcca   540
   ggcggggagt accaggagct gctttggggt gcagacgcag agaagaaaca gcagtgcagc   600
   ttcaagggca aggacccaca gcgcgactgt caaaactaca tcaagatcct cctgccgctc   660
   agcggcagtc acctgttcac ctgtggcaca gcagccttca gccccatgtg tacctacatc   720
   aacatagaga acttcaccct ggcaagggac gagaagggga atgttctcct ggaagatggc   780
   aagggccgtt gtcccttcga cccgaatttc aagtccactg ccctggtggt tgatggcgag   840
   ctctacactg gaacagtcag cagcttccaa gggaatgacc cggccatctc gcggagccaa   900
   agccttcgcc ccaccaagac cgagagctcc ctcaactggc tgcaagaccc agcttttgtg   960
   gcctcagcct acattcctga gagcctgggc agcttgcaag gcgatgatga caagatctac  1020
   tttttcttca gcgagactgg ccaggaattt gagttctttg agaacaccat tgtgtcccgc  1080
   attgcccgca tctgcaaggg cgatgagggt ggagagcggg tgctacagca gcgctggacc  1140
   tccttcctca aggcccagct gctgtgctca cggcccgacg atggcttccc cttcaacgtg  1200
   ctgcaggatg tcttcacgct gagccccagc ccccaggact ggcgtgacac ccttttctat  1260
   ggggtcttca cttcccagtg gcacagggga actacagaag gctctgccgt ctgtgtcttc  1320
   acaatgaagg atgtgcagag agtcttcagc ggcctctaca aggaggtgaa ccgtgagaca  1380
   cagcagatgg tacaccgtga cccacccgtg cccacacccc ggcctggagc gtgcatcacc  1440
   aacagtgccc gggaaaggaa gatcaactca tccctgcagc tcccagaccg cgtgctgaac  1500
   ttcctcaagg accacttcct gatggacggg caggtccgaa gccgcatgct gctgctgcag  1560
   ccccaggctc gctaccagcg cgtggctgta caccgcgtcc ctggcctgca ccacacctac  1620
   gatgtcctct tcctgggcac tggtgacggc cggctccaca aggcagtgag cgtgggcccc  1680
   cgggtgcaca tcattgagga gctgcagatc ttctcatcgg gacagcccgt gcagaatctg  1740
   ctcctggaca cccacagggg gctgctgtat gcggcctcac actcgggcgt agtccaggtg   1800
   cccatggcca actgcagcct gtacaggagc tgtggggact gcctcctcgc ccgggacccc  1860
   tactgtgctt ggagcggctc cagctgcaag cacgtcagcc tctaccagcc tcagctggcc  1920
   accaggccgt ggatccagga catcgaggga gccagcgcca aggacctttg cagcgcgtct  1980
   tcggttgtgt ccccgtcttt tgtaccaaca ggggagaagc catgtgagca agtccagttc  2040
   cagcccaaca cagtgaacac tttggcctgc ccgctcctct ccaacctggc gacccgactc  2100
   tggctacgca acggggcccc cgtcaatgcc tcggcctcct gccacgtgct acccactggg  2160
   gacctgctgc tggtgggcac ccaacagctg gggcagttcc agtgctggtc actagaggag  2220
   ggcttccagc agctggtagc cagctactgc ccagaggtgg tggaggacgg ggtggcagac  2280
   caaacagatg agggtggcag tgtacccgtc attatcagca catcgcgtgt gagtgcacca  2340
   gctggtggca aggccagctg gggtgcagac aggtcctact ggaaggagtt cctggtgatg   2400
   tgcacgctct ttgtgctggc cgtgctgctc ccagttttat tcttgctcta ccggcaccgg  2460
   aacagcatga aagtcttcct gaagcagggg gaatgtgcca gcgtgcaccc caagacctgc  2520
   cctgtggtgc tgccccctga gacccgccca ctcaacggcc tagggccccc tagcaccccg  2580
   ctcgatcacc gagggtacca gtccctgtca gacagccccc cggggtcccg agtcttcact  2640
   gagtcagaga agaggccact cagcatccaa gacagcttcg tggaggtatc cccagtgtgc  2700
   ccccggcccc gggtccgcct tggctcggag atccgtgact ctgtggtgtg agagctgact  2760
   tccagaggac gctgccctgg cttcaggggc tgtgaatgct cggagagggt caactggacc  2820
   tcccctccgc tctgctcttc gtggaacacg accgtggtgc ccggcccttg ggagccttgg  2880
   ggccagctgg cctgctgctc tccagtcaag tagcgaagct cctaccaccc agacacccaa  2940
   acagccgtgg ccccagaggt cctggccaaa tatgggggcc tgcctaggtt ggtggaacag  3000
   tgctccttat gtaaactgag ccctttgttt aaaaaacaat tccaaatgtg aaactagaat  3060
   gagagggaag agatagcatg gcatgcagca cacacggctg ctccagttca tggcctccca  3120
   ggggtgctgg ggatgcatcc aaagtggttg tctgagacag agttggaaac cctcaccaac  3180
   tggcctcttc accttccaca ttatcccgct gccaccggct gccctgtctc actgcagatt  3240
   caggaccagc ttgggctgcg tgcgttctgc cttgccagtc agccgaggat gtagttgttg  3300
   ctgccgtcgt cccaccacct cagggaccag agggctaggt tggcactgcg gccctcacca  3360
   ggtcctgggc tcggacccaa ctcctggacc tttccagcct gtatcaggct gtggccacac  3420
   gagaggacag cgcgagctca ggagagattt cgtgacaatg tacgcctttc cctcagaatt  3480
   cagggaagag actgtcgcct gccttcctcc gttgttgcgt gagaacccgt gtgccccttc  3540
   ccaccatatc caccctcgct ccatctttga actcaaacac gaggaactaa ctgcaccctg  3600
   gtcctctccc cagtccccag ttcaccctcc atccctcacc ttcctccact ctaagggata 3660
   tcaacactgc ccagcacagg ggccctgaat ttatgtggtt tttatacatt ttttaataag 3720
   atgcacttta tgtcattttt taataaagtc tgaagaatta ctgttt 3766
<210> 10
   <211> 837
   <212> PRT
   <213> Homo sapiens
<400> 10
   Met Leu Arg Thr Ala Met Gly Leu Arg Ser Trp Leu Ala Ala Pro Trp
   5 10 15
   Gly Ala Leu Pro Pro Arg Pro Pro Leu Leu Leu Leu Leu Leu Leu Leu
   20 25 30
   Leu Leu Leu Gln Pro Pro Pro Pro Thr Trp Ala Leu Ser Pro Arg Ile
   35 40 45
   Ser Leu Pro Leu Gly Ser Glu Glu Arg Pro Phe Leu Arg Phe Glu Ala
   50 55 60
   Glu His Ile Ser Asn Tyr Thr Ala Leu Leu Leu Ser Arg Asp Gly Arg
   65 70 75 80
   Thr Leu Tyr Val Gly Ala Arg Glu Ala Leu Phe Ala Leu Ser Ser Asn
   85 90 95
   Leu Ser Phe Leu Pro Gly Gly Glu Tyr Gln Glu Leu Leu Trp Gly Ala
   100 105 110
   Asp Ala Glu Lys Lys Gln Gln Cys Ser Phe Lys Gly Lys Asp Pro Gln
   115 120 125
   Arg Asp Cys Gln Asn Tyr Ile Lys Ile Leu Leu Pro Leu Ser Gly Ser
   130 135 140
   His Leu Phe Thr Cys Gly Thr Ala Ala Phe Ser Pro Met Cys Thr Tyr
   145 150 155 160
   Ile Asn Met Glu Asn Phe Thr Leu Ala Arg Asp Glu Lys Gly Asn Val
   165 170 175
   Leu Leu Glu Asp Gly Lys Gly Arg Cys Pro Phe Asp Pro Asn Phe Lys
   180 185 190
   Ser Thr Ala Leu Val Val Asp Gly Glu Leu Tyr Thr Gly Thr Val Ser
   195 200 205
   Ser Phe Gln Gly Asn Asp Pro Ala Ile Ser Arg Ser Gln Ser Leu Arg
   210 215 220
   Pro Thr Lys Thr Glu Ser Ser Leu Asn Trp Leu Gln Asp Pro Ala Phe
   225 230 235 240
   Val Ala Ser Ala Tyr Ile Pro Glu Ser Leu Gly Ser Leu Gln Gly Asp
   245 250 255
   Asp Asp Lys Ile Tyr Phe Phe Phe Ser Glu Thr Gly Gln Glu Phe Glu
   260 265 270
   Phe Phe Glu Asn Thr Ile Val Ser Arg Ile Ala Arg Ile Cys Lys Gly
   275 280 285
   Asp Glu Gly Gly Glu Arg Val Leu Gln Gln Arg Trp Thr Ser Phe Leu
   290 295 300
   Lys Ala Gln Leu Leu Cys Ser Arg Pro Asp Asp Gly Phe Pro Phe Asn
   305 310 315 320
   Val Leu Gln Asp Val Phe Thr Leu Ser Pro Ser Pro Gln Asp Trp Arg
   325 330 335
   Asp Thr Leu Phe Tyr Gly Val Phe Thr Ser Gln Trp His Arg Gly Thr
   340 345 350
   Thr Glu Gly Ser Ala Val Cys Val Phe Thr Met Asn Asp Val Gln Arg
   355 360 365
   Val Phe Ser Gly Leu Tyr Lys Glu Val Asn Arg Glu Thr Gln Gln Met
   370 375 380
   Val His Arg Asp Pro Pro Val Pro Thr Pro Arg Pro Gly Ala Cys Ile
   385 390 395 400
   Thr Asn Ser Ala Arg Glu Arg Lys Ile Asn Ser Ser Leu Gln Leu Pro
   405 410 415
   Asp Arg Val Leu Asn Phe Leu Lys Asp His Phe Leu Met Asp Gly Gln
   420 425 430
   Val Arg Ser Arg Met Leu Leu Leu Gln Pro Gln Ala Arg Tyr Gln Arg
   435 440 445
   Val Ala Val His Arg Val Pro Gly Leu His His Thr Tyr Asp Val Leu
   450 455 460
   Phe Leu Gly Thr Gly Asp Gly Arg Leu His Lys Ala Val Ser Val Gly
   465 470 475 480
   Pro Arg Val His Ile Ile Glu Glu Leu Gln Ile Phe Ser Ser Gly Gln
   485 490 495
   Pro Val Gln Asn Leu Leu Leu Asp Thr His Arg Gly Leu Leu Tyr Ala
   500 505 510
   Ala Ser His Ser Gly Val Val Gln Val Pro Met Ala Asn Cys Ser Leu
   515 520 525
   Tyr Arg Ser Cys Gly Asp Cys Leu Leu Ala Arg Asp Pro Tyr Cys Ala
   530 535 540
   Trp Ser Gly Ser Ser Cys Lys His Val Ser Leu Tyr Gln Pro Gln Leu
   545 550 555 560
   Ala Thr Arg Pro Trp Ile Gln Asp Ile Glu Gly Ala Ser Ala Lys Asp
   565 570 575
   Leu Cys Ser Ala Ser Ser Val Val Ser Pro Ser Phe Val Pro Thr Gly
   580 585 590
   Glu Lys Pro Cys Glu Gln Val Gln Phe Gln Pro Asn Thr Val Asn Thr
   595 600 605
   Leu Ala Cys Pro Leu Leu Ser Asn Leu Ala Thr Arg Leu Trp Leu Arg
   610 615 620
   Asn Gly Ala Pro Val Asn Ala Ser Ala Ser Cys His Val Leu Pro Thr
   625 630 635 640
   Gly Asp Leu Leu Leu Val Gly Thr Gln Gln Leu Gly Glu Phe Gln Cys
   645 650 655
   Trp Ser Leu Glu Glu Gly Phe Gln Gln Leu Val Ala Ser Tyr Cys Pro
   660 665 670
   Glu Val Val Glu Asp Gly Val Ala Asp Gln Thr Asp Glu Gly Gly Ser
   675 680 685
   Val Pro Val Ile Ile Ser Thr Ser Arg Val Ser Ala Pro Ala Gly Gly
   690 695 700
   Lys Ala Ser Trp Gly Ala Asp Arg Ser Tyr Trp Lys Glu Phe Leu Val
   705 710 715 720
   Met Cys Thr Leu Phe Val Leu Ala Val Leu Leu Pro Val Leu Phe Leu
   725 730 735
   Leu Tyr Arg His Arg Asn Ser Met Lys Val Phe Leu Lys Gln Gly Glu
   740 745 750
   Cys Ala Ser Val His Pro Lys Thr Cys Pro Val Val Leu Pro Pro Glu
   755 760 765
   Thr Arg Pro Leu Asn Gly Leu Gly Pro Pro Ser Thr Pro Leu Asp His
   770 775 780
   Arg Gly Tyr Gln Ser Leu Ser Asp Ser Pro Pro Gly Ser Arg Val Phe
   785 790 795 800
   Thr Glu Ser Glu Lys Arg Pro Leu Ser Ile Gln Asp Ser Phe Val Glu
   805 810 815
   Val Ser Pro Val Cys Pro Arg Pro Arg Val Arg Leu Gly Ser Glu Ile
   820 825 830
   Arg Asp Ser Val Val
   835
<210> 11
   <211> 2511
   <212> DNA
   <213> Homo sapiens
<400> 11
<210> 12
   <211> 3766
   <212> DNA
   <213> Homo sapiens
<400> 12
   gctctgccca agccgaggct gcggggccgg cgccggcggg aggactgcgg tgccccgcgg     60
   aggggctgag tttgccaggg cccacttgac cctgtttccc acctcccgcc ccccaggtcc    120
   ggaggcgggg gcccccgggg cgactcgggg gcggaccgcg gggcggagct gccgcccgtg    180
   agtccggccg agccacctga gcccgagccg cgggacaccg tcgctcctgc tctccgaatg    240
   ctgcgcaccg cgatgggcct gaggagctgg ctcgccgccc catggggcgc gctgccgcct    300
   cggccaccgc tgctgctgct cctgctgctg ctgctcctgc tgcagccgcc gcctccgacc    360
   tgggcgctca gcccccggat cagcctgcct ctgggctctg aagagcggcc attcctcaga    420
   ttcgaagctg aacacatctc caactacaca gcccttctgc tgagcaggga tggcaggacc    480
   ctgtacgtgg gtgctcgaga ggccctcttt gcactcagta gcaacctcag cttcctgcca    540
   ggcggggagt accaggagct gctttggggt gcagacgcag agaagaaaca gcagtgcagc    600
   ttcaagggca aggacccaca gcgcgactgt caaaactaca tcaagatcct cctgccgctc    660
   agcggcagtc acctgttcac ctgtggcaca gcagccttca gccccatgtg tacctacatc    720
   aacatggaga acttcaccct ggcaagggac gagaagggga atgtcctcct ggaagatggc    780
   aagggccgtt gtcccttcga cccgaatttc aagtccactg ccctggtggt tgatggcgag    840
   ctctacactg gaacagtcag cagcttccaa gggaatgacc cggccatctc gcggagccaa    900
   agccttcgcc ccaccaagac cgagagctcc ctcaactggc tgcaagaccc agcttttgtg    960
   gcctcagcct acattcctga gagcctgggc agcttgcaag gcgatgatga caagatctac 1020
   tttttcttca gcgagactgg ccaggaattt gagttctttg agaacaccat tgtgtcccgc 1080
   attgcccgca tctgcaaggg cgatgagggt ggagagcggg tgctacagca gcgctggacc 1140
   tccttcctca aggcccagct gctgtgctca cggcccgacg atggcttccc cttcaacgtg 1200
   ctgcaggatg tcttcacgct gagccccagc ccccaggact ggcgtgacac ccttttctat 1260
   ggggtcttca cttcccagtg gcacagggga actacagaag gctctgccgt ctgtgtcttc 1320
   acaatgaatg atgtgcagag agtcttcagc ggcctctaca aggaggtgaa ccgtgagaca 1380
   cagcagatgg tacaccgtga cccacccgtg cccacacccc ggcctggagc gtgcatcacc 1440
   aacagtgccc gggaaaggaa gatcaactca tccctgcagc tcccagaccg cgtgctgaac 1500
   tttctcaagg accacttcct gatggacggg caggtccgaa gccgcatgct gctgctgcag 1560
   ccccaggctc gctaccagcg cgtggctgta caccgcgtcc ctggcctgca ccacacctac 1620
   gatgtcctct tcctgggcac tggtgacggc cggctccaca aggcagtgag cgtgggcccc 1680
   cgggtgcaca tcattgagga gctgcagatc ttctcatcgg gacagcccgt gcagaatctg 1740
   ctcctggaca cccacagggg gctgctgtat gcggcctcac actcgggcgt agtccaggtg 1800
   cccatggcca actgcagcct gtaccggagc tgtggggact gcctcctcgc ccgggacccc 1860
   tactgtgctt ggagcggctc cagctgcaag cacgtcagcc tctaccagcc tcagctggcc 1920
   accaggccgt ggatccagga catcgaggga gccagcgcca aggacctttg cagcgcgtct 1980
   tcggttgtgt ccccgtcttt tgtaccaaca ggggagaagc catgtgagca agtccagttc 2040
   cagcccaaca cagtgaacac tttggcctgc ccgctcctct ccaacctggc gacccgactc 2100
   tggctacgca acggggcccc cgtcaatgcc tcggcctcct gccacgtgct acccactggg 2160
   gacctgctgc tggtgggcac ccaacagctg gggagttcca agtgctggtc actagaggag  2220
   ggcttccagc agctggtagc cagctactgc ccagaggtgg tggaggacgg ggtggcagac 2280
   caaacagatg agggtggcag tgtacccgtc attatcagca catcgcgtgt gagtgcacca 2340
   gctggtggca aggccagctg gggtgcagac aggtcctact ggaaggagtt cctggtgatg 2400
   tgcacgctct ttgtgctggc cgtgctgctc ccagttttat tcttgctcta ccggcaccgg 2460
   aacagcatga aagtcttcct gaagcagggg gaatgtgcca gcgtgcaccc caagacctgc 2520
   cctgtggtgc tgccccctga gacccgccca ctcaacggcc tagggccccc tagcacccca 2580
   ctcgatcacc gagggtacca gtccctgtca gacagccccc cggggtcccg agtcttcact 2640
   gagtcagaga agaggccact cagcatccaa gacagcttcg tggaggtatc cccagtgtgc 2700
   ccccggcccc gggtccgcct tggctcggag atccgtgact ctgtggtgtg agagctgact 2760
   tccagaggac gctgccctgg cttcaggggc tgtgaatgct cggagagggt caactggacc 2820
   tcccctccgc tctgctcttc gtggaacacg accgtggtgc ccggcccttg ggagccttgg 2880
   ggccagctgg cctgctgctc tccagtcaag tagcgaagct cctaccaccc agacacccaa 2940
   acagccgtgg ccccagaggt cctggccaaa tatgggggcc tgcctaggtt ggtggaacag 3000
   tgctccttat gtaaactgag ccctttgttt aaaaaacaat tccaaatgtg aaactagaat 3060
   gagagggaag agatagcatg gcatgcagca cacacggctg ctccagttca tggcctccca 3120
   ggggtgctgg ggatgcatcc aaagtggttg tctgagacag agttggaaac cctcaccaac 3180
   tggcctcttc accttccaca ttatcccgct gccaccggct gccctgtctc actgcagatt 3240
   caggaccagc ttgggctgcg tgcgttctgc cttgccagtc agccgaggat gtagttgttg 3300
   ctgccgtcgt cccaccacct cagggaccag agggctaggt tggcactgcg gccctcacca 3360
   ggtcctgggc tcggacccaa ctcctggacc tttccagcct gtatcaggct gtggccacac 3420
   gagaggacag cgcgagctca ggagagattt cgtgacaatg tacgcctttc cctcagaatt 3480
   cagggaagag actgtcgcct gccttcctcc gttgttgcgt gagaacccgt gtgccccttc 3540
   ccaccatatc caccctcgct ccatctttga actcaaacac gaggaactaa ctgcaccctg 3600
   gtcctctccc cagtccccag ttcaccctcc atccctcacc ttcctccact ctaagggata 3660
   tcaacactgc ccagcacagg ggccctgaat ttatgtggtt tttatacatt ttttaataag 3720
   atgcacttta tgtcattttt taataaagtc tgaagaatta ctgttt 3766
<210> 13
   <211> 20
   <212> DNA
   <213> Artificial Sequence
<220>
   <223> Oligonucleotide
<400> 13
   cagtgccaac ctagccctct 20
<210> 14
   <211> 20
   <212> DNA
   <213> Artificial Sequence
<220>
   <223> Oligonucleotide
<400> 14
   tctcccgatc caaccgtgac 20
<210> 15
   <211> 20
   <212> DNA
   <213> Artificial Sequence
<220>
   <223> Oligonucleotide
<400> 15
   caacaactac atcctcggct 20
<210> 16
   <211> 20
   <212> DNA
   <213> Artificial Sequence
<220>
   <223> Oligonucleotide
<400> 16
   tcggctccta catcaacaac 20
<210> 17
   <211> 24
   <212> DNA
   <213> Artificial Sequence
<220>
   <223> Primer
<400> 17
   cctcgcccgg gacccctact gtgc 24
<210> 18
   <211> 27
   <212> DNA
   <213> Artificial Sequence
<220>
   <223> Primer
<400> 18
   cttggcgctg gctccctcga tgtcctg 27
<210> 19
   <211> 28
   <212> DNA
   <213> Artificial Sequence
<220>
   <223> Primer
<400> 19
   aattgaattc atgctgcgca ccgcgatg 28
<210> 20
   <211> 30
   <212> DNA
   <213> Artificial Sequence
<220>
   <223> Primer
<400> 20
   aagctctaga caccacagag tcacggatct 30
<210> 21
   <211> 30
   <212> DNA
   <213> Artificial Sequence
<220> primer
   <223> Primer
<400> 21
   aagctctaga tcacaccaca gagtcacgga 30
<210> 22
   <211> 12
   <212> PRT
   <213> Homo sapiens
<400> 22
   Asn Ser Ala Arg Glu Arg Lys Ile Asn Ser Ser Cys
   5 10
<210> 23
   <211> 15
   <212> PRT
   <213> Homo sapiens
<400> 23
   Ser Val Val Ser Pro Ser Phe Val Pro Thr Gly Glu Lys Pro Cys
   5 10 15
<210> 24
   <211> 15
   <212> PRT
   <213> Homo sapiens
<400> 24
   Pro Leu Asp His Arg Gly Tyr Gln Ser Leu Ser Asp Ser Pro Cys
   5 10 15
<210> 25
   <211> 14
   <212> PRT
   <213> Homo sapiens
<400> 25
   Ser Arg Val Phe Thr Glu Ser Glu Lys Arg Pro Leu Ser Cys
   5 10
<210> 26
   <211> 99
   <212> PRT
   <213> Homo sapiens
<400> 26
   Arg His Arg Asn Ser Met Lys Val Phe Leu Lys Gln Gly Glu Cys Ala
   5 10 15
   Ser Val His Pro Lys Thr Cys Pro Val Val Leu Pro Pro Glu Thr Arg
   20 25 30
   Pro Leu Asn Gly Leu Gly Pro Pro Ser Thr Pro Leu Asp His Arg Gly
   35 40 45
   Tyr Gln Ser Leu Ser Asp Ser Pro Pro Gly Ser Arg Val Phe Thr Glu
   50 55 60
   Ser Glu Lys Arg Pro Leu Ser Ile Gln Asp Ser Phe Val Glu Val Ser
   65 70 75 80
   Pro Val Cys Pro Arg Pro Arg Val Arg Leu Gly Ser Glu Ile Arg Asp
   85 90 95
   Ser Val Val
<210> 27
   <211> 926
   <212> PRT
   <213> Homo sapiens
<400> 27
   Met Pro Val Arg Lys Gln Asp Thr Gln Arg Ala Leu His Leu Leu Glu
                   5                   10                  15
   Glu Tyr Arg Ser Lys Leu Ser Gln Thr Glu Asp Arg Gln Leu Arg Ser
               20                  25                  30
   Ser Ile Glu Arg Val Ile Asn Ile Phe Gln Ser Asn Leu Phe Gln Ala
           35                  40                  45
   Leu Ile Asp Ile Gln Glu Phe Tyr Glu Val Thr Leu Leu Asp Asn Pro
       50                  55                  60
   Lys Cys Ile Asp Arg Ser Lys Pro Ser Glu Pro Ile Gln Pro Val Asn
   65                  70                  75                  80
   Thr Trp Glu Ile Ser Ser Leu Pro Ser Ser Thr Val Thr Ser Glu Thr
                   85                  90                  95
   Leu Pro Ser Ser Leu Ser Pro Ser Val Glu Lys Tyr Arg Tyr Gln Asp
               100                 105                 110
   Glu Asp Thr Pro Pro Gln Glu His Ile Ser Pro Gln Ile Thr Asn Glu
           115                 120                 125
   Val Ile Gly Pro Glu Leu Val His Val Ser Glu Lys Asn Leu Ser Glu
       130                 135                 140
   Ile Glu Asn Val His Gly Phe Val Ser His Ser His Ile Ser Pro Ile
   145                 150                 155                 160
   Lys Pro Thr Glu Ala Val Leu Pro Ser Pro Pro Thr Val Pro Val Ile
                   165                 170                 175
   Pro Val Leu Pro Val Pro Ala Glu Asn Thr Val Ile Leu Pro Thr Ile
               180                 185                 190
   Pro Gln Ala Asn Pro Pro Pro Val Leu Val Asn Thr Asp Ser Leu Glu
           195                 200                 205
   Thr Pro Thr Tyr Val Asn Gly Thr Asp Ala Asp Tyr Glu Tyr Glu Glu
       210                 215                 220
   Ile Thr Leu Glu Arg Gly Asn Ser Gly Leu Gly Phe Ser Ile Ala Gly
   225 230 235 240
   Gly Thr Asp Asn Pro His Ile Gly Asp Asp Ser Ser Ile Phe Ile Thr
   245 250 255
   Lys Ile Ile Thr Gly Gly Ala Ala Ala Gln Asp Gly Arg Leu Arg Val
   260 265 270
   Asn Asp Cys Ile Leu Gln Val Asn Glu Val Asp Val Arg Asp Val Thr
   275 280 285
   His Ser Lys Ala Val Glu Ala Leu Lys Glu Ala Gly Ser Ile Val Arg
   290 295 300
   Leu Tyr Val Lys Arg Arg Lys Pro Val Ser Glu Lys Ile Met Glu Ile
   305 310 315 320
   Lys Leu Ile Lys Gly Pro Lys Gly Leu Gly Phe Ser Ile Ala Gly Gly
   325 330 335
   Val Gly Asn Gln His Ile Pro Gly Asp Asn Ser Ile Tyr Val Thr Lys
   340 345 350
   Ile Ile Glu Gly Gly Ala Ala His Lys Asp Gly Lys Leu Gln Ile Gly
   355 360 365
   Asp Lys Leu Leu Ala Val Asn Asn Val Cys Leu Glu Glu Val Thr His
   370 375 380
   Glu Glu Ala Val Thr Ala Leu Lys Asn Thr Ser Asp Phe Val Tyr Leu
   385 390 395 400
   Lys Val Ala Lys Pro Thr Ser Met Tyr Met Asn Asp Gly Tyr Ala Pro
   405 410 415
   Pro Asp Ile Thr Asn Ser Ser Ser Gln Pro Val Asp Asn His Val Ser
   420 425 430
   Pro Ser Ser Phe Leu Gly Gln Thr Pro Ala Ser Pro Ala Arg Tyr Ser
   435 440 445
   Pro Val Ser Lys Ala Val Leu Gly Asp Asp Glu Ile Thr Arg Glu Pro
   450 455 460
   Arg Lys Val Val Leu His Arg Gly Ser Thr Gly Leu Gly Phe Asn Ile
   465 470 475 480
   Val Gly Gly Glu Asp Gly Glu Gly Ile Phe Ile Ser Phe Ile Leu Ala
   485 490 495
   Gly Gly Pro Ala Asp Leu Ser Gly Glu Leu Arg Lys Gly Asp Arg Ile
   500 505 510
   Ile Ser Val Asn Ser Val Asp Leu Arg Ala Ala Ser His Glu Gln Ala
   515 520 525
   Ala Ala Ala Leu Lys Asn Ala Gly Gln Ala Val Thr Ile Val Ala Gln
   530 535 540
   Tyr Arg Pro Glu Glu Tyr Ser Arg Phe Glu Ala Lys Ile His Asp Leu
   545 550 555 560
   Arg Glu Gln Met Met Asn Ser Ser Ile Ser Ser Gly Ser Gly Ser Leu
   565 570 575
   Arg Thr Ser Gln Lys Arg Ser Leu Tyr Val Arg Ala Leu Phe Asp Tyr
   580 585 590
   Asp Lys Thr Lys Asp Ser Gly Leu Pro Ser Gln Gly Leu Asn Phe Lys
   595 600 605
   Phe Gly Asp Ile Leu His Val Ile Asn Ala Ser Asp Asp Glu Trp Trp
   610 615 620
   Gln Ala Arg Gln Val Thr Pro Asp Gly Glu Ser Asp Glu Val Gly Val
   625 630 635 640
   Ile Pro Ser Lys Arg Arg Val Glu Lys Lys Glu Arg Ala Arg Leu Lys
   645 650 655
   Thr Val Lys Phe Asn Ser Lys Thr Arg Asp Lys Gly Gln Ser Phe Asn
   660 665 670
   Asp Lys Arg Lys Lys Asn Leu Phe Ser Arg Lys Phe Pro Phe Tyr Lys
   675 680 685
   Asn Lys Asp Gln Ser Glu Gln Glu Thr Ser Asp Ala Asp Gln His Val
   690 695 700
   Thr Ser Asn Ala Ser Asp Ser Glu Ser Ser Tyr Arg Gly Gln Glu Glu
   705 710 715 720
   Tyr Val Leu Ser Tyr Glu Pro Val Asn Gln Gln Glu Val Asn Tyr Thr
   725 730 735
   Arg Pro Val Ile Ile Leu Gly Pro Met Lys Asp Arg Ile Asn Asp Asp
   740 745 750
   Leu Ile Ser Glu Phe Pro Asp Lys Phe Gly Ser Cys Val Pro His Thr
   755 760 765
   Thr Arg Pro Lys Arg Asp Tyr Glu Val Asp Gly Arg Asp Tyr His Phe
   770 775 780
   Val Thr Ser Arg Glu Gln Met Glu Lys Asp Ile Gln Glu His Lys Phe
   785 790 795 800
   Ile Glu Ala Gly Gln Tyr Asn Asn His Leu Tyr Gly Thr Ser Val Gln
   805 810 815
   Ser Val Arg Glu Val Ala Gly Lys Gly Lys His Cys Ile Leu Asp Val
   820 825 830
   Ser Gly Asn Ala Ile Lys Arg Leu Gln Ile Ala Gln Leu Tyr Pro Ile
   835 840 845
   Ser Ile Phe Ile Lys Pro Lys Ser Met Glu Asn Ile Met Glu Met Asn
   850 855 860
   Lys Arg Leu Thr Glu Glu Gln Ala Arg Lys Thr Phe Glu Arg Ala Met
   865 870 875 880
   Lys Leu Glu Gln Glu Phe Thr Glu His Phe Thr Ala Ile Val Gln Gly
   885 890 895
   Asp Thr Leu Glu Asp Ile Tyr Asn Gln Val Lys Gln Ile Ile Glu Glu
   900 905 910
   Gln Ser Gly Ser Tyr Ile Trp Val Pro Ala Lys Glu Lys Leu
   915 920 925
<210> 28
   <211> 2778
   <212> DNA
   <213> Homo sapiens
<400> 28
   atgccggtcc ggaagcaaga tacccagaga gcattgcacc ttttggagga atatcgttca 60
   aaactaagcc aaactgaaga cagacagctc agaagttcca tagaacgggt tattaacata 120
   tttcagagca acctctttca ggctttaata gatattcaag aattttatga agtgacctta 180
   ctggataatc caaaatgtat agatcgttca aagccgtctg aaccaattca acctgtgaat 240
   acttgggaga tttccagcct tccaagctct actgtgactt cagagacact gccaagcagc 300
   cttagcccta gtgtagagaa atacaggtat caggatgaag atacacctcc tcaagagcat 360
   atttccccac aaatcacaaa tgaagtgata ggtccagaat tggttcatgt ctcagagaag 420
   aacttatcag agattgagaa tgtccatgga tttgtttctc attctcatat ttcaccaata 480
   aagccaacag aagctgttct tccctctcct cccactgtcc ctgtgatccc tgtcctgcca 540
   gtccctgctg agaatactgt catcctaccc accataccac aggcaaatcc tcccccagta 600
   ctggtcaaca cagatagctt ggaaacacca acttacgtta atggcacaga tgcagattat 660
   gaatatgaag aaatcacact tgaaagggga aattcagggc ttggtttcag cattgcagga 720
   ggtacggaca acccacacat tggagatgac tcaagtattt tcattaccaa aattatcaca 780
   gggggagcag ccgcccaaga tggaagattg cgggtcaatg actgtatatt acaagtaaat 840
   gaagtagatg ttcgtgatgt aacacatagc aaagcagttg aagcgttgaa agaagcaggg 900
   tctattgtac gcttgtatgt aaaaagaagg aaaccagtgt cagaaaaaat aatggaaata 960
   aagctcatta aaggtcctaa aggtcttggg tttagcattg ctggaggtgt tggaaatcag 1020
   catattcctg gggataatag catctatgta accaaaataa ttgaaggagg tgcagcacat 1080
   aaggatggca aacttcagat tggagataaa cttttagcag tgaataacgt atgtttagaa 1140
   gaagttactc atgaagaagc agtaactgcc ttaaagaaca catctgattt tgtttatttg 1200
   aaagtggcaa aacccacaag tatgtatatg aatgatggct atgcaccacc tgatatcacc 1260
   aactcttctt ctcagcctgt tgataaccat gttagcccat cttccttctt gggccagaca 1320
   ccagcatctc cagccagata ctccccagtt tctaaagcag tacttggaga tgatgaaatt 1380
   acaagggaac ctagaaaagt tgttcttcat cgtggctcaa cgggccttgg tttcaacatt 1440
   gtaggaggag aagatggaga aggaatattt atttccttta tcttagccgg aggacctgct 1500
   gatctaagtg gagagctcag aaaaggagat cgtattatat cggtaaacag tgttgacctc 1560
   agagctgcta gtcatgagca ggcagcagct gcattgaaaa atgctggcca ggctgtcaca 1620
   attgttgcac aatatcgacc tgaagaatac agtcgttttg aagctaaaat acatgattta 1680
   cgggagcaga tgatgaatag tagtattagt tcagggtcag gttctcttcg aactagccag 1740
   aagcgatccc tctatgtcag agcccttttt gattatgaca agactaaaga cagtgggctt 1800
   cccagtcagg gactgaactt caaatttgga gatatcctcc atgttattaa tgcttctgat 1860
   gatgaatggt ggcaagccag gcaggttaca ccagatggtg agagcgatga ggtcggagtg 1920
   attcccagta aacgcagagt tgagaagaaa gaacgagccc gattaaaaac agtgaaattc 1980
   aattctaaaa cgagagataa agggcagtca ttcaatgaca agcgtaaaaa gaacctcttt 2040
   tcccgaaaat tccccttcta caagaacaag gaccagagtg agcaggaaac aagtgatgct 2100
   gaccagcatg taacttctaa tgccagcgat agtgaaagta gttaccgtgg tcaagaagaa 2160
   tacgtcttat cttatgaacc agtgaatcaa caagaagtta attatactcg accagtgatc 2220
   atattgggac ctatgaaaga caggataaat gatgacttga tctcagaatt tcctgacaaa 2280
   tttggatcct gtgttcctca tacaactaga ccaaaacgag attatgaggt agatggaaga 2340
   gattatcatt ttgtgacttc aagagagcag atggaaaaag atatccagga acataaattc 2400
   attgaagctg gccagtataa caatcatcta tatggaacaa gtgttcagtc tgtacgagaa 2460
   gtagcaggaa agggcaaaca ctgtatcctt gatgtgtctg gaaatgccat aaagagatta 2520
   cagattgcac agctttaccc tatctccatt tttattaaac ccaaatccat ggaaaatatc 2580
   atggaaatga ataagcgtct aacagaagaa caagccagaa aaacatttga gagagccatg 2640
   aaactggaac aggagtttac tgaacatttc acagctattg tacaggggga tacgctggaa 2700
   gacatttaca accaagtgaa acagatcata gaagaacaat ctggttctta catctgggtt 2760
   ccggcaaaag aaaagcta 2778
<210> 29
   <211> 817
   <212> PRT
   <213> Homo sapiens
<400> 29
   Met His Lys His Gln His Cys Cys Lys Cys Pro Glu Cys Tyr Glu Val
   5 10 15
   Thr Arg Leu Ala Ala Leu Arg Arg Leu Glu Pro Pro Gly Tyr Gly Asp
   20 25 30
   Trp Gln Val Pro Asp Pro Tyr Gly Pro Gly Gly Gly Asn Gly Ala Ser
   35 40 45
   Ala Gly Tyr Gly Gly Tyr Ser Ser Gln Thr Leu Pro Ser Gln Ala Gly
   50 55 60
   Ala Thr Pro Thr Pro Arg Thr Lys Ala Lys Leu Ile Pro Thr Gly Arg
   65 70 75 80
   Asp Val Gly Pro Val Pro Leu Lys Pro Val Pro Gly Lys Ser Thr Pro
   85 90 95
   Lys Leu Asn Gly Ser Gly Pro Ser Trp Trp Pro Glu Cys Thr Cys Thr
   100 105 110
   Asn Arg Asp Trp Tyr Glu Gln Val Asn Gly Ser Asp Gly Met Phe Lys
   115 120 125
   Tyr Glu Glu Ile Val Leu Glu Arg Gly Asn Ser Gly Leu Gly Phe Ser
   130 135 140
   Ile Ala Gly Gly Ile Asp Asn Pro His Val Pro Asp Asp Pro Gly Ile
   145 150 155 160
   Phe Ile Thr Lys Ile Ile Pro Gly Gly Ala Ala Ala Met Asp Gly Arg
   165 170 175
   Leu Gly Val Asn Asp Cys Val Leu Arg Val Asn Glu Val Glu Val Ser
   180 185 190
   Glu Val Val His Ser Arg Ala Val Glu Ala Leu Lys Glu Ala Gly Pro
   195 200 205
   Val Val Arg Leu Val Val Arg Arg Arg Gln Pro Pro Pro Glu Thr Ile
   210 215 220
   Met Glu Val Asn Leu Leu Lys Gly Pro Lys Gly Leu Gly Phe Ser Ile
   225 230 235 240
   Ala Gly Gly Ile Gly Asn Gln His Ile Pro Gly Asp Asn Ser Ile Tyr
   245 250 255
   Ile Thr Lys Ile Ile Glu Gly Gly Ala Ala Gln Lys Asp Gly Arg Leu
   260 265 270
   Gln Ile Gly Asp Arg Leu Leu Ala Val Asn Asn Thr Asn Leu Gln Asp
   275 280 285
   Val Arg His Glu Glu Ala Val Ala Ser Leu Lys Asn Thr Ser Asp Met
   290 295 300
   Val Tyr Leu Lys Val Ala Lys Pro Gly Ser Leu His Leu Asn Asp Met
   305 310 315 320
   Tyr Ala Pro Pro Asp Tyr Ala Ser Thr Phe Thr Ala Leu Ala Asp Asn
   325 330 335
   His Ile Ser His Asn Ser Ser Leu Gly Tyr Leu Gly Ala Val Glu Ser
   340 345 350
   Lys Val Ser Tyr Pro Ala Pro Pro Gln Val Pro Pro Thr Arg Tyr Ser
   355 360 365
   Pro Ile Pro Arg His Met Leu Ala Glu Glu Asp Phe Thr Arg Glu Pro
   370 375 380
   Arg Lys Ile Ile Leu His Lys Gly Ser Thr Gly Leu Gly Phe Asn Ile
   385 390 395 400
   Val Gly Gly Glu Asp Gly Glu Gly Ile Phe Val Ser Phe Ile Leu Ala
   405 410 415
   Gly Gly Pro Ala Asp Leu Ser Gly Glu Leu Arg Arg Gly Asp Arg Ile
   420 425 430
   Leu Ser Val Asn Gly Val Asn Leu Arg Asn Ala Thr His Glu Gln Ala
   435 440 445
   Ala Ala Ala Leu Lys Arg Ala Gly Gln Ser Val Thr Ile Val Ala Gln
   450 455 460
   Tyr Arg Pro Glu Glu Tyr Ser Arg Phe Glu Ser Lys Ile His Asp Leu
   465 470 475 480
   Arg Glu Gln Met Met Asn Ser Ser Met Ser Ser Gly Ser Gly Ser Leu
   485 490 495
   Arg Thr Ser Glu Lys Arg Ser Leu Tyr Val Arg Ala Leu Phe Asp Tyr
   500 505 510
   Asp Arg Thr Arg Asp Ser Cys Leu Pro Ser Gln Gly Leu Ser Phe Ser
   515 520 525
   Tyr Gly Asp Ile Leu His Val Ile Asn Ala Ser Asp Asp Glu Trp Trp
   530 535 540
   Gln Ala Arg Leu Val Thr Pro His Gly Glu Ser Glu Gln Ile Gly Val
   545 550 555 560
   Ile Pro Ser Lys Lys Arg Val Glu Lys Lys Glu Arg Ala Arg Leu Lys
   565 570 575
   Thr Val Lys Phe His Ala Arg Thr Gly Met Ile Glu Ser Asn Arg Asp
   580 585 590
   Phe Pro Gly Leu Ser Asp Asp Tyr Tyr Gly Ala Lys Asn Leu Lys Gly
   595 600 605
   Gln Glu Asp Ala Ile Leu Ser Tyr Glu Pro Val Thr Arg Gln Glu Ile
   610 615 620
   His Tyr Ala Arg Pro Val Ile Ile Leu Gly Pro Met Lys Asp Arg Val
   625 630 635 640
   Asn Asp Asp Leu Ile Ser Glu Phe Pro His Lys Phe Gly Ser Cys Val
   645 650 655
   Pro His Thr Thr Arg Pro Arg Arg Asp Asn Glu Val Asp Gly Gln Asp
   660 665 670
   Tyr His Phe Val Val Ser Arg Glu Gln Met Glu Lys Asp Ile Gln Asp
   675 680 685
   Asn Lys Phe Ile Glu Ala Gly Gln Phe Asn Asp Asn Leu Tyr Gly Thr
       690                 695                 700
   Ser Ile Gln Ser Val Arg Ala Val Ala Glu Arg Gly Lys His Cys Ile
   705                 710                 715                 720
   Leu Asp Val Ser Gly Asn Ala Ile Lys Arg Leu Gln Gln Ala Gln Leu
                   725                 730                 735
   Tyr Pro Ile Ala Ile Phe Ile Lys Pro Lys Ser Ile Glu Ala Leu Met
               740                 745                 750
   Glu Met Asn Arg Arg Gln Thr Tyr Glu Gln Ala Asn Lys Ile Tyr Asp
           755                 760                 765
   Lys Ala Met Lys Leu Glu Gln Glu Phe Gly Glu Tyr Phe Thr Ala Ile
       770                 775                 780
   Val Gln Gly Asp Ser Leu Glu Glu Ile Tyr Asn Lys Ile Lys Gln Ile
   785                 790                 795                 800
   Ile Glu Asp Gln Ser Gly His Tyr Ile Trp Val Pro Ser Pro Glu Lys
                   805                 810                 815
   Leu
<210> 30
   <211> 2451
   <212> DNA
   <213> Homo sapiens
<400> 30
   atgcacaagc accagcactg ctgtaagtgc cctgagtgct atgaggtgac ccgcctggcc   60
   gccctgcggc gcctcgagcc tccggggtac ggcgactggc aagtccccga cccttacggg  120
   ccaggtgggg gcaacggcgc cagcgcgggt tatgggggct acagctcgca gaccttgccc  180
   tcgcaggcgg gggccacccc cacccctcgc accaaggcca agctcatccc caccggccgg  240
   gatgtggggc cggtgcctct taagccagtc ccgggcaaga gcacccccaa actcaacggc  300
   agcggcccca gctggtggcc agagtgcacc tgtaccaacc gggactggta tgagcaggtg  360
   aatggcagtg atggcatgtt caaatatgag gaaatcgtac ttgagagggg caactctggc  420
   ctgggcttca gtatcgcagg tggcatcgac aatccccatg tccctgatga ccctggcatt  480
   tttattacca agattatccc tggtggagca gctgccatgg atgggaggct gggggtgaat  540
   gactgtgtgc tgcgggtgaa tgaggtggaa gtgtcggagg tggtacacag ccgggcggtg  600
   gaggcgctga aagaggcagg ccctgtggtg cgattggtgg tgcggaggcg acagcctcca  660
   cccgagacca tcatggaggt caacctgctc aaagggccca aggcctgggg tttcagcatt  720
   gctgggggta ttggcaacca gcacatccca ggagacaaca gcatctacat caccaagatc  780
   attgaggggg gtgctgctca gaaggatgga cgcctacaga ttggggaccg gctgctggcg  840
   gtgaacaaca ccaatctgca ggatgtgagg cacgaggaag ctgtggcctc actgaagaac  900
   acatctgata tggtgtattt gaaggtggcc aagccaggca gcctccacct caacgacatg  960
   tacgctcccc ctgactacgc cagcactttt actgccttgg ctgacaacca cataagccat 1020
   aattccagcc tgggttatct cggggctgtg gagagcaagg tcagctaccc tgctcctcct 1080
   caggttcccc ccacccgcta ctctcctatt cccaggcaca tgctggctga ggaggacttc 1140
   accagagagc ctcgcaagat catcctgcac aaaggctcca caggcctggg cttcaacatc 1200
   gtaggaggag aggatggaga aggcattttt gtctccttca tcctggcagg aggcccagct 1260
   gacctgagtg gggagctgcg caggggagac cggatcttat cggtgaatgg agtgaatctg 1320
   aggaatgcaa ctcatgagca ggctgcagct gctctgaaac gggccggcca gtcagtcacc 1380
   attgtggccc agtacagacc tgaagaatac agtcgctttg aatcgaagat acatgactta 1440
   cgagaacaaa tgatgaacag cagcatgagc tctgggtctg ggtccctccg aacaagtgaa 1500
   aagaggtcct tgtatgtcag ggccctgttt gattatgatc ggactcggga cagctgcctg 1560
   ccaagccagg ggctcagctt ctcttatggt gacattctgc atgtcattaa tgcctctgat 1620
   gatgagtggt ggcaggcaag gctggtgacc ccacacggag aaagtgagca gatcggtgtg 1680
   atccccagta agaagagggt ggaaaagaaa gaaagagctc gattgaaaac tgtgaagttc 1740
   catgccagga cggggatgat tgagtctaac agggacttcc cggggttaag tgacgattat 1800
   tatggagcaa agaacctgaa aggacaagag gatgctattt tgtcatatga gccagtgaca 1860
   cggcaagaaa ttcactatgc aaggcctgtg atcatcctgg gcccaatgaa ggaccgagtc 1920
   aatgatgacc tgatctccga atttccacat aaatttggat cctgtgtgcc acatactacc 1980
   cggcctcgac gtgataatga ggtggatgga caagactacc actttgtggt gtcccgagaa 2040
   caaatggaga aagatattca ggacaacaag ttcatcgagg cgggccaatt taatgataac 2100
   ctctatggga ccagcatcca gtcagtgcgg gcagttgcag agaggggcaa gcactgcatc 2160
   ttagatgttt ccggcaatgc tatcaagaga ctgcagcaag cacaacttta ccccattgcc 2220
   attttcatca agcccaagtc cattgaagcc cttatggaaa tgaaccgaag gcagacatat 2280
   gaacaagcaa ataagatcta tgacaaagcc atgaaactgg agcaggaatt tggagagtac 2340
   tttacagcca ttgtacaggg tgactcactg gaagagattt ataacaaaat caaacaaatc 2400
   attgaggacc agtctgggca ctacatttgg gtcccatccc ctgaaaaact c          2451

## Description

### TECHNICAL FIELD

The present invention relates to novel binding proteins, and so on. More particularly, the present invention relates to a complex of Semaphorin 4B, Semaphorin 4B-M1, Semaphorin 4B-M2 or Semaphorin 4B-M3 with a protein capable of binding to said protein, an antibody to said complex, an antibody that inhibits or promotes the formation of said complex, an antibody that promotes or inhibits the dissociation of said complex, screening of a compound or its salt that inhibits or promotes the formation of said complex, screening of a compound or its salt that promotes or inhibits the dissociation of said complex, a compound or its salt which is obtainable by said screening, an agent for the prevention/treatment of, or a diagnostic agent for, cancer or neurodegenerative diseases, and the like.

### BACKGROND OF INVENTION

Genes for Semaphorin 4B (WO 00/012708, WO 00/078961) (hereinafter sometimes abbreviated as SEMA4B), SEMA4B-M1, SEMA4B-M2 or SEMA4B-M3 (hereinafter these three genes are sometimes collectively referred to as SEMA4B-Ms) encode a single-transmembrane protein expressed on the cytoplasmic membrane of cancer cells and are overexpressed in human cancer tissues such as lung cancer, ovarian cancer, pancreatic cancer, etc. Since these antisense oligonucleotides induce the apoptosis of cancer cells, it is reported that SEMA4B and SEMA4B-Ms are involved in sustaining survival via the suppression of apoptosis in cancer cells (WO 2004/058817).

SEMA4B is also reported as one of the genes overexpressed under hypoxic conditions (WO 02/46465). It is also reported that several hundreds of base sequences including SEMA4B, etc. can be used for search of compounds for diagnosis and treatment for lung cancer, based on the gene chip analysis (WO 02/86443). It is reported that NOV7 having 93% homology with SEMA4B on an amino acid level is overexpressed in cancer (WO 02/06329).

Inagaki et al. (Journal of Biological Chemistry 2001, Vol. 276, pp. 9174-9181) describe the interaction of Semaphorin 4C with post-synaptic density protein PSD-95 isoforms containing inter alia the domain SAP97/DLG-1. However, this publication does not refer to Semaphorin 4B.

Lim et al. (Journal of Biological Chemistry 2002, Vol. 277, pp. 21697-21711) describe that the PDZ domains of PSD-95 selectively bind at the carboxyl terminus of their binding partners, like the NR2B subunit of the N-methyl-D-aspartate-type glutamate receptor.

Clark et al. (Genome Research 2003, Vol. 13, pp. 2265-2270) published a large scale effort to identify novel human secreted and transmembrane proteins. This effort resulted in a total number of 879 secreted and transmembrane proteins wherein 9 are Semaphroins and Plexin repeat receptors.
Burkhardt et al. (FEBS Letters 2005, Vol. 579, pp. 3821-3828) describe the interaction of Semaphorin 4B with post-synaptic density protein PSD-95, which is characterized by a PDZ binding motive at the carboxy terminus of the Semaphorin 4B.

Human discs large homolog 1 (DLG1, GenBank NM_004087.1) is a gene cloned from a library derived from B lymphoblasts and a human homolog of cancer suppressor gene DLG in Drosophila (Proc. Natl. Acad. Sci. USA, 91, 9818-9822, 1994).

Human discs large homolog 3 (DLG3, GenBank NM_021120.1) is a gene cloned from a library derived from human fetal brain and is a human homolog of cancer suppressor gene DLG in Drosophila as in DLG1 (Oncogene, 14, 2425-2433, 1997).

It is reported on the physiological role that DLG1 and DLG3 form a complex with a tumor suppressor gene APC to promote degradation of β-catenin and thus suppress cell growth (Oncogene, 19, 365-372, 2000; Int. J. Cancer, 86, 480-488, 2000). Concerning DLG1, it is also pointed out that there is a possibility to be involved in cellular proliferation through its binding to TOPK (PBK), which is a mitotically active serine/threonine kinase (Proc. Natl. Acad. Sci. USA 97, 5167-5172, 2000).

### DISCLOSURE OF THE INVENTION

A safe agent for the prevention/treatment of cancer, which targets at a protein complex specifically expressed in cancer cells or neurons to induce growth inhibition of cancer cells, and a safe and excellent agent, which suppresses neuronal apoptosis to prevent/treat neurodegenerative diseases, have been earnestly desired.

By regulating an anti-apoptosis signal mediated by SEMA4B or SEMA4B-Ms, it can be expected to kill cancer cells thereby to exert an anti-tumor effect, or to suppress neuronal cell death thereby to help prevent or treat neurodegenerative diseases. At this point of time, however, there are only few reports on the intracellular binding protein or intracellular signal transduction pathway of SEMA4B or SEMA4B-Ms.

In order to solve the foregoing problems, the present inventors made extensive studies and as a result, found that DLG1 and DLG3, which are suggested to function as a cancer suppressor gene, bind to the intracellular domain of SEMA4B or SEMA4B-Ms, using the yeast-two hybrid assay (Trends in Genet., 10, 286-292, 1994; Annu. Rev. Genet., 31, 663-704, 1997; The Yeast Two-Hybrid System, Oxford University Press, Bartel and Fields, 1997). The inventors further found how to evaluate the activity of regulating the SEMA4B-dependent or SEMA4B-Ms-dependent anti-apoptosis signal. Based on these findings, the inventors have continued further studies and come to accomplish the present invention.

That is, the present invention provides the following features, and so on.
(1) A complex comprising (a) a protein comprising the amino acid sequence of SEQ ID NO: 26, or a salt thereof and (b) (i) a protein comprising an amino acid sequence having at least 95% homology to the amino acid sequence of SEQ ID NO: 27 or/and SEQ ID NO: 29, and which binds to the protein defined in (a) above , or a salt thereof. The following embodiments are further described herein:
   (1a) A complex comprising (a) a protein comprising the same or substantially the same amino acid sequence as the amino acid sequence represented by SEQ ID NO: 26, a partial peptide thereof, or a salt thereof and (b) a protein comprising the same or substantially the same amino acid sequence as the amino acid sequence represented by SEQ ID NO: 27, a partial peptide thereof, or a salt thereof.
   (1b) A complex comprising (a) a protein comprising the same or substantially the same amino acid sequence as the amino acid sequence represented by SEQ ID NO: 26, a partial peptide thereof, or a salt thereof and (b) a protein comprising the same or substantially the same amino acid sequence as the amino acid sequence represented by SEQ ID NO: 29, a partial peptide thereof, or a salt thereof.
   (1c) A complex comprising (a) a protein comprising the same or substantially the same amino acid sequence as the amino acid sequence represented by SEQ ID NO: 26, a partial peptide thereof, or a salt thereof, (b) a protein comprising the same or substantially the same amino acid sequence as the amino acid sequence represented by SEQ ID NO: 27, a partial peptide thereof, or a salt thereof, and (c) a protein comprising the same or substantially the same amino acid sequence as the amino acid sequence represented by SEQ ID NO: 29, a partial peptide thereof, or a salt thereof.
   (1d) The complex according to (1) above, wherein the partial peptide of the protein comprising the same or substantially the same amino acid sequence as the amino acid sequence represented by SEQ ID NO: 27 is a peptide having the 142^{nd} to 310^{th} amino acid residues of the amino acid sequence represented by SEQ ID NO: 27.
   (1e) The complex according to (1) above, wherein the partial peptide of the protein comprising the same or substantially the same amino acid sequence as the amino acid sequence represented by SEQ ID NO: 29 is a peptide having the 81^{st} to 353^{rd} amino acid residues of the amino acid sequence represented by SEQ ID NO: 29.
   The present invention further provides:
(2) The complex according to (1) above, wherein the protein comprising the amino acid sequence of SEQ ID NO: 26 is a protein comprising the amino acid sequence of SEQ ID NO: 1, SEQ ID NO: 4, SEQ ID NO: 7 or SEQ ID NO: 10.
   The following embodiments are further described herein:
   (2a) The complex according to (1) above, wherein the protein comprising the same or substantially the same amino acid sequence as the amino acid sequence represented by SEQ ID NO: 26 is a protein consisting of an amino acid sequence represented by SEQ ID NO: 1.
   (2b) The complex according to (1) above, wherein the protein comprising the same or substantially the same amino acid sequence as the amino acid sequence represented by SEQ ID NO: 26 is a protein consisting of an amino acid sequence represented by SEQ ID NO: 4, SEQ ID NO: 7 or SEQ ID NO: 10.
   The present invention further provides:
(3) An antibody to the complex according to (1) above.
   The following embodiments are further described herein:
   (3a) An antibody to the complex or a part of the complex according to (1) above.
(4) An antibody which inhibits the formation of the complex according to (1) above.
(5) An antibody which promotes the dissociation of the complex according to (1) above.
(6) An antibody which promotes the formation of the complex according to (1) above.
(7) An antibody which inhibits the dissociation of the complex according to (1) above.
   The present invention further provides:
(8) A medicament comprising the antibody according to (3) above
   The following embodiments are further described herein:
   (8a) A medicament comprising the antibody according to (4) above.
(9) A medicament comprising the antibody according to (5) above.
(10) A medicament comprising the antibody according to (6) above.
(11) A medicament comprising the antibody according to (7) above.
(12) The medicament according to (8) or (9) above, which is an apoptosis promoter of cancer cells, a growth inhibitor of cancer cells, or an agent for the prevention/treatment of cancer.
(13) The medicament according to (10) or (11) above, which is an apoptosis inhibitor of nerve cells, or an agent for the prevention/treatment of neurodegenerative disease.
   The present invention further provides:
(14) A diagnostic agent comprising the antibody according to (3) above.
   The following embodiments are further described herein:
   (14a) A diagnostic agent comprising the antibody according to one of (3a) to (7) above.
(15) The diagnostic agent according to (14) and (14a) above, which is a diagnostic agent for cancer or neurodegenerative disease.
(16) A method of screening a compound or its salt that inhibits or promotes the binding of (a) a protein comprising the amino acid sequence of SEQ ID NO: 26, or a salt thereof, to (b) (i) a protein comprising the the amino acid sequence of SEQ ID NO: 27 or SEQ ID NO: 29 or (ii) a protein comprising an amino acid sequence having at least 95% homology to the amino acid sequence of SEQ ID NO: 27 or SEQ ID NO: 29, or a salt thereof, which comprises using the protein defined in (a) above, or a salt thereof and the protein defined in (b) above, or a salt thereof.
   The following embodiments are further described herein:
   (16a) The screening method according to (16) above, which comprises using a cell capable of producing a protein comprising the same or substantially the same amino acid sequence as the amino acid sequence represented by SEQ ID NO: 26, a partial peptide thereof, or a salt thereof.
   (16b) The screening method according to (16) above, comprises using a cell capable of producing a protein comprising the same or substantially the same amino acid sequence as the amino acid sequence represented by SEQ ID NO: 27 or SEQ ID NO: 29, a partial peptide thereof, or a salt thereof.
   (16c) The screening method according to (16) above, wherein the partial peptide of the protein comprising the same or substantially the same amino acid sequence as the amino acid sequence represented by SEQ ID NO: 27 is a peptide having the 142^{nd} to 310^{th} amino acid residues of the amino acid sequence represented by SEQ ID NO: 27.
   (16d) The screening method according to (16) above, wherein the partial peptide of the protein comprising the same or substantially the same amino acid sequence as the amino acid sequence represented by SEQ ID NO: 29 is a peptide having the 81^{st} to 353^{rd} amino acid residues of the amino acid sequence represented by SEQ ID NO: 29.
   (16e) The screening method according to (16) above, wherein the partial peptide of the protein comprising the same or substantially the same amino acid sequence as the amino acid sequence represented by SEQ ID NO: 26 is a peptide having having the amino acid sequence represented by SEQ ID NO: 26.
   The present invention further provides:
(17) A kit for screening a compound or its salt that inhibits or promotes the binding of (a) a protein comprising the amino acid sequence of SEQ ID NO: 26, or a salt thereof, to (b) (i) a protein comprising the amino acid sequence of SEQ ID NO: 27 or SEQ ID NO: 29 or (ii) a protein comprising an amino acid sequence having at least 95% homology to the amino acid sequence of SEQ ID NO: 27 or SEQ ID NO: 29, or a salt thereof, which comprises the protein defined in (a) above, or a salt thereof and the protein defined in (b) above, or a salt thereof.
   The following embodiments are further described herein:
   (17a) A compound or its salt that inhibits or promotes the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof, obtainable by using the screening method according to (16) above or the screening kit according to (17).
   (17b) The compound or its salt according to (17a) above, wherein the compound is a compound that inhibits the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or/and SEQ ID NO: 29, a partial peptide thereof, or a salt thereof.
   (17c) The compound or its salt according to (17a) above, wherein the compound is a compound that promotes the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or/and SEQ ID NO: 29, a partial peptide thereof, or a salt thereof.
   (17d) A medicament comprising the compound or its salt according to (17b) above. (17e) A medicament comprising the compound or its salt according to (17c) above. (17f) The medicament according to (17d) above, which is an apoptosis promoter of cancer cells, a growth inhibitor of cancer cells, or an agent ofr the prevention/treatment of cancer.
   (17g) The medicament according to (17e) above, which is an apoptosis inhibitor of nerve cells, or an agent for the prevention/treatment of neurodegenerative disease.
(18) A compound or its salt that inhibits the binding of (a) a protein comprising the same or substantially the same amino acid sequence as the amino acid sequence represented by SEQ ID NO: 26, a partial peptide thereof, or a salt thereof, to (b) a protein(s) comprising the same or substantially the same amino acid sequence as the amino acid sequence(s) represented by SEQ ID NO: 27 or/and SEQ ID NO: 29, a partial peptide thereof, or a salt thereof.
(19) An apoptosis promoter of cancer cells, a growth inhibitor of cancer cells, or an agent for the prevention/treatment of cancer, which comprises the compound or its salt according to (18) above.
(20) A compound or its salt that promotes the binding of (a) a protein comprising the same or substantially the same amino acid sequence as the amino acid sequence represented by SEQ ID NO: 26, a partial peptide thereof, or a salt thereof, to (b) a protein(s) comprising the same or substantially the same amino acid sequence as the amino acid sequence(s) represented by SEQ ID NO: 27 or/and SEQ ID NO: 29, a partial peptide thereof, or a salt thereof.
(21) An agent for the prevention/treatment of an apoptosis inhibitor of nerve cells, or an agent for the prevention/treatment of neurodegenerative disease, which comprises the compound or its salt according to (20) above.
   The present invention further provides:
(22) A method of screening a compound or its salt that promotes or inhibits the dissociation of a complex comprising (a) a protein comprising the amino acid sequence of SEQ ID NO: 26, or a salt thereof, and (b) (i) a protein comprising the amino acid sequence of SEQ ID NO: 27 or SEQ ID NO: 29 or (ii) a protein comprising an amino acid sequence having at least 95% homology to the amino acid sequence of SEQ ID NO: 27 or SEQ ID NO: 29 and which binds to the protein defined in (a) above, or a salt thereof, which comprises using the protein defined in (a) above, or a salt thereof and the protein defined in (b) above, or a salt thereof.
(23) A kit for screening a compound or its salt that promotes or inhibits the dissociation of a complex comprising (a) a protein comprising the amino acid sequence of SEQ ID NO: 26, or a salt thereof, and (b) (i) a protein comprising the amino acid sequence of SEQ ID NO: 27 or SEQ ID NO: 29 or (ii) a protein comprising an amino acid sequence having at least 95% homology to the amino acid sequence of SEQ ID NO: 27 or SEQ ID NO: 29, and which binds to the protein defined in (a) above and which binds to the protein defined in (a) above, or a salt thereof, which comprises using the protein defined in (a) above, and the protein defined in (b) above.
   The following embodiments are further described herein:
(24) A compound or its salt that promotes the dissociation of a complex comprising (a) a protein comprising the same or substantially the same amino acid sequence as the amino acid sequence represented by SEQ ID NO: 26, a partial peptide thereof, or a salt thereof, and (b) a protein(s) comprising the same or substantially the same amino acid sequence as the amino acid sequence(s) represented by SEQ ID NO: 27 or/and SEQ ID NO: 29, a partial peptide thereof, or a salt thereof.
(25) An apoptosis promoter of cancer cells, a growth inhibitor of cancer cells, or an agent for the prevention/treatment of cancer comprising the compound or its salt according to (24) above.
(26) A compound or its salt that inhibits the dissociation of a complex comprising (a) a protein comprising the same or substantially the same amino acid sequence as the amino acid sequence represented by SEQ ID NO: 26, a partial peptide thereof, or a salt thereof, and (b) a protein(s) comprising the same or substantially the same amino acid sequence as the amino acid sequence(s) represented by SEQ ID NO: 27 or/and SEQ ID NO: 29, a partial peptide thereof, or a salt thereof.
(27) An apoptosis inhibitor of nerve cells or a neurodegenerative disease, comprising the compound or its salt according to (26) above.
(28) A method of promoting the apoptosis of cancer cells or a method of inhibiting the growth of cancer cells, which comprises inhibiting the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof.
(29) A method of preventing/treating cancer, which comprises inhibiting the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof.
(30) A method of inhibiting the apoptosis of nerve cells or a method of preventing/treating neurodegenerative disease, which comprises promoting the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof.
(31) A method of promoting the apoptosis of cancer cells or inhibiting the growth of cancer cells, which comprises promoting the dissociation of a complex comprising (a) a protein comprising the same or substantially the same amino acid sequence as the amino acid sequence represented by SEQ ID NO: 26, a partial peptide thereof, or a salt thereof, and (b) a protein comprising the same or substantially the same amino acid sequence as the amino acid sequence represented by SEQ ID NO: 27 or SEQ ID NO: 29, a partial peptide thereof, or a salt thereof.
(32) A method of preventing/treating cancer, which comprises promoting the dissociation of a complex comprising (a) a protein comprising the same or substantially the same amino acid sequence as the amino acid sequence represented by SEQ ID NO: 26, a partial peptide thereof, or a salt thereof, and (b) a protein comprising the same or substantially the same amino acid sequence as the amino acid sequence represented by SEQ ID NO: 27 or SEQ ID NO: 29, a partial peptide thereof, or a salt thereof.
(33) A method of inhibiting the apoptosis of nerve cells or a method of preventing/treating neurodegenerative disease, which comprises inhibiting the dissociation of a complex comprising (a) a protein comprising the same or substantially the same amino acid sequence as the amino acid sequence represented by SEQ ID NO: 26, a partial peptide thereof, or a salt thereof, and (b) a protein comprising the same or substantially the same amino acid sequence as the amino acid sequence represented by SEQ ID NO: 27 or SEQ ID NO: 29, a partial peptide thereof, or a salt thereof.
   The present invention further provides:
(34) A method of screening a compound or its salt having a preventive/therapeutic effect on cancer or neurodegenerative disease, which comprises using the protein according to claim (1) (a) and the protein according to claim (1) (b).
   The following embodiments are further described herein:
(35) Use of a substance (e.g., a compound or its salt, or an antibody) that inhibits the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof, to manufacture an apoptosis promoter of cancer cells or a growth inhibitor of cancer cells.
(36) Use of a substance (e.g., a compound or its salt, or an antibody) that inhibits the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof, to manufacture an agent for the prevention/treatment of cancer.
(37) Use of a substance (e.g., a compound or its salt, or an antibody) that promotes the binding of (a) a protein comprising the same or substantially the same amino acid sequence as the amino acid sequence represented by SEQ ID NO: 26, a partial peptide thereof, or a salt thereof, to (b) a protein comprising the same or substantially the same amino acid sequence as the amino acid sequence represented by SEQ ID NO: 27 or SEQ ID NO: 29, a partial peptide thereof, or a salt thereof, to manufacture an apoptosis inhibitor of nerve cells, or an agent for the prevention/treatment of neurodegenerative disease.
(38) Use of a substance (e.g., a compound or its salt) that promotes the dissociation of a complex comprising (a) a protein comprising the same or substantially the same amino acid sequence as the amino acid sequence represented by SEQ ID NO: 26, a partial peptide thereof, or a salt thereof, and (b) a protein comprising the same or substantially the same amino acid sequence as the amino acid sequence represented by SEQ ID NO: 27 or SEQ ID NO: 29, a partial peptide thereof, or a salt thereof, to manufacture an apoptosis promoter of cancer cells or a growth inhibitor of cancer cells.
(39) Use of a salt of substance (e.g., a compound or its salt, or an antibody) that promotes the dissociation of a complex comprising (a) a protein comprising the same or substantially the same amino acid sequence as the amino acid sequence represented by SEQ ID NO: 26, a partial peptide thereof, or a salt thereof, and (b) a protein comprising the same or substantially the same amino acid sequence as the amino acid sequence represented by SEQ ID NO: 27 or SEQ ID NO: 29, a partial peptide thereof, or a salt thereof, to manufacture an agent for the prevention/treatment of cancer.
(40) Use of a substance (e.g., a compound or its salt, or an antibody) that inhibits the dissociation of a complex comprising (a) a protein comprising the same or substantially the same amino acid sequence as the amino acid sequence represented by SEQ ID NO: 26, a partial peptide thereof, or a salt thereof, and (b) a protein comprising the same or substantially the same amino acid sequence as the amino acid sequence represented by SEQ ID NO: 27 or SEQ ID NO: 29, a partial peptide thereof, or a salt thereof, to manufacture an apoptosis inhibitor of nerve cells, or an agent for the prevention/treatment of neurodegenerative disease.
   In the present specification the following features are further described.
(41) The screening method according to (16) above, which comprises either immobilizing the protein comprising the same or substantially the same amino acid sequence as the amino acid sequence represented by SEQ ID NO: 27 or SEQ ID NO: 29, a partial peptide thereof, or a salt thereof (hereinafter DLG1 protein or DLG3 protein) onto a solid phase (e.g., an EIA plate) using an antibody to the DLG1 protein or DLG3 protein or the DLG1 protein or DLG3 protein fused to a Tag protein (e.g., His-Tag, GST (glutathione-S-transferase), etc.); then measuring and comparing (i) the binding amount in the case where the protein comprising the same or substantially the same amino acid sequence as the amino acid sequence represented by SEQ ID NO: 26, a partial peptide thereof, or a salt thereof (hereinafter the SEMA4B protein) (e.g., a partial peptide corresponding to the cytoplasmic domain (e.g., a peptide having the amino acid sequence represented by SEQ ID NO: 26, etc.)), which is labeled with a labeling agent (e.g., biotin, etc.), is brought in contact with the immobilized DLG1 protein or DLG3 protein and (ii) the binding amount in the case where the SEMA4B protein, which is labeled with a labeling agent (e.g., biotin, etc.), and a test compound are brought in contact with the immobilized DLG1 protein or DLG3 protein; and selecting the test compound that decreases the binding amount in the case (ii) above by at least about 20%, preferably by at least about 30% and more preferably by at least about 50%, as compared to the case (i) above, as a compound that inhibits the binding of the SEMA4B protein to the DLG1 protein or DLG3 protein, or, the test compound that increases the activity in the case (ii) above by at least about 20%, preferably by at least about 30% and more preferably by at least about 50%, as compared to the case (i) above, as a compound that promotes the binding of the SEMA4B protein to the DLG1 protein or DLG3 protein (wherein, when a partial peptide of the DLG1 protein or DLG3 protein is used as said protein, there is preferably used a partial peptide having the activity of binding to the SEMA4B protein (e.g., a peptide having the 142-310 amino acid sequence in the amino acid sequence represented by SEQ ID NO: 27, a peptide having the 81-353 amino acid sequence in the amino acid sequence represented by SEQ ID NO: 29, etc.), or the like. In immobilizing a tagged protein onto a solid phase, nickel is used when it is His-Tag and glutathione is used, when it is GST.).
(42) The screening method according to (16) above, which comprises immobilizing the protein comprising the same or substantially the same amino acid sequence as the amino acid sequence represented by SEQ ID NO: 26, a partial peptide thereof, or a salt thereof (hereinafter SEMA4B protein) (e.g., a partial peptide corresponding to the cytoplasmic domain (e.g., a peptide having the amino acid sequence represented by SEQ ID NO: 26, etc.)), which is labeled with a labeling agent (e.g., biotin, etc.), onto an avidin-labeled solid phase (e.g., a plate); then measuring and comparing (i) the binding amount in the case where the protein comprising the same or substantially the same amino acid sequence as the amino acid sequence represented by SEQ ID NO: 27 or SEQ ID NO: 29, a partial peptide thereof, or a salt thereof (hereinafter the DLG1 protein or DLG3 protein) is brought in contact with the immobilized SEMA4B protein and (ii) the binding amount in the case where the DLG1 protein or DLG3 protein and a test compound are simultaneously brought in contact with the immobilized SEMA4B protein; and selecting the test compound that decreases the binding amount in the case (ii) above by at least about 20%, preferably by at least about 30% and more preferably by at least about 50%, as compared to the case (i) above, as a compound that inhibits the binding of the SEMA4B protein to the DLG1 protein or DLG3 protein, or the test compound that increases the activity in the case (ii) above by at least about 20%, preferably by at least about 30% and more preferably by at least about 50%, as compared to the case (i) above, as a compound that promotes the binding of the SEMA4B protein to the DLG1 protein or DLG3 protein (wherein, when a partial peptide of the DLG1 protein or DLG3 protein is used as said protein, there is preferably used a partial peptide having the activity of binding to the SEMA4B protein (e.g., a peptide having the 142-310 amino acid sequence in the amino acid sequence represented by SEQ ID NO: 27, a peptide having the 81-353 amino acid sequence in the amino acid sequence represented by SEQ ID NO: 29, etc.), or the like.
(43) The screening method according to (16) above, which comprises measuring and comparing (i) the binding amount in the case where the protein comprising the same or substantially the same amino acid sequence as the amino acid sequence represented by SEQ ID NO: 27 or SEQ ID NO: 29, a partial peptide thereof, or a salt thereof (hereinafter the DLG1 protein or DLG3 protein) is brought in contact with an immobilized protein comprising the same or substantially the same amino acid sequence as the amino acid sequence represented by SEQ ID NO: 26, a partial peptide thereof, or a salt thereof (hereinafter SEMA4B protein) (e.g., a partial peptide corresponding to the cytoplasmic domain (e.g., a peptide having the amino acid sequence represented by SEQ ID NO: 26, etc.)), and (ii) the binding amount in the case where the DLG1 protein or DLG3 protein and a test compound are brought in contact simultaneously with the immobilized SEMA4B protein; selecting the test compound that decreases the binding amount in the case (ii) above by at least about 20%, preferably by at least about 30% and more preferably by at least about 50%, as compared to the case (i) above, as a compound that inhibits the binding of the SEMA4B protein to the DLG1 protein or DLG3 protein, or the test compound that increases the activity in the case (ii) above by at least about 20%, preferably by at least about 30% and more preferably by at least about 50%, as compared to the case (i) above, as a compound that promotes the binding of the SEMA4B protein to the DLG1 protein or DLG3 protein (wherein the DLG1 protein or DLG3 protein used may be a protein fused to a Tag protein; in this case, the DLG1 protein or DLG3 protein may be detected/quantified using an antibody to said protein, or may be detected/quantified using an antibody to the Tag protein).
(44) The screening method according to (16), which comprises co-expressing (a) a DNA encoding a chimeric protein wherein a reporter gene binding domain is fused to the partial peptide (e.g., a peptide having the amino acid sequence represented by SEQ ID NO: 26, etc.) or a salt thereof of the protein comprising the same or substantially the same amino acid sequence as the amino acid sequence represented by SEQ ID NO: 26, a partial peptide thereof, or a salt thereof (hereinafter the SEMA4B protein) and (b) a DNA encoding a chimeric protein wherein a reporter gene transcriptional activation domain is fused to the protein comprising the same or substantially the same amino acid sequence as the amino acid sequence represented by SEQ ID NO: 27 or SEQ ID NO: 29, a partial peptide thereof, or a salt thereof (hereinafter the DLG1 protein or DLG3 protein) in yeast (e.g., *Saccharomyces cerevisiae,* more preferably *S. cerevisiae* Y190 strain) thereby to express the phenotypes of β-galactosidase as the reporter gene and histidine biosynthesis gene HIS3 by the two-hybrid action; incubating the yeast strain for a given period of time in the presence of a test compound; and selecting the compound that decreases the β-galactosidase activity of the yeast strain or the compound that changes the yeast strain histidine-auxotrophic, as a compound that inhibits the binding of the SEMA4B protein to the DLG1 protein or DLG3 protein.
(45) The screening method according to (22), which comprises either immobilizing the protein comprising the same or substantially the same amino acid sequence as the amino acid sequence represented by SEQ ID NO: 27 or SEQ ID NO: 29, a partial peptide thereof, or a salt thereof (hereinafter DLG1 protein or DLG3 protein) onto a solid phase (e.g., an EIA plate) using an antibody to the DLG1 protein or DLG3 protein or the DLG1 protein or DLG3 protein fused to a Tag protein (e.g., His-Tag, GST (glutathione-S-transferase), etc.); then measuring and comparing (i) the binding amount in the case where the protein comprising the same or substantially the same amino acid sequence as the amino acid sequence represented by SEQ ID NO: 26, a partial peptide thereof, or a salt thereof (hereinafter the SEMA4B protein) (e.g., a partial peptide corresponding to the cytoplasmic domain (e.g., a peptide having the amino acid sequence represented by SEQ ID NO: 26, etc.)), which is labeled with a labeling agent (e.g., biotin, etc.), is brought in contact with the immobilized DLG1 protein or DLG3 protein and (ii) the binding amount in the case where the SEMA4B protein labeled with a labeling agent (e.g., biotin, etc.) and a test compound are brought in contact simultaneously with the immobilized DLG1 protein or DLG3 protein; and selecting the test compound that decreases the binding amount in the case (ii) above by at least about 20%, preferably by at least about 30% and more preferably by at least about 50%, as compared to the case (i) above, as a compound that promotes the dissociation of the complex of the SEMA4B protein with the DLG1 protein or DLG3 protein, or, the test compound that increases the activity in the case (ii) above by at least about 20%, preferably by at least about 30% and more preferably by at least about 50%, as compared to the case (i) above, as a compound that inhibits the dissociation of the complex of the SEMA4B protein with the DLG1 protein or DLG3 protein (wherein, when a partial peptide of the DLG1 protein or DLG3 protein is used as said protein, there is preferably used a partial peptide having the activity of binding to the SEMA4B protein (e.g., a peptide having the 142-310 amino acid sequence in the amino acid sequence represented by SEQ ID NO: 27, a peptide having the 81-353 amino acid sequence in the amino acid sequence represented by SEQ ID NO: 29, etc.) or the like. In immobilizing a tagged protein onto a solid phase, nickel is used when it is His-Tag and when it is GST, glutathione is used.).
(46) The screening method according to (22) above, which comprises immobilizing the protein comprising the same or substantially the same amino acid sequence as the amino acid sequence represented by SEQ ID NO: 26, a partial peptide thereof, or a salt thereof (hereinafter SEMA4B protein) (e.g., a partial peptide corresponding to the cytoplasmic domain (e.g., a peptide having the amino acid sequence represented by SEQ ID NO: 26, etc.)), which is labeled with a labeling agent (e.g., biotin, etc.), onto an avidin-labeled solid phase (e.g., a plate); then measuring and comparing (i) the binding amount in the case where the protein comprising the same or substantially the same amino acid sequence as the amino acid sequence represented by SEQ ID NO: 27 or SEQ ID NO: 29, a partial peptide thereof, or a salt thereof (hereinafter the DLG1 protein or DLG3 protein) is brought in contact with the immobilized SEMA4B protein and (ii) the binding amount in the case where the DLG1 protein or DLG3 protein and a test compound are simultaneously brought in contact with the immobilized SEMA4B protein; and selecting the test compound that decreases the binding amount in the case (ii) above by at least about 20%, preferably by at least about 30% and more preferably by at least about 50%, as compared to the case (i) above, as a compound that promotes the dissociation of complex of the SEMA4B protein with the DLG1 protein or DLG3 protein, or, the test compound that increases the activity in the case (ii) above by at least about 20%, preferably by at least about 30% and more preferably by at least about 50%, as compared to the case (i) above, as a compound that inhibits the dissociation of the complex of the SEMA4B protein with the DLG1 protein or DLG3 protein (wherein, when a partial peptide of the DLG1 protein or DLG3 protein is used as said protein, there is preferably used a partial peptide having the activity of binding to the SEMA4B protein (e.g., a peptide having the 142-310 amino acid sequence in the amino acid sequence represented by SEQ ID NO: 27, a peptide having the 81-353 amino acid sequence in the amino acid sequence represented by SEQ ID NO: 29, etc.), or the like.
(47) The screening method according to (22) above, which comprises measuring and comparing (i) the binding amount in the case where the protein comprising the same or substantially the same amino acid sequence as the amino acid sequence represented by SEQ ID NO: 27 or SEQ ID NO: 29, a partial peptide thereof, or a salt thereof (hereinafter the DLG1 protein or DLG3 protein) is brought in contact with an immobilized protein comprising the same or substantially the same amino acid sequence as the amino acid sequence represented by SEQ ID NO: 26, a partial peptide thereof, or a salt thereof (hereinafter SEMA4B protein) (e.g., a partial peptide corresponding to the cytoplasmic domain of the SEMA4B protein (e.g., a peptide having the amino acid sequence represented by SEQ ID NO: 26, etc.)) and (ii) the binding amount in the case where the DLG1 protein or DLG3 protein and a test compound are brought in contact simultaneously with the immobilized SEMA4B protein; and selecting the test compound that decreases the binding amount in the case (ii) above by at least about 20%, preferably by at least about 30% and more preferably by at least about 50%, as compared to the case (i) above, as a compound that promotes the dissociation of the complex of the SEMA4B protein with the DLG1 protein or DLG3 protein, or, the test compound that increases the activity in the case (ii) above by at least about 20%, preferably by at least about 30% and more preferably by at least about 50%, as compared to the case (i) above, as a compound that inhibits the dissociation of the complex of the SEMA4B protein with the DLG1 protein or DLG3 protein (wherein the DLG1 protein or DLG3 protein used may be a protein fused to a Tag protein; in this case, the DLG1 protein or DLG3 protein may be detected/quantified using an antibody to said protein, or may be detected/quantified using an antibody to the Tag protein).
(48) The screening method according to (16), which comprises co-expressing (a) a DNA encoding a chimeric protein wherein a reporter gene binding domain is fused to the partial peptide (e.g., a peptide having the amino acid sequence represented by SEQ ID NO: 26, etc.) or a salt thereof of the protein comprising the same or substantially the same amino acid sequence as the amino acid sequence represented by SEQ ID NO: 26, a partial peptide thereof, or a salt thereof (hereinafter the SEMA4B protein) and (b) a DNA encoding a chimeric protein wherein a reporter gene transcriptional activation domain is fused to the protein comprising the same or substantially the same amino acid sequence as the amino acid sequence represented by SEQ ID NO: 27 or SEQ ID NO: 29, a partial peptide thereof, or a salt thereof (hereinafter the DLG1 protein or DLG3 protein) in yeast (e.g., *Saccharomyces cerevisiae,* more preferably *S. cerevisiae* Y190 strain) thereby to express the phenotypes of β-galactosidase as the reporter gene and histidine biosynthesis gene HIS3 by the two-hybrid action; incubating the yeast strain for a given period of time in the presence of a test compound; and selecting the compound that decreases the β-galactosidase activity of the yeast strain or the compound that converts the yeast strain into the histidine auxotroph, as a compound that promotes the dissociation of the complex of the SEMA4B protein with the DLG1 protein or DLG3 protein.

### BEST MODE FOR CARRYING OUT THE INVENTION

Hereinafter, the protein comprising the same or substantially the same amino acid sequences as the amino acid sequences represented by SEQ ID NO: 26 is sometimes briefly referred to as the SEMA4B protein. The protein comprising the same or substantially the same amino acid sequences as the amino acid sequences represented by SEQ ID NO: 27 is briefly referred to as the DLG1 protein. The protein comprising the same or substantially the same amino acid sequences as the amino acid sequences represented by SEQ ID NO: 29 is briefly referred to as the DLG3 protein.

These SEMA4B protein, DLG1 protein and DLG3 protein are collectively referred to as the protein of the present invention or the protein used in the present invention.

The protein of the present invention may be any protein derived from any cells of human and warm-blooded animals (e.g., guinea pig, rat, mouse, fowl, rabbit, swine, sheep, bovine, monkey, etc.) (e.g., hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), erythrocyte, megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; or proteins derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the proteins may also be synthetic proteins.

Throughout the present specification, the protein is represented in accordance with the conventional way of describing peptides, that is, the N-terminus (amino terminus) at the left hand and the C-terminus (carboxyl terminus) at the right hand. In the protein of the present invention, the C-terminus may be in any form of a carboxyl group (-COOH), a carboxylate (-COO-), an amide (-CONH₂) and an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂-alkyl group, e.g., benzyl, phenethyl, etc., or an α-naphthyl-C₁₋₂-alkyl group such as α-naphthylmethyl, etc.; pivaloyloxymethyl and the like.

Where the protein of the present invention contains a carboxyl group (or a carboxylate) at a position other than the C-terminus, it may be amidated or esterified and such an amide or ester is also included within the protein of the present invention. The ester group may be the same group as that described with respect to the C-terminus described above.

Furthermore, examples of the protein of the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Homology of the following amino acid sequences can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Proteins comprising the same or substantially the same amino acid sequence as the amino acid sequence represented by SEQ ID NO: 1, SEQ ID NO: 4, SEQ ID NO: 7 or SEQ ID NO: 10, etc. are used as the SEMA4B protein.

The amino acid sequence which has substantially the same amino acid sequence as that represented by SEQ ID NO: 1 includes an amino acid sequence having at least about 95% homology, preferably at least about 98% homology and more preferably at least about 99% homology, to the amino acid sequence represented by SEQ ID NO: 1; etc.

Preferably, the protein comprising substantially the same amino acid sequence as the amino acid sequence represented by SEQ ID NO: 1 is, for example, a protein comprising substantially the same amino acid sequence as the amino acid sequence represented by SEQ ID NO: 1 and having the activity substantially equivalent to that of the protein comprising the amino acid sequence represented by SEQ ID NO: 1, or the like.

The amino acid sequence which has substantially the same amino acid sequence as that represented by SEQ ID NO: 4 includes an amino acid sequence having at least about 99.9% homology to the amino acid sequence represented by SEQ ID NO: 4; etc.

Preferably, the protein comprising substantially the same amino acid sequence as the amino acid sequence represented by SEQ ID NO: 4 is, for example, a protein comprising substantially the same amino acid sequence as the amino acid sequence represented by SEQ ID NO: 4 and having the activity substantially equivalent to that of the protein comprising the amino acid sequence represented by SEQ ID NO: 4, or the like.

The amino acid sequence which has substantially the same amino acid sequence as that represented by SEQ ID NO: 7 includes an amino acid sequence having at least about 99.9% homology to the amino acid sequence represented by SEQ ID NO: 7; etc.

Preferably, the protein comprising substantially the same amino acid sequence as the amino acid sequence represented by SEQ ID NO: 7 is, for example, a protein comprising substantially the same amino acid sequence as the amino acid sequence represented by SEQ ID NO: 7 and having the activity substantially equivalent to that of the protein comprising the amino acid sequence represented by SEQ ID NO: 7, or the like.

The amino acid sequence which has substantially the same amino acid sequence as that represented by SEQ ID NO: 10 includes an amino acid sequence having at least about 99.9% homology to the amino acid sequence represented by SEQ ID NO: 10; etc.

Preferably, the protein comprising substantially the same amino acid sequence as the amino acid sequence represented by SEQ ID NO: 10 is, for example, a protein comprising substantially the same amino acid sequence as the amino acid sequence represented by SEQ ID NO: 10 and having the activity substantially equivalent to that of the protein comprising the amino acid sequence represented by SEQ ID NO: 10, or the like.

The substantially equivalent is used to mean that the property of these properties is equivalent in terms of quality (e.g., physiologically or pharmacologically). Thus, the activity described above is preferably equivalent (e.g., about 0.01 to 100 times, preferably about 0.1 to 10 times, more preferably 0.5 to 2 times), but differences in degree such as a level of the activity, quantitative factors such as a molecular weight of the protein may be present and allowable.

Examples of the SEMA4B protein include so-called muteins such as proteins comprising (i) the amino acid sequence represented by SEQ ID NO: 1, SEQ ID NO: 4, SEQ ID NO: 7 or SEQ ID NO: 10, of which at least 1 or 2 (e.g., about 1 to about 100, preferably about 1 to about 30, preferably about 1 to about 10 and more preferably several (1 to 5)) amino acids are deleted; (ii) the amino acid sequence represented by SEQ ID NO: 1, SEQ ID NO: 4, SEQ ID NO: 7 or SEQ ID NO: 10, to which at least 1 or 2 (e.g., about 1 to about 100, preferably about 1 to about 30, more preferably about 1 to about 10 and most preferably several (1 to 5)) amino acids are added; (iii) the amino acid sequence represented by SEQ ID NO: 1, SEQ ID NO: 4, SEQ ID NO: 7 or SEQ ID NO: 10, in which at least 1 or 2 (e.g., about 1 to about 100, preferably about 1 to about 30, more preferably about 1 to about 10 and most preferably several (1 to 5)) amino acids are inserted; (iv) the amino acid sequence represented by SEQ ID NO: 1, SEQ ID NO: 4, SEQ ID NO: 7 or SEQ ID NO: 10, in which at least 1 or 2 (e.g., about 1 to about 100, preferably about 1 to about 30, more preferably about 1 to about 10 and most preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Specific examples of the SEMA4B protein are a protein comprising the amino acid sequence represented by SEQ ID NO: 1, a protein comprising the amino acid sequence represented by SEQ ID NO: 4, a protein comprising the amino acid sequence represented by SEQ ID NO: 7, a protein comprising the amino acid sequence represented by SEQ ID NO: 10, and the like.

The partial peptide of the SEMA4B protein may be any peptide as long as it is a partial peptide of the SEMA4B protein described above and preferably has the property equivalent to that of the SEMA4B protein.

For example, there are used peptides containing, e.g., at least 20, preferably at least 50, more preferably at least 70, much more preferably at least 100, and most preferably at least 200 amino acids in the constituent amino acid sequence of the SEMA4B protein, etc.

Specific examples include peptides having the amino acid sequence represented by SEQ ID NO: 26, etc.

The amino acid sequence which has substantially the same amino acid sequence as that represented by SEQ ID NO: 27 or SEQ ID NO: 29 includes an amino acid sequence having at least about 70% homology, preferably at least about 80% homology, more preferably at least about 90% homology and most preferably at least about 95% homology to the amino acid sequence represented by SEQ ID NO: 27, SEQ ID NO: 29; etc.

Preferably, the protein comprising substantially the same amino acid sequence as the amino acid sequence represented by SEQ ID NO: 27 or SEQ ID NO: 29 is, for example, a protein comprising substantially the same amino acid sequence as the amino acid sequence represented by SEQ ID NO: 27 or SEQ ID NO: 29 and having the activity substantially equivalent to that of the protein comprising the amino acid sequence represented by SEQ ID NO: 27 or SEQ ID NO: 29, or the like.

The substantially equivalent is used to mean that the property of these properties is equivalent in terms of quality (e.g., physiologically or pharmacologically). Thus, the activity described above is preferably equivalent (e.g., about 0.01 to 100 times, preferably about 0.1 to 10 times, more preferably 0.5 to 2 times), but differences in degree such as a level of the activity, quantitative factors such as a molecular weight of the protein may be present and allowable.

Examples of the DLG1 protein include so-called muteins such as proteins comprising (i) the amino acid sequence represented by SEQ ID NO: 27, of which at least 1 or 2 (e.g., about 1 to about 100, preferably about 1 to about 30, more preferably about 1 to about 10 and most preferably several (1 to 5)) amino acids are deleted; (ii) the amino acid sequence represented by SEQ ID NO: 27, to which at least 1 or 2 (e.g., about 1 to about 100, preferably about 1 to about 30, more preferably about 1 to about 10 and most preferably several (1 to 5)) amino acids are added; (iii) the amino acid sequence represented by SEQ ID NO: 27, in which at least 1 or 2 (e.g., about 1 to about 100, preferably about 1 to about 30, more preferably about 1 to about 10 and most preferably several (1 to 5)) amino acids are inserted; (iv) the amino acid sequence represented by SEQ ID NO: 27, in which at least 1 or 2 (e.g., about 1 to about 100, preferably about 1 to about 30, more preferably about 1 to about 10 and most preferably several (1 to 5)) amino acids are substituted by other amino acids; or (v) a combination of these amino acid sequences; and the like.

Examples of the DLG3 protein include so-called muteins such as proteins comprising (i) the amino acid sequence represented by SEQ ID NO: 29, of which at least 1 or 2 (e.g., about 1 to about 100, preferably about 1 to about 30, more preferably about 1 to about 10 and most preferably several (1 to 5)) amino acids are deleted; (ii) the amino acid sequence represented by SEQ ID NO: 29, to which at least 1 or 2 (e.g., about 1 to about 100, preferably about 1 to about 30, more preferably about 1 to about 10 and most preferably several (1 to 5)) amino acids are added; (iii) the amino acid sequence represented by SEQ ID NO: 29, in which at least 1 or 2 (e.g., about 1 to about 100, preferably about 1 to about 30, more preferably about 1 to about 10 and most preferably several (1 to 5)) amino acids are inserted; (iv) the amino acid sequence represented by SEQ ID NO: 29, in which at least 1 or 2 (e.g., about 1 to about 100, preferably about 1 to about 30, more preferably about 1 to about 10 and most preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Specific examples of the DLG1 protein include a protein comprising the amino acid sequence represented by SEQ ID NO: 27, etc.

Specific examples of the DLG3 protein include a protein comprising the amino acid sequences represented by SEQ ID NO: 29, etc.

The partial peptide of the DLG1 protein or DLG3 protein may be any peptide as long as it is a partial peptide of the DLG1 protein or DLG3 protein described above and preferably has the property equivalent to that of the DLG1 protein or DLG3 protein.

For example, there are used peptides containing, e.g., at least 20, preferably at least 50, more preferably at least 70, much more preferably at least 100, and most preferably at least 200 amino acids in the constituent amino acid sequence of the DLG1 protein or DLG3 protein, etc.

Specific examples of the partial peptides of the DLG1 protein include a partial peptide having the 142-310 amino acid sequence in the amino acid sequence represented by SEQ ID NO: 27, and the like.

Specific examples of the partial peptides of the DLG3 protein include a partial peptide having the 81-353 amino acid sequence in the amino acid sequence represented by SEQ ID NO: 29, and the like.

The partial peptide of the protein of the present invention (the partial peptide used in the present invention) may be peptides containing the amino acid sequence, of which at least 1 or 2 (preferably about 1 to about 10, more preferably several (1 to 5)) amino acids may be deleted; peptides, to which at least 1 or 2 (preferably about 1 to about 20, more preferably about 1 to about 10 and most preferably several (1 to 5)) amino acids may be added; peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 20, more preferably about 1 to about 10, much more preferably several and most preferably about 1 to about 5) amino acids may be substituted by other amino acids.

In the partial peptide used in the present invention, the C-terminus may be in any form of a carboxyl group (-COOH), carboxylate (-COO⁻), an amide (-CONH₂) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated peptides such as so-called glycopeptides having sugar chains; etc., as in the protein of the present invention described above.

The partial peptide used in the present invention may also be used as an antigen for producing antibodies.

For salts of the protein of the present invention or its partial peptide, preferred are physiologically acceptable salts with acids (e.g., organic acids, inorganic acids) or bases (e.g., alkali metal salts), especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or its partial peptide of the present invention, or salts thereof can be manufactured by REFERENCE EXAMPLES later described, or from the human or mammalian cells or tissues described above by publicly known methods for purification of receptor proteins, or by culturing transformants bearing DNA encoding the protein. Alternatively, the protein or salts thereof can be manufactured by peptide synthesis or its modifications later described.

Where they are manufactured from human or mammalian tissues or cells, human or mammalian tissues or cells are homogenized, and then extracted with an acid, etc., and the extract is isolated and purified by a combination of chromatography techniques such as reverse phase chromatography, ion exchange chromatography, and the like.

To synthesize the protein or its partial peptide of the present invention, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the protein or partial peptide is cut out from the resin and at the same time, the protecting groups are removed. Then, intramolecular disulfide bond-forming reaction is performed in a highly diluted solution to obtain the objective protein or partial peptide or its amides

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20 to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia. The elimination of the protecting group by the acid treatment described above is carried out generally at a temperature of approximately -20 to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups, activation of functional groups involved in the reaction, or the like may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (i) to (v) below.
(i) M. Bodanszky & M. A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Examples of the DNA encoding the SEMA4B protein may be any one of:
(i) a DNA comprising the base sequence represented by SEQ ID NO: 2, or a DNA comprising a base sequence hybridizable to the base sequence represented by SEQ ID NO: 2 under high stringent conditions and encoding a protein which has the properties of substantially the same nature as those of the protein comprising the amino acid sequence represented by SEQ ID NO: 1 described above,
(ii) a DNA comprising the base sequence represented by SEQ ID NO: 5, or a DNA comprising a base sequence hybridizable to the base sequence represented by SEQ ID NO: 5 under high stringent conditions and encoding a protein which has the properties of substantially the same nature as those of the protein comprising the amino acid sequence represented by SEQ ID NO: 4 described above,
(iii) a DNA comprising the base sequence represented by SEQ ID NO: 8, or a DNA comprising a base sequence hybridizable to the base sequence represented by SEQ ID NO: 8 under high stringent conditions and encoding a protein which has the properties of substantially the same nature as those of the protein comprising the amino acid sequence represented by SEQ ID NO: 7 described above,
(iv) a DNA comprising the base sequence represented by SEQ ID NO: 11, or a DNA comprising a base sequence hybridizable to the base sequence represented by SEQ ID NO: 11 under high stringent conditions and encoding a protein which has the properties of substantially the same nature as those of the protein comprising the amino acid sequence represented by SEQ ID NO: 10 described above,

As the DNA that is hybridizable to the base sequence represented by SEQ ID NO: 2 under high stringent conditions, there are employed, for example, DNAs comprising base sequences having at least about 95% homology, preferably at least about 98% homology, and more preferably at least about 99% homology, to the base sequence represented by SEQ ID NO: 2; and the like.

As the DNA that is hybridizable to the base sequence represented by SEQ ID NO: 5 under high stringent conditions, there are employed, for example, DNAs comprising base sequences having at least about 99.9% homology to the base sequence represented by SEQ ID NO: 5; and the like.

As the DNA that is hybridizable to the base sequence represented by SEQ ID NO: 8 under high stringent conditions, there are employed, for example, DNAs comprising base sequences having at least about 99.9% homology to the base sequence represented by SEQ ID NO: 8; and the like.

As the DNA that is hybridizable to the base sequence represented by SEQ ID NO: 11 under high stringent conditions, there are employed, for example, DNAs comprising base sequences having at least about 99.9% homology to the base sequence represented by SEQ ID NO: 11; and the like.

Homology of the base sequences can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, there are employed: (i) a DNA comprising the base sequence represented by SEQ ID NO: 2, a DNA comprising the base sequence represented by SEQ ID NO: 3, etc. as the DNA encoding the protein comprising the amino acid sequence represented by SEQ ID NO: 1; (ii) a DNA comprising the base sequence represented by SEQ ID NO: 5, a DNA comprising the base sequence represented by SEQ ID NO: 6, etc. as the DNA encoding the protein comprising the amino acid sequence represented by SEQ ID NO: 4; (iii) a DNA comprising the base sequence represented by SEQ ID NO: 8, a DNA comprising the base sequence represented by SEQ ID NO: 9, etc. as the DNA encoding the protein comprising the amino acid sequence represented by SEQ ID NO: 7; (iv) a DNA comprising the base sequence represented by SEQ ID NO: 11, a DNA comprising the base sequence represented by SEQ ID NO: 12, etc. as the DNA encoding the protein comprising the amino acid sequence represented by SEQ ID NO: 10; and the like.

As the DNA encoding the DLG1 protein, there are employed, for example, a DNA comprising the base sequence represented by SEQ ID NO: 28, or a DNA comprising a base sequence hybridizable to the base sequence represented by SEQ ID NO: 28 under high stringent conditions and encoding a protein having the activities of substantially the same nature as those of the protein comprising the amino acid sequences represented by SEQ ID NO: 27, and the like.

As the DNA encoding the DLG3 protein, there are employed, for example, a DNA comprising the base sequence represented by SEQ ID NO: 30, or a DNA comprising a base sequence hybridizable to the base sequence represented by SEQ ID NO: 30 under high stringent conditions and encoding a protein having the activities of substantially the same nature as those of the protein comprising the amino acid sequences represented by SEQ ID NO: 29, and the like.

Examples of the DNA hybridizable to the DNA containing the base sequence represented by SEQ ID NO: 28 or SEQ ID NO: 30 under highly stringent conditions include a DNA containing a base sequence having at least about 70% homology, preferably at least about 80% homology, more preferably at least about 90% homology and most preferably at least about 95% homology, to the base sequence represented by SEQ ID NO: 28 or SEQ ID NO: 30, etc.

Homology of the base sequences can be measured under the following conditions (an expectation value = 10; gaps are allowed; filtering = ON, match score = 1, mismatch score = 3) using a homology scoring algorithm NCBI BLAST (National Center for Biotechnology Information Basic Local Alignment Search Tool).

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

More specifically, as a DNA encoding the protein comprising the amino acid sequence represented by SEQ ID NO: 27, there may be employed a DNA comprising the base sequence represented by SEQ ID NO: 28; etc., as a DNA encoding the protein comprising the amino acid sequence represented by SEQ ID NO: 29, there may be employed a DNA comprising the base sequence represented by SEQ ID NO: 30; etc.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide of the SEMA4B protein, there are employed, for example, a DNA comprising a part of the DNA having the base sequence represented by SEQ ID NO: 2, SEQ ID NO: 5, SEQ ID NO: 8 or SEQ ID NO: 11, or a DNA comprising a base sequence hybridizable to the base sequence represented by SEQ ID NO: 2, SEQ ID NO: 5, SEQ ID NO: 8 or SEQ ID NO: 11 I under high stringent conditions and comprising a part of DNA encoding a protein having the activities of substantially the same nature as those of the SEMA4B protein, and the like.

The DNA hybridizable to the base sequence represented by SEQ ID NO: 2, SEQ ID NO: 5, SEQ ID NO: 8 or SEQ ID NO: 11 indicates the same meaning as described above.

Methods for the hybridization and the high stringent conditions that can be used are the same as those described above.

As the DNA encoding the partial peptide of the DLG1 protein, there are employed, for example, a DNA comprising a part of the DNA having the base sequence represented by SEQ ID NO: 28, or a DNA comprising a base sequence hybridizable to the base sequence represented by SEQ ID NO: 28 under high stringent conditions and comprising a part of DNA encoding a protein having the activities of substantially the same nature as those of the protein represented by SEQ ID NO: 27, and the like.

As the DNA encoding the partial peptide of the DLG3protein, there are employed, for example, a DNA comprising a part of the DNA having the base sequence represented by SEQ ID NO: 30, or a DNA comprising a base sequence hybridizable to the base sequence represented by SEQ ID NO: 30 under high stringent conditions and comprising a part of DNA encoding a protein having the activities of substantially the same nature as those of the protein represented by SEQ ID NO: 29, and the like.

The DNA hybridizable to the base sequence represented by SEQ ID NO: 28 or SEQ ID NO: 30 indicates the same meaning as described above.

Methods for the hybridization and the high stringent conditions that can be used are the same as those described above.

For cloning of DNAs that completely encode the protein or partial peptide of the present invention (hereinafter sometimes merely referred to as the protein of the present invention in the description of cloning ofDNAs encoding the protein and partial peptide and their expression), the DNA can be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the protein of the present invention. The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol.

Substitution of the base sequence of DNA can be effected by publicly known methods such as the ODA-LA PCR method, the Gapped duplex method, the Kunkel method, etc., or its modification, using PCR, a publicly known kit available as Mutan^{™}-super Express Km (manufactured by Takara Shuzo Co., Ltd.) or Mutan^{™}-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus *Escherichia,* preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus *Escherichia* is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus *Escherichia,* bacteria belonging to the genus *Bacillus,* yeast, insect cells, insects, animal cells, etc.

Specific examples of the bacteria belonging to the genus *Escherichia* include *Escherichia coli* K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB 101 [Journal of Molecular Biology, 41, 459 (1969)], C600 [Genetics, 39, 440 (1954)], etc.

Examples of the bacteria belonging to the genus *Bacillus* include *Bacillus subtilis* MI114 [Gene, 24, 255 (1983)], 207-21 [Journal of Biochemistry, 95, 87 (1984)], etc.

Examples of yeast include *Saccharomyces cereviseae* AH22, AH22R⁻, NA87-11A, DKD-5D, 20B-12, *Schizosaccharomyces pombe* NCYC1913, NCYC2036, *Pichia pastoris* KM71, etc.

Examples of insect cells include, for the virus AcNPV, *Spodoptera frugiperda* cell (Sf cell), MG1 cell derived from mid-intestine of *Trichoplusia ni,* High Five^{™} cell derived from egg of *Trichoplusia ni,* cells derived from *Mamestra brassicae,* cells derived from *Estigmena acrea,* etc.; and for the virus BmNPV, *Bombyx mori* N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of *Bombyx mori* can be used [Maeda et al., Nature, 315, 592 (1985)].

Examples of animal cells include monkey cell COS-7, Vero, Chinese hamster cell CHO (hereinafter abbreviated as CHO cell), dhfr gene-deficient Chinese hamster cell CHO (hereinafter abbreviated as CHO (dhfr⁻) cell), mouse L cell, mouse AtT-20, mouse myeloma cell, mouse ATDC5 cell, rat GH3, human FL cell, etc.

Bacteria belonging to the genus *Escherichia* can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus *Escherichia* or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus *Escherichia* is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus *Escherichia* are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100^{™}, etc. When the protein is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

Hereinafter, the SEMA4B protein or its partial peptide, or salts thereof are merely referred to as the SEMA4B protein; the DLG1 protein or its partial peptide, or salts thereof are merely referred to as the DLG1 protein; and the DLG3 protein or its partial peptide, or salts thereof are merely referred to as the DLG3 protein, respectively.
(1) The complex comprising the SEMA4B protein and the DLG1 protein (preferably, the complex where the SEMA4B protein binds to the DLG1 protein), (2) the complex comprising the SEMA4B protein and DLG3 protein (preferably, the complex where the SEMA4B protein binds to the DLG3 protein), (3) the complex comprising the SEMA4B protein, DLG1 protein and DLG3 protein (preferably, the complex where the SEMA4B protein binds to the DLG1 protein and the DLG3 protein) are sometimes collectively referred to as the complex of the present invention.

The complex of the present invention may contain, in addition to the SEMA4B protein, the DLG1 protein or DLG3 protein, a protein other than those described above, a peptide, RNA, a nucleic acid, a lipid, sugar, an amide group, a phosphate group, etc. For example, the SEMA4B protein, the DLG1 protein and the DLG3 protein may be amidated, may contain a phosphorylated amino acid or may contain a lipid (e.g., myristic acid, etc.)-bound amino acid, and are not limited even though these proteins may undergo any post-translational modification. The region which undergoes post-translational modification includes, for example, the region corresponding to the amino acid sequence represented by SEQ ID NO: 26, etc.

The complex of the present invention includes, e.g., (1) the complex wherein the cytoplasmic domain of the SEMA4B protein (e.g., a peptide having the amino acid sequence represented by SEQ ID NO: 26, etc.) binds to the DLG1 protein (preferably, a region having the activity of binding to the SEMA4B protein), (2) the complex wherein the cytoplasmic domain of the SEMA4B protein (e.g., a peptide having the amino acid sequence represented by SEQ ID NO: 26, etc.) binds to the DLG3 protein (preferably, a region having the activity of binding to the SEMA4B protein), (3) the complex wherein the cytoplasmic domain of the SEMA4B protein (e.g., a peptide having the amino acid sequence represented by SEQ ID NO: 26, etc.) binds to the DLG1 protein (preferably, a region having the activity of binding to the SEMA4B protein) and the DLG3 protein (preferably, a region having the activity of binding to the SEMA4B protein), and the like.

Examples of the region having the activity of binding to the SEMA4B protein of the DLG1 protein include the 142-310 amino acid sequence in the amino acid sequence represented by SEQ ID NO: 27.

The region having the activity of binding to the SEMA4B protein of the DLG3 protein includes, for example, the 81-353 amino acid sequence in the amino acid sequence represented by SEQ ID NO: 29.

The antibodies to the protein or its partial peptide or its salts of the present invention (hereinafter they are sometimes merely referred to as the protein of the present invention in the description of the antibodies), and the antibodies to the complex of the present invention (hereinafter they are sometimes collectively referred to as the antibodies of the present invention) may be any of polyclonal antibodies and monoclonal antibodies, as long as they are capable of recognizing the protein of the present invention or the complex of the present invention.

Furthermore, the antibodies described herein further include the antibody that inhibits the formation of the complex of the present invention, the antibody that promotes the formation of the complex of the present invention, the antibody that promotes the dissociation of the complex of the present invention, and the antibody that inhibits the dissociation of the complex of the present invention As these antibodies, the antibodies to the SEMA4B protein, the DLG1 protein or DLG3 protein, particularly, the antibodies to the SEMA4B protein described in WO2004/58817, etc. are preferably used.

Specifically, the antibodies to the SEMA4B protein used include antibodies capable of recognizing the following:
(1) an amino acid sequence having the 402^{nd} - 412^{th} amino acids of the sequence of SEQ ID NO: 1, or an amino acid sequence having said amino acid sequence with Cys being added at the C terminus;
(2) an amino acid sequence having the 582^{nd} - 596^{th} amino acids of the sequence of SEQ ID NO: 1,
(3) an amino acid sequence having the 781^{st} - 794^{th} amino acids of the sequence of SEQ ID NO: 1, or an amino acid sequence having said amino acid sequence with Cys being added at the C terminus;
(4) an amino acid sequence having the 797^{th} - 809^{th} amino acids of the sequence of SEQ ID NO: 1, or an amino acid sequence having said amino acid sequence with Cys being added at the C terminus; and the like.

The antibodies of the present invention can be manufactured by publicly known methods for manufacturing antibodies or antisera, using as antigens the protein of the present invention or the complex of the present invention, or a part thereof (hereinafter sometimes briefly referred to as the antigen protein),

### [Preparation of Monoclonal Antibody]

### (a) Preparation of Monoclonal Antibody-Producing Cells

The antigen protein is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused to myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, and (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20: 1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of Monoclonal Antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of Polyclonal Antibody]

The polyclonal antibody of the present invention can be manufactured by publicly known methods or modifications thereof. For example, a warm-blooded animal is immunized with an immunogen (protein antigen) per se, or with a conjugate of immunogen and a carrier protein formed in a manner similar to the method described above for the manufacture of monoclonal antibodies. The product containing the antibody to the antigen protein is collected from the immunized animal followed by separation and purification of the antibody.

In the conjugate of immunogen and a carrier protein used to immunize a warm-blooded animal, the type of carrier protein and the mixing ratio of carrier to hapten may be any type and in an), ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

Hereinafter, the protein of the present invention or partial peptide, or a salt thereof (hereinafter sometimes briefly referred to as the protein of the present invention), the DNA encoding the protein or partial peptide of the present invention (hereinafter sometimes briefly referred to as the DNA of the present invention), the antibodies of the present invention, the complexes of the present invention, etc. are described below in terms of their applications.

### [1] Screening of drug candidate compounds for disease

The SEMA4B protein is overexpressed in cancer tissues. In addition, when the function (activity, expression, etc.) of the SEMA4B protein is inhibited, cancer cells induce apoptosis.

The DLG1 protein or DLG3 protein binds to the SEMA4B protein at the cytoplasmic domain and is considered to play the function of the SEMA4B protein.

Thus, the substance (e.g., a compound or its salt, an antibody) that inhibits the formation of the complex of the present invention, for example, the substance (e.g., a compound or its salt, an antibody) that inhibits (1) the binding of the SEMA4B protein to the DLG1 protein, or (2) the binding of the SEMA4B protein to the DLG3 protein, has an apoptosis induction effect of cancer cells and can be used as an agent for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.), etc., as an apoptosis promoter of cancer cells, etc.

The substance (e.g., a compound or its salt, an antibody) that promotes the formation of the complex of the present invention, for example, the substance (e.g., a compound or its salt, an antibody) that promotes (1) the binding of the SEMA4B protein to the DLG1 protein, (2) the binding of the SEMA4B protein to the DLG3 protein has a neuronal apoptosis suppressing activity and can be used as an agent for preventing/treating, for example, neurodegenerative diseases (e.g., Alzheimer's disease (familial Alzheimer's disease, juvenile Alzheimer's disease, sporadic Alzheimer's disease, etc.)), as a neuronal apoptosis inhibitor (suppresser), etc.

The substance (e.g., a compound or its salt, an antibody) that promotes the dissociation of the complex of the present invention, for example, the substance (e.g., a compound or its salt, an antibody) that promotes (3) the dissociation of the complex of the SEMA4B protein with the DLG1 protein, or (4) the dissociation of the complex of the SEMA4B protein and the DLG3 protein has a neuronal apoptosis induction activity and can be used as an agent for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.), as an apoptosis promoter of cancer cells, etc.

The substance (e.g., a compound or its salt, an antibody) that inhibits the dissociation of the complex of the present invention, for example, the substance (e.g., a compound or its salt, an antibody) that inhibits (3) the dissociation of the complex of the SEMA4B protein with the DLG1 protein, or (4) the dissociation of the complex of the SEMA4B protein and the DLG3 protein has a neuronal apoptosis suppressing activity and can be used as an agent for preventing/treating, for example, neurodegenerative diseases (e.g., Alzheimer's disease (familial Alzheimer's disease, juvenile Alzheimer's disease, sporadic Alzheimer's disease, etc.)), as a neuronal apoptosis inhibitor (suppresser), etc.

Accordingly, the protein of the present invention is useful as a reagent for screening the compound or its salts that inhibit or promote the aforesaid binding (1)-(2), or the compound or its salts that promote or inhibit the dissociation of the aforesaid complex of (3)-(4).

That is, the present invention provides a method of screening the compound or its salt that inhibits or promotes the binding in (1)-(2) described above, or the compound or its salt that promotes or inhibits the dissociation of the complex in (3)-(4) described above, which comprises using the protein of the present invention.

For the screening method of the present invention, the protein of the present invention is employed, or a peptide corresponding to the cytoplasmic domain of SEMA4B protein may be employed. Furthermore, a cell (preferably, a transformant (yeast, a cell such as animal cell, etc.) transformed by the DNA encoding the protein of the present invention) capable of producing the protein of the present invention may also be used for the screening method of the present invention. The transformant may be a transformant transformed (a) by DNA encoding the SEMA4B protein (e.g., DNA encoding the peptide corresponding to the cytoplasmic domain of the SEMA4B protein) and (b) by DNA encoding the DLG1 protein or the DLG3 protein.

### [1a] Screening by in vitro binding test

The DLG1 protein or DLG3 protein is immobilized onto a solid phase (e.g., an EIA plate), using the antibody to the DLG1 protein or DLG3 protein. Alternatively, the DLG1 protein or DLG3 protein is fused to a Tag protein (e.g., His-Tag, GST (glutathione-S-transferase), etc.) and then immobilized onto a solid phase.

In the case that the partial peptide of the protein is used as the DLG1 protein or DLG3 protein, it is preferred to use a partial peptide having the activity of binding to the SEMA4B protein (e.g., a peptide having the 142-310 amino acid in the amino acid sequence represented by SEQ ID NO: 27, a peptide having the 81-353 amino acid in the amino acid sequence represented by SEQ ID NO: 29, etc.), or the like.

In immobilization of the tagged protein onto a solid phase, nickel is used for His-Tag and, for GST glutathione is used.
(i) The binding amount in the case where the SEMA4B protein (e.g., a partial peptide corresponding to the cytoplasmic domain (e.g., a peptide having the amino acid sequence represented by SEQ ID NO: 26, etc.)) labeled with a labeling agent (e.g., biotin, etc.) is brought in contact with the immobilized DLG1 protein or DLG3 protein and (ii) the binding amount in the case where the SEMA4B protein (e.g., a partial peptide corresponding to cytoplasmic domain (e.g., a peptide having the amino acid sequence represented by SEQ ID NO: 26, etc.)) and a test compound are simultaneously brought in contact with the immobilized DLG1 protein or DLG3 protein are determined and compared between (i) and (ii).

The binding amount is determined by publicly known methods, e.g., by measuring the immobilized SEMA4B protein using an assay kit commercially available or the antibody to the SEMA4B protein.

Examples of the test compound include peptides, proteins, non-peptide compounds, synthetic compounds, fermentation products, cell extracts, plant extracts, animal tissue extracts, etc.

For example, when a test compound decreases the binding amount in the case (ii) described above by at least about 20%, preferably at least 30% and more preferably at least about 50%, as compared to the case (i) above, the test compound is selected as the compound that inhibits the binding of the SEMA4B protein to the DLG1 protein or DLG3 protein (hereinafter sometimes briefly referred to as a binding inhibitor); when a test compound increases the binding amount in the case (ii) described above by at least about 20%, preferably at least 30% and more preferably at least about 50%, as compared to the case (i) above, the test compound is selected as the compound that promotes the binding of the SEMA4B protein to the DLG1 protein or DLG3 protein (hereinafter sometimes briefly referred to as a binding promoter).

Further when a test compound decreases the binding amount in the case (ii) described above by at least about 20%, preferably at least 30% and more preferably at least about 50%, as compared to the case (i) above, the test compound is selected as the compound that promotes the dissociation of the complex of the SEMA4B protein with the DLG1 protein or DLG3 protein (hereinafter sometimes briefly referred to as a dissociation promoter), when a test compound increases the binding amount in the case (ii) described above by at least about 20%, preferably at least 30% and more preferably at least about 50%, as compared to the case (i) above, the test compound is selected as the compound that inhibits the dissociation of the complex of the SEMA4B protein with the DLG1 protein or DLG3 protein (hereinafter sometimes briefly referred to as a dissociation inhibitor).

Furthermore, the SEMA4B protein (e.g., a partial peptide corresponding to the cytoplasmic domain of the SEMA4B protein (e.g., a peptide having the amino acid sequence represented by SEQ ID NO: 26, etc.), etc.) may be immobilized onto a solid phase and brought in contact with the DLG1 protein or DLG3 protein, and the binding amount is determined in a similar way. In immobilization of the SEMA4B protein (e.g., a partial peptide corresponding to the cytoplasmic domain of the SEMA4B protein (e.g., a peptide having the amino acid sequence represented by SEQ ID NO: 26, etc.), etc.) onto a solid phase, it is preferred to use the SEMA4B protein labeled with a labeling agent (e.g., biotin, etc.) and an avidin-labeled solid phase (e.g., a plate).
(iii) The binding amount in the case where the DLG1 protein or DLG3 protein is brought in contact with the immobilized SEMA4B protein (e.g., a partial peptide corresponding to the cytoplasmic domain (e.g., a peptide having the amino acid sequence represented by SEQ ID NO: 26, etc.)) and (iv) the binding amount in the case where the DLG1 protein or DLG3 protein and a test compound are simultaneously brought in contact with the immobilized SEMA4B protein (e.g., a partial peptide corresponding to cytoplasmic domain (e.g., a peptide having the amino acid sequence represented by SEQ ID NO: 26, etc.)) are determined and compared between (iii) and (iv).

The binding amount is determined by publicly known methods, for example, by measuring the immobilized the DLG1 protein or DLG3 protein using the antibody to said protein.

Examples of the test compound include peptides, proteins, non-peptide compounds, synthetic compounds, fermentation products, cell extracts, plant extracts, animal tissue extracts, etc.

In this method, the DLG1 protein or DLG3 protein used may be the protein fused to a Tag protein. In this case, the DLG1 protein or DLG3 protein may be detected and quantified by antibodies to the protein, or may be detected and quantified by antibodies to the Tag protein.

For example, when a test compound decreases the binding amount in the case (iv) described above by at least about 20%, preferably at least 30% and more preferably at least about 50%, as compared to the case (iii) above, the test compound is selected as the binding inhibitor, when a test compound increases the binding amount in the case (iv) described above by at least about 20%, preferably at least 30% and more preferably at least about 50%, as compared to the case (iii) above, the test compound is selected as the binding promoter.

Furthermore, when a test compound decreases the binding amount in the case (iv) described above by at least about 20%, preferably at least 30% and more preferably at least about 50%, as compared to the case (iii) above, the test compound is selected as the dissociation promoter, when a test compound increases the binding amount in the case (iv) described above by at least about 20%, preferably at least 30% and more preferably at least about 50%, as compared to the case (iii) above, the test compound is selected as the dissociation inhibitor.

### [1b] Screening by the two-hybrid method

### [16-1] Screening by the yeast two-hybrid method

When (a) a DNA encoding a chimeric protein wherein a reporter gene binding domain is fused to the partial peptide (e.g., a peptide having the amino acid sequence represented by SEQ ID NO: 26, etc.) corresponding to the cytoplasmic domain of the SEMA4B protein and (b) a DNA encoding a chimeric protein wherein a reporter gene transcriptional activation domain is fused to the DLG1 protein or DLG3 protein, are co-expressed in yeast (e.g., *Saccharomyces cerevisiae,* preferably *S. cerevisiae* Y190 strain), the phenotypes of β-galactosidase gene and histidine synthetic gene HIS3, which are reporter genes, are expressed. The yeast strain is cultured for a given period of time in the presence of a test compound, and the test compound that reduces the β-galactosidase activity in the yeast strain or can convert the yeast strain into a histidine auxotroph is selected as the binding inhibitor or the dissociation promoter.

The β-galactosidase activity can be measured according to a publicly known method using as a substrate X-Gal (5-bromo-4-chloro-3 -indolyl-β-D-galactopyranoside), ONPG (o-nitrophenyl β-D-galactopyranoside) or CPRG (chlorophenyl red-β-D-galactopyranoside). Expression of the HIS3 phenotype can be measured by culturing the yeast in the minimum medium free of histidine.

Among the compounds screened, such compounds that have cytotoxicity and inhibit the activity of the reporter gene product itself by interaction with the reporter gene product can be excluded as pseudo-positive compounds.

### [1b-2] Screening by the animal cell two-hybrid method

In an animal cell (e.g., CHO cell), a reporter gene, (e.g., chloramphenicol acetyltransferase (CAT) gene or fire fly luciferase gene, etc.) is introduced. The transcription regulatory region of the reporter gene is designed to induce expression of the reporter gene in an animal cell, by introducing the GAL4-GAL1 transcription regulatory system of the yeast two-hybrid system into the animal cell, using as the transcription regulatory region of the reporter gene, e.g., a promoter functioning in an animal cell (e.g., a minimal promoter (TATA box, etc.) derived from adenovirus E1b, etc.) and linked to, e.g., the transcription activating sequence (UAS) of GAL1 at the downstream. Co-expression of (a) a DNA encoding a chimeric protein wherein the GAL4-DNA-bound domain is fused to the partial peptide (e.g., a peptide having the amino acid sequence represented by SEQ ID NO: 26, etc.) corresponding to the cytoplasmic domain of the SEMA4B protein and (b) a DNA encoding a chimeric protein wherein a herpes simplex-derived VP16 protein is fused to the DLG1 protein or DLG3 protein, are co-expressed in the obtained cells, gives an animal cell strain capable of expressing the reporter gene by the two-hybrid effect. This cell strain is cultured for a given period of time in the presence of a test compound, the activity of the reporter gene product is assayed, and the compound that reduces the activity is selected as the binding inhibitor or the dissociation promoter.

The animal cell strain can be cultured in a manner similar to incubation of the transformant described above, a host to which is an animal cell. The activity of the reporter gene product (e.g., CAT, luciferase, etc.) can be assayed using a commercially available kit in accordance with a publicly known method.

In the compounds thus screened, these compounds which are cytotoxic and inhibit the activity of the reporter gene product through interaction, etc. with the reporter gene product are excluded as pseudo-positive compounds.

Examples of the test compounds include peptides, proteins, non-peptide compounds, synthetic compounds, fermentation products, cell extracts, plant extracts, animal tissue extracts, etc.

The screening kit of the present invention comprises the DLG1 protein or DLG3 protein, and may further contain the SEMA4B protein (e.g., partial peptide corresponding to cytoplasmic domain, etc.). Also, the screening kit of the present invention comprises a cell (preferably a transformant (e.g., yeast, a cell such as an animal cell, etc.) transformed by a DNA encoding the protein of the present invention) capable of producing the protein of the present invention. The transformant may be transformants, which are transformed by a DNA encoding the DLG1 protein or DLG3 protein and by a DNA encoding the SEMA4B protein (e.g., a partial peptide corresponding to the cytoplasmic domain, etc.).

Examples of the compounds or salts thereof obtained by using the screening method or screening kit of the present invention are the test compounds described above (e.g., peptides, proteins, non-peptide compounds, synthetic compounds, fermentation products, cell extracts, vegetable extracts, animal tissue extracts, blood plasma, etc.) and include the compounds that inhibit or promote the binding of the SEMA4B protein to the DLG1 protein or DLG3 protein, or the compounds that promote or inhibit the dissociation of the complex of the SEMA4B protein with the DLG1 protein or DLG3 protein.

The salts of these compounds employed are salts similar to those of the protein of the present invention described above.

The compound that inhibits the binding of the SEMA4B protein to the DLG1 protein or DLG3 protein is useful as an agent for the prevention/treatment of, for example, cancer (e.g., colorectal cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.), or as an apoptosis promoter of cancer cells.

The compound that promotes the binding of the SEMA4B protein to the DLG1 protein or DLG3 protein is useful as an agent for the prevention/treatment of, e.g., neurodegenerative diseases(e.g., Alzheimer's diseases (familial Alzheimer's disease, juvenile Alzheimer's disease, sporadic Alzheimer's disease, etc.)), or as a neuronal apoptosis inhibitor (suppresser).

The compound that promotes the dissociation of the complex of the SEMA4B protein with the DLG1 protein or DLG3 protein is useful as an agent for preventing/treating, for example, cancer (e.g., colorectal cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.), or as a neuronal apoptosis promoter.

The compound that inhibits the dissociation of the complex of the SEMA4B protein with the DLG1protein or DLG3 protein is useful as an agent for preventing/treating, for example, neurodegenerative diseases (e.g., Alzheimer's diseases (familial Alzheimer's disease, juvenile Alzheimer's disease, sporadic Alzheimer's disease, etc.)), or as a neuronal apoptosis inhibitor (suppresser).

Where the compounds or their salts obtained by using the screening method or screening kit of the present invention are used as the preventive/therapeutic agents described above, these compounds can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the compound or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid compound or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components, unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the above compound (preferably, the binding inhibitor or the dissociation promoter) or its salt may vary depending upon its action, target disease, subject to be administered, route of administration, etc. For example, when the compound is orally administered for the purpose of treating, e.g., breast cancer, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the compound or its salt may vary depending upon subject to be administered, target disease, etc. When the compound or its salt is administered to an adult (as 60 kg body weight) in the form of an injectable preparation for the purpose of treating, e.g., breast cancer, it is advantageous to administer the compound or its salt at cancerous lesions by way of injection in a daily dose of about 0.01 to about 30 mg, preferably about 0.1 to about 20 mg, and more preferably about 0.1 to about 10 mg. For other animal species, the corresponding dose as converted per 60 kg weight can be administered.

### [2] Quantification for the complex of the present invention

The antibody of the present invention is capable of specifically recognizing the protein of the present invention or the complex of the present invention and therefore can be used for quantification of the protein of the present invention or the complex of the present invention in a test sample fluid, in particular, for quantification by sandwich immunoassay; etc.

That is, the present invention provides:
(i) a method of quantifying the protein of the present invention or the complex of the present invention in a test sample fluid, which comprises competitively reacting the antibody of the present invention, a test sample fluid and a labeled form of the protein of the present invention or the complex of the present invention, and measuring the ratio of the labeled form of the protein of the present invention or the complex of the present invention bound to said antibody; and,
(ii) a method of quantifying the protein of the present invention or the complex of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that the two antibodies recognize different sites of the protein of the present invention or the complex of the present invention.

The monoclonal antibody to the protein of the present invention or the complex of the present invention (hereinafter sometimes referred to as the monoclonal antibody of the present invention) can be used to quantify the complex of the present invention. In addition, the complex can be detected by means of a tissue staining as well. For these purposes, the antibody molecule per se may be used or F(ab')₂, Fab' or Fab fractions of the antibody molecule may also be used.

The method of quantifying the protein of the present invention or the complex of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., β-galactosidase, β-glucosidase, alkaline phosphatase, peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For insolubilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the insolubilized monoclonal antibody of the present invention is reacted with a test fluid (primary reaction), then with a labeled form of another monoclonal antibody of the present invention (secondary reaction), and the activity of the label on the insolubilizing carrier is measured, whereby the amount of the complex of the present invention in the test fluid can be quantified. The order of the primary and secondary reactions may be reversed, and the reactions may be performed simultaneously or with an interval. The methods of labeling and insolubilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention or the complex of the present invention by the sandwich method of the present invention, antibodies that bind to different sites of the protein of the present invention or the complex of the present invention are preferably used as the monoclonal antibodies of the present invention used for the primary and secondary reactions. That is, in the antibodies used for the primary and secondary reactions are, for example, when the antibody used in the secondary reaction recognizes the complex part formed by the C-terminal region of the SEMA4B protein, it is preferred to use the antibody recognizing the other site, e.g., the complex part formed by the N-terminal region of the SEMA4B protein.

The monoclonal antibodies of the present invention can be used for assay systems other than the sandwich method, for example, the competitive method, the immunometric method, nephrometry, etc.

In the competitive method, an antigen in a test fluid and a labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, an antigen in a test fluid and an immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of the antigen in the test fluid is small and only a small amount of precipitates is obtained, it is advantageous to use laser nephrometry utilizing scattering of laser.

For applying each of these immunological methods to the quantification method of the present invention, any particular conditions or procedures are not required Quantification system for the complex of the present invention is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

As described above, the protein of the present invention or the complex of the present invention can be quantified with high sensitivity, using the antibody of the present invention.

Furthermore, when an increased level of the protein of the present invention or the complex of the present invention is detected by quantifying the level of the protein of the present invention or the complex of the present invention using the antibody of the present invention, it can be diagnosed that one suffers from, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.); or it is highly likely to suffer from these disease in the future. On the other hand, when an decreased level of the complex of the present invention is detected by quantifying the level of the complex of the present invention using the antibody of the present invention, it can be diagnosed that one suffers from, for example, neurodegenerative diseases (e.g., Alzheimer's diseases (familial Alzheimer's disease, juvenile Alzheimer's disease, sporadic Alzheimer's disease, etc.); or it is highly likely to suffer from these disease in the future.

Moreover, the antibody of the present invention can be used to detect the protein of the present invention or the complex of the present invention, which is present in a test sample such as a body fluid, a tissue, etc. The antibody can also be used to prepare an antibody column for purification of the complex of the present invention, detect the complex of the present invention in each fraction upon purification, analyze the behavior of the complex of the present invention in the cells under investigation; etc.

### [3] Medicament comprising the antibody of the present invention

When the antibody of the present invention inhibits the formation of the complex of the present invention, the antibody of the present invention has the apoptosis promoting activity and thus, can be used as an agent for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.), or as an apoptosis promoter of cancer cells.

On the other hand, when the antibody of the present invention promotes the formation of the complex of the present invention, the antibody of the present invention has the neuronal apoptosis suppressing activity and thus, can be used as an agent for preventing/treating, for example, neurodegenerative diseases (e.g., Alzheimer's diseases (familial Alzheimer's disease, juvenile Alzheimer's disease, sporadic Alzheimer's disease, etc.)), or as a neuronal apoptosis inhibitor (suppresser).

Furthermore, when the antibody of the present invention promotes dissociation of the complex of the present invention, the antibody of the present invention has the apoptosis promoting activity and thus can be used as an agent for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.), or as an apoptosis promoter of cancer cells.

On the other hand, when the antibody of the present invention inhibits the dissociation of the complex of the present invention, the antibody of the present invention has the neuronal apoptosis suppressing activity and hence, can be used as an agent for preventing/treating, for example, neurodegenerative diseases (e.g., Alzheimer's diseases (familial Alzheimer's disease, juvenile Alzheimer's disease, sporadic Alzheimer's disease, etc.)), or as a neuronal apoptosis inhibitor (suppresser).

The aforesaid preventive/therapeutic agents for diseases and promoters comprising the antibody of the present invention are safe and low toxic. Accordingly, they can be administered to human or a mammal (e.g., rat, rabbit, sheep, swine, bovine, cat, dog, monkey, etc.) orally or parenterally (e.g., intravascularly, subcutaneously, etc.) either as liquid preparations as they are or as pharmaceutical compositions of adequate dosage form. Preferably, they can be administered also in the form of vaccine in a conventional manner.

The antibody of the present invention may be administered in itself or as an appropriate pharmaceutical composition. The pharmaceutical composition used for the administration may contain the antibody of the present invention and its salt, a pharmacologically acceptable carrier, a diluent or an excipient. Such a composition is provided in the form of pharmaceutical preparations suitable for oral or parenteral administration.

Examples of the composition for parenteral administration are injectable preparations, suppositories, vaccine, etc. The injectable preparations may include dosage forms such as intravenous, subcutaneous, intracutaneous and intramuscular injections, drip infusions, etc. These injectable preparations may be prepared by methods publicly known. The injectable preparations may be prepared, e.g., by dissolving, suspending or emulsifying the antibody of the present invention or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Advantageously, the pharmaceutical compositions for oral or parenteral use described above are prepared into pharmaceutical preparations in a unit dose suited to fit a dose of the active ingredients. Such unit dose preparations include, for example, tablets, pills, capsules, injections (ampoules), suppositories, etc. The amount of the aforesaid antibody contained is generally about 5 to 500 mg per dosage unit form; especially in the form of injection, it is preferred that the aforesaid antibody is contained in about 5 to 100 mg and in about 10 to 250 mg for the other forms.

The dose of the aforesaid preventive/therapeutic agent or regulator comprising the antibody of the present invention may vary depending upon subject to be administered, target disease, conditions, route of administration, etc. For example, when it is used for the purpose of treating/preventing, e.g., breast cancer in an adult, it is advantageous to intravenously administer the antibody of the present invention in a single dose of about 0.01 to about 20 mg/kg body weight, preferably about 0.1 to about 10 mg/kg body weight and more preferably about 0.1 to about 5 mg/kg body weight in approximately 1 to 5 times a day, preferably in approximately 1 to 3 times a day. In other parenteral administration and oral administration, the preventive/therapeutic agent or regulator can be administered in a dose corresponding to the dose given above. When the condition is especially severe, the dose may be increased according to the condition.

The antibody of the present invention may be administered in itself or in the form of an appropriate pharmaceutical composition. The pharmaceutical composition used for the administration may contain a pharmacologically acceptable carrier with the aforesaid antibody or its salts, a diluent or excipient. Such a composition is provided in the form of pharmaceutical preparations suitable for oral or parenteral administration (e.g., intravascular injection, subcutaneous injection, etc.).

Each composition described above may further contain other active components unless formulation causes any adverse interaction with the antibody described above.

In the specification, when the codes of bases, amino acids, etc. are abbreviated, they are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | :adenosine triphosphate |
| EDTA : | ethylenediaminetetraacetic acid |
| EGTA : | ethylene glycol-bis(beta-aminoethyl ether)- tetraacetic acid |
| SDS : | sodium dodecyl sulfate |
| Gly : | glycine |
| Ala : | alanine |
| Val : | valine |
| Leu : | leucine |
| lie : | isoleucine |
| Ser: | serine |
| Thr : | threonine |
| Cys : | cysteine |
| Met : | methionine |
| Glu : | glutamic acid |
| Asp : | aspartic acid |
| Lys : | lysine |
| Arg : | arginine |
| His : | histidine |
| Phe : | phenylalanine |
| Tyr : | tyrosine |
| Trp : | tryptophan |
| Pro : | proline |
| Asn : | asparagine |
| Gln : | glutamine |
| pGlu: | pyroglutamic acid |
| Sec : | selenocysteine |

Substituents, protecting groups and reagents generally used in this specification are presented as the codes below.

| | |
|---|---|
| Me: | methyl group |
| Et : | ethyl group |
| Bu : | butyl group |
| Ph : | phenyl group |
| TC : | thiazolidine-4(R)-carboxamido group |
| Tos : | p-toluenesulfonyl |
| CHO : | formyl |
| Bzl : | benzyl |
| Cl₂-Bzl : | 2,6-dichlorobenzyl |
| Bom : | benzyloxymethyl |
| Z : | benzyloxycarbonyl |
| Cl-Z : | 2-chlorobenzyloxycarbonyl |
| Br-Z: | 2-bromobenzyl oxycarbonyl |
| Boc: | t-butoxycarbonyl |
| DNP : | dinitrophenol |
| Trt : | trityl |
| Bum : | t-butoxymethyl |
| Fmoc : | N-9-fluorenyl methoxycarbonyl |
| HOBt: | 1-hydroxybenztriazole |
| HOOBt : | 3,4-dihydro-3-hydroxy-4-oxo-1,2,3-benzotriazine |
| HONB: | 1-hydroxy-5-norbornene-2,3-dicarboxyimide |
| DCC : | N,N'-dicyclohexylcarbodiimide |

The sequence identification numbers in the sequence listing of the specification indicate the following sequences.

### [SEQ ID NO: 1]

This shows the amino acid sequence of SEMA4B.

### [SEQ ID NO: 2]

This shows the base sequence of DNA encoding SEMA4B having the amino acid sequence represented by SEQ ID NO: 1.

### [SEQ ID NO:3]

This shows the base sequence of DNA containing the full-length gene encoding SEMA4B.

### [SEQ ID NO: 4]

This shows the amino acid sequence of SEMA4B-M1.

### [SEQ ID NO: 5]

This shows the base sequence of DNA encoding SEMA4B-M1 having the amino acid sequence represented by SEQ ID NO: 4.

### [SEQ ID NO: 6]

This shows the base sequence of DNA containing the full-length gene encoding SEMA4B-M1.

### [SEQ ID NO: 7]

This shows the amino acid sequence of SEMA4B-M2.

### [SEQ ID NO: 8]

This shows the base sequence of DNA encoding SEMA4B-M2 having the amino acid sequence represented by SEQ ID NO: 7.

### [SEQ ID NO: 9]

This shows the base sequence of DNA containing the full-length gene encoding SEMA4B-M2.

### [SEQ ID NO: 10]

This shows the amino acid sequence of SEMA4B-M3.

### [SEQ ID NO: 11]

This shows the base sequence of DNA encoding SEMA4B-M3 having the amino acid sequence represented by SEQ ID NO: 10.

### [SEQ ID NO: 12]

This shows the base sequence of DNA containing the full-length gene encoding SEMA4B-M3.

### [SEQ ID NO: 13]

This shows the base sequence of antisense oligonucleotide used in REFERENCE EXAMPLES 2, 3, 15 and 16.

### [SEQ ID NO: 14]

This shows the base sequence of oligonucleotide used in REFERENCE EXAMPLES 2, 3, 15 and 16.

### [SEQ ID NO: 15]

This shows the base sequence of antisense oligonucleotide used in EXAMPLE 3.

### [SEQ ID NO: 16]

This shows the base sequence of oligonucleotide used in REFERENCE EXAMPLE 3.

### [SEQ ID NO: 17]

This shows the base sequence of primer used in REFERENCE EXAMPLE 3.

### [SEQ ID NO: 18]

This shows the base sequence of primer used in REFERENCE EXAMPLE 3.

### [SEQ ID NO: 19]

This shows the base sequence of primer used in REFERENCE EXAMPLES 4, 6 and 7.

### [SEQ ID NO: 20]

This shows the base sequence of primer used in REFERENCE EXAMPLES 4 and 7.

### [SEQ ID NO: 21]

This shows the base sequence of primer used in REFERENCE EXAMPLE 6.

### [SEQ ID NO: 22]

This shows the base sequence of Peptide 1 used in EXAMPLE 8.

### [SEQ ID NO: 23]

This shows the base sequence of Peptide 2 used in REFERENCE EXAMPLE 8.

### [SEQ ID NO: 24]

This shows the base sequence of Peptide 3 used in REFERENCE EXAMPLE 8.

### [SEQ ID NO: 25]

This shows the base sequence of Peptide 4 used in REFERENCE EXAMPLE 8.

### [SEQ ID NO: 26]

This shows the 739-837 amino acid sequence in the amino acid sequences of SEMA4B, SEMA4B-M1, SEMA4B-M2 and SEMA4B-M3.

### [SEQ ID NO: 27]

This shows the amino acid sequence of DLG1.

### [SEQ ID NO: 28]

This shows the base sequence of DNA encoding DLG1.

### [SEQ ID NO: 29]

This shows the amino acid sequence of DLG3.

### [SEQ ID NO: 30]

This shows the base sequence of DNA encoding DLG3.

The transformant, *Escherichia coli* TOP10/SEMA4B-M1/pCR4-TOPO obtained in REFERENCE EXAMPLE 4 later described has been on deposit since March 4, 2003 under the Accession Number FERM BP-8316 at the National Institute of Advanced Industrial Science and Technology, International Patent Organism Depositary, located at Central 6, 1-1-1 Higashi, Tsukuba, Ibaraki, Japan (postal code 305-8566).

The transformant, *Escherichia coli* TOP 10/SEMA4B-M2/pCR4-TOPO obtained in REFERENCE EXAMPLE 4 later described has been on deposit since March 4, 2003 under the Accession Number FERM BP-8317 at the National Institute of Advanced Industrial Science and Technology, International Patent Organism Depositary, located at Central 6, 1-1-1 Higashi, Tsukuba, Ibaraki, Japan (postal code 305-8566).

The transformant, *Escherichia coli* TOP 10/SEMA4B-M3/pCR4-TOPO obtained in REFERENCE EXAMPLE 4 later described has been on deposit since March 4, 2003 under the Accession Number FERM BP-8318 at the National Institute of Advanced Industrial Science and Technology, International Patent Organism Depositary, located at Central 6, 1-1-1 Higashi, Tsukuba, Ibaraki, Japan (postal code 305-8566).

Hereinafter, the present invention will be described specifically with reference to REFERENCE EXAMPLES and EXAMPLE but is not deemed to be limited thereto. The gene manipulation procedures using *Escherichia coli* were performed according to the methods described in the Molecular Cloning, 2nd, Cold Spring Harbor Lab. Press, (1989).

### REFERENCE EXAMPLE 1

### Gene Expression Analysis

In order to clarify a group of genes with overexpression specifically in lung cancer tissues, gene expression analysis was performed by oligonucleotide microarray (Human Genome U95A, U95B, U95C, U95D, U95E; Affymetrix) on total RNAs extracted from 4 lung cancer tissues and 5 normal lung tissues (TABLE 1) as samples. The experimental procedures were performed as instructed in the Affymetrix manual (Expression Analysis Technical Manual).

As a result, overexpression of the genes for Semaphorin 4B (SEMA4B) and Semaphorin 4B-M1 (SEMA4B-M1), Semaphorin 4B-M2 (SEMA4B-M2) and Semaphorin 4B-M3 (SEMA4B-M3) later described in REFERENCE EXAMPLE 4 was detected in 3 lung cancer tissues (lot. 0011-192-01285, lot. 0011-192-01293 and lot. 0011-192-01297) (TABLE 2).

**[TABLE 1]**

| RNA-Extracted Tissue | Distribution Source |
|---|---|
| Lung cancer tissue (lot. 0009-192-00122) | BioClinical Partners, Inc. |
| Lung cancer tissue (lot. 0011-192-0128-5) | BioClinical Partners, Inc. |
| Lung cancer tissue (lot. 0011-192-01293) | BioClinical Partners, Inc. |
| Lung cancer tissue (lot. 0011-192-01297) | BioClinical Partners, Inc. |
| Normal lung tissue (lot. 0009-192-00150) | BioClinical Partners, Inc. |
| Normal lung tissue (lot. 0009-192-00168) | BioClinical Partners, Inc. |
| Normal lung tissue (lot. 0011-192-01283) | BioClinical Partners, Inc. |
| Normal lung tissue (lot. 0011-192-01285) | BloClinical Partners, Inc. |
| Normal lung tissue (lot. 0011-192-01297) | BioClinical Partners, Inc. |

**[TABLE 2]**

| Tissue | Gene Expression Level |
|---|---|
| Lung cancer tissue (lot. 0009-192-00122) | ND |
| Lung cancer tissue (lot. 0011-192-01285) | 10 |
| Lung cancer tissue (lot. 0011-192-01293) | 9.5 |
| Lung cancer tissue (lot. 0011-192-01297) | 1.9 |
| Normal lung tissue (lot. 0009-192-00150) | ND |
| Normal lung tissue (lot. 0009-192-00168) | ND |
| Normal lung tissue (lot. 0011-192-01283) | ND |
| Normal lung tissue (lot. 0011-192-01285) | ND |
| Normal lung tissue (lot. 0011-192-01297) | ND |

The gene expression level was normalized by taking as 1 the median value of the expression levels of all genes, which expression was detected by the oligonucleotide microarray.
ND: not detected

### REFERENCE EXAMPLE 2

### Apoptosis Induction in Human Lung Cancer Cell Line

The expression of the SEMA4B gene and the SEMA4B-M1, SEMA4B-M2 and SEMA4B-M3 genes described in REFERENCE EXAMPLE 4 was suppressed to see if apoptosis was induced in the human lung cancer cell line.

First, human non-small-cell lung cancer cell line NCI-H1703 purchased from American Type Culture Collection (ATCC) was suspended in RPMI-1640 medium (containing 25 mM HEPES) (Invitrogen) supplemented with 10% fetal calf serum (ATCC), and plated on a 96-well flat bottom tissue culture plate (BD Falcon) at a cell density of 10,000 cells/well (0.1 ml of medium volume) and then incubated overnight at 37°C in a 5% carbon dioxide gas flow, followed by transfection of an antisense oligonucleotide.

Specifically, after the antisense oligonucleotide sequence (SEQ ID NO: 13) hybridizable to a sequence in the 3' untranslated region of the protein having the amino acid sequences represented by SEQ ID NO: 1, SEQ ID NO: 4, SEQ ID NO: 7 and SEQ ID NO: 10 was designed, phosphorothioated oligonucleotide was synthesized, purified on HPLC and provided for use in transfection experiment (hereinafter merely referred to as the antisense oligonucleotide). For control, the reverse sequence (SEQ ID NO: 14) of the base sequence represented by SEQ ID NO: 13 was similarly phosphorothioated, purified on HPLC and provided for use (hereinafter merely referred to as the control oligonucleotide).

The antisense oligonucleotide or the control oligonucleotide diluted in Opti-MEM (Invitrogen) was mixed with Oligofectamine (Invitrogen) diluted in Opti-MEM (Invitrogen) to 5-fold and settled at room temperature for 5 minutes, in a ratio of 8:3 (volume ratio). The resulting mixture was dispensed to the plate in 40 µL/well. The final concentration of the oligonucleotide was adjusted to become 250 nM. After incubation was continued for further 3 days under the conditions described above, the apoptosis induction activity of the two oligonucleotides above was assayed with Cell Death Detection ELISA^{PLUS} Kit (Roche Diagnostics) in accordance with the protocol attached thereto.

As a result, the antisense oligonucleotide (SEQ ID NO: 13) showed the apoptosis induction activity of approximately 1.6 times higher than the control oligonucleotide (SEQ ID NO: 14), indicating that there were statistically significant differences (P≦0.01) (TABLE 3).

**[TABLE 3]**

| | Apoptosis Induction Activity (A₄₀₅-A₄₉₂) | |
|---|---|---|
| | Mean Value | Standard Deviation |
| Blank | 0. 212 | 0. 032 |
| Control oligonucleotide (SEQ ID NO: 14) | 0. 410 | 0. 017 |
| Antisense oligonucleotide (SEQ ID NO: 13) | 0. 538 | 0. 035 |

### REFERENCE EXAMPLE 3

### Reduction in Gene Expression Level by SEMA4B Antisense Oligonucleotide

It was examined if the expression levels of the SEMA4B gene and the SEMA4B-M1, SEMA4B-M2 and SEMA4B-M3 genes described in REFERENCE EXAMPLE 4 were reduced by administration of the antisense oligonucleotide.

Human non-small-cell lung cancer cell line NCI-H1703 used in REFERENCE EXAMPLE 2 was suspended in the same medium as in REFERENCE EXAMPLE 2, and plated on a 24-well flat bottom tissue culture plate (BD Falcon) at a cell density of 60,000 cells/well (0.6 ml of medium volume). The cells were incubated overnight at 37°C in a 5% carbon dioxide gas flow, followed by transfection of the antisense oligonucleotide. However, the oligonucleotide solution was added in a volume of 240 µL/well and two antisense oligonucleotides (SEQ ID NO: 13 and SEQ ID NO: 15) and two oligonucleotides (SEQ ID NO: 14 and SEQ ID NO: 16) for control were used.

Concerning the antisense oligonucleotide and the control oligonucleotide from SEQ ID NO: 15 and the control oligonucleotide from SEQ ID NO: 16, the antisense oligonucleotide sequence (SEQ ID NO: 15) hybridizable to a sequence in the 3' untranslated region of the protein having the amino acid sequences represented by SEQ ID NO: 1, SEQ ID NO: 4, SEQ ID NO: 7 and SEQ ID NO: 10 was designed. Then, the phosphorothioated oligonucleotide was synthesized, purified on HPLC and used for transfection experiment. The reverse sequence (SEQ ID NO: 16) of the base sequence represented by SEQ ID NO: 15 was similarly phosphorothioated, purified on HPLC and provided for use.

Following the transfection, incubation was continued at 37°C for further 24 hours in a 5% carbon dioxide gas flow and the total RNA was then extracted by RNeasy (registered trademark) Mini Total RNA Kit (QIAGEN). Using as a template about 300 ng of the total RNA, reverse transcription was carried out on TaqMan Reverse Transcription Reagents (Applied Biosystems) in accordance with the protocol attached thereto. Using as a template cDNA in an amount corresponding to 7-9 ng when converted into the total RNA, the number of expression copies of the SEMA4B, SEMA4B-M1, SEMA4B-M2 and SEMA4B-M3 genes was determined using two primers (SEQ ID NO: 17 and SEQ ID NO: 18) and SYBR Green PCR Master Mix (Applied Biosystems). The expression level of β-actin gene contained in the same amount of template cDNA was assayed on TaqMan β-actin Control Reagents (Applied Biosystems), which was used as internal standard.

When distilled water was used in place of the oligonucleotide solution (hereinafter briefly referred to as the non-transfection group), the total expression level of the SEMA4B, SEMA4B-M1, SEMA4B-M2 and SEMA4B-M3 genes was 6.6% based on the expression level of β-actin gene, whereas in the groups given with the antisense oligonucleotides (SEQ ID NO: 13 and SEQ ID NO: 15), the expression levels were 0.98% and 1.1%, indicating that a statistically significant (P≦0.05) reduction in the expression level was observed.

On the other hand, the expression levels were 4.1% and 3.4% in the groups given with the control oligonucleotides (SEQ ID NO: 14 and SEQ ID NO: 16), indicating that any statistically significant reduction in the expression level was not observed when compared to the non-transfection group.

These results revealed that the suppressed expression of the SEMA4B, SEMA4B-M1, SEMA4B-M2 and SEMA4B-M3 genes were correlated to the induction of apoptosis.

### REFERENCE EXAMPLE 4

### Cloning and Base Sequencing of cDNAs Encoding SEMA4B, SEMA4B-M1, SEMA4B-M2 and SEMA4B-M3

Using human lung cancer cell line (A549)-derived Marathon-Ready cDNA (CLONTECH) as a template, PCR was carried out by using two primers (SEQ ID NO: 19 and SEQ ID NO: 20). The reaction solution (50 µl) was composed of 1 µl of the above cDNA, 2. 5 U of PfuTurbo Hotstart DNA Polymerase (STRATAGENE), 1.0 µM each of the primers (SEQ ID NO: 19 and SEQ ID NO: 20), 200 µM of dNTPs and 25 µl of 2 x GC Buffer I (Takara Bio.). PCR was carried out by reacting at 95°C for 1 minute and then repeating 30 times the cycle set to include 95°C for 1 minute, 60°C for 1 minute and 72°C for 4 minutes, and extension was performed at 72°C for 5 minutes. In order to add dATP to the PCR product at the 3' end, 5 U Ex Taq DNA Polymerase (Takara Bio.) was added and the mixture was kept at 72°C for 7 minutes. The PCR product obtained was purified using PCR Purification Kit (QIAGEN). The purified product was subcloned to plasmid vector pCR4-TOPO (Invitrogen) according to the protocol of TOPO TA PCR Cloning Kit (Invitrogen). The clones were transfected to *Escherichia coli* TOP10 and the clones bearing cDNA were selected in ampicillin-containing LB agar medium. The base sequences of individual clones were analyzed to give the base sequences of cDNAs represented by SEQ ID NO: 2, SEQ ID NO: 5, SEQ ID NO: 8 and SEQ ID NO: 11, respectively.

The base sequences in which the 13-249 base sequence and the 2761-3778 base sequence in the base sequence for the SEMA4B gene (GenBank Accession No. NM_198925 gene) are added to the base sequences represented by SEQ ID NO: 2, SEQ ID NO: 5, SEQ ID NO: 8 and SEQ ID NO: 11 at the 5' and 3' ends thereof are represented by SEQ ID NO: 3, SEQ ID NO: 6, SEQ ID NO: 9 and SEQ ID NO: 12, respectively.

The amino acid sequence (SEQ ID NO: 1) encoded by the base sequence represented by SEQ ID NO: 2 completely coincided with the SEMA4B protein encoded by the SEMA4B gene (GenBank Accession No. NM_198925 gene).

The protein having the amino acid sequence (SEQ ID NO: 4) encoded by the base sequence represented by SEQ ID NO: 5, the protein having the amino acid sequence (SEQ ID NO: 7) encoded by the base sequence represented by SEQ ID NO: 8 and the protein having the amino acid sequence (SEQ ID NO: 10) encoded by the base sequence represented by SEQ ID NO: 11 were named SEMA4B-M1, SEMA4B-M2 and SEMA4B-M3, respectively.

In the amino acid sequence (SEQ ID NO: 4) of SEMA4B-M1, Ser at the 208 position is replaced by Ile in the amino acid sequence (SEQ ID NO: 1) of SEMA4B.

In the base sequence (SEQ ID NO: 5) of DNA encoding the SEMA4B-M1, g at the 90 position is replaced by a, g at the 111 position by a and g at the 623 position by t, respectively, in the base sequence (SEQ ID NO: 2) of DNA encoding the SEMA4B, and the substitution at the 623 position is accompanied by amino acid substitution.

In the amino acid sequence (SEQ ID NO: 7) of SEMA4B-M2, Met at the 163 position is replaced by Ile in the amino acid sequence (SEQ ID NO: 1) of SEMA4B.

In the base sequence (SEQ ID NO: 8) of DNA encoding the SEMA4B-M2, g at the 150 position is replaced by a, g at the 489 position by a, c at the 528 position by t, t at the 1266 position by c, c at the 1588 position by a and a at the 2343 position by g, respectively, in the base sequence (SEQ ID NO: 2) of DNA encoding the SEMA4B, and the substitution at the 489 position is accompanied by amino acid substitution.

In the amino acid sequence (SEQ ID NO: 10) of SEMA4B-M3, Lys at the 364 position is replaced by Asn in the amino acid sequence (SEQ ID NO: 1) of SEMA4B.

In the base sequence (SEQ ID NO: 11) of DNA encoding the SEMA4B -M3, g at the 1092 position in the base sequence (SEQ ID NO: 2) of DNA encoding the SEMA4B is replaced by t, which is accompanied by amino acid substitution.

The plasmid bearing DNA having the base sequence represented by SEQ ID NO: 2 was named SEMA4B/pCR4-TOPO, the plasmid bearing DNA having the base sequence represented by SEQ ID NO: 5 was named SEMA4B-M1/pCR4-TOPO, the plasmid bearing DNA having the base sequence represented by SEQ ID NO: 8 was named SEMA4B-M2/pCR4-TOPO and the plasmid bearing DNA having the base sequence represented by SEQ ID NO: 11 was named SEMA4B-M3/pCR4-TOPO, respectively.

Furthermore, the plasmid SEMA4B/pCR4-TOPO-transfected transformant was named *Escherichia coli* TOP10/SEMA4B/pCR4-TOPO, the plasmid SEMA4B-M1/pCR4-TOPO-transfected transformant was named *Escherichia coli* TOP 10/SEMA4B-M1/pCR4-TOPO, the plasmid SEMA4B-M2/pCR4-TOPO-transfected transformant was named *Escherichia coli* TOP 10/SEMA4B-M2/pCR4-TOPO and the plasmid SEMA4B-M3/pCR4-TOPO-transfected transformant was named *Escherichia coli* TOP 10/SEMA4B-M3/pCR4-TOPO, respectively.

### REFERENCE EXAMPLE 5

### Study of Gene Expression Level in Human Cell Line

The following 86 strains of brain tumor cell lines SK-N-MC, SK-N-AS, SK-N-BE, SK-N-DZ, SK-N-FI, SK-N-SH, D341Med, Daoy, DBTRG-05MG, U-118 MG, U-87 MG, CCF-STTG1 and SW 1088; human breast cancer cell lines HCC1937, ZR-75-1, AU565, MCF-7 and MDA-MB-231; human colon cancer cell lines Caco-2, COLO201, COLO 205, COLO 320DM, HCT-8, HT-29, LoVo, LS123, SNU-C1, SK-CO-1, SW 403, SW 48, SW480, SW 620, SW 837 and SW 948; human embryonic kidney cell line HEK293; human small cell cancer cell lines NCI-H187, NCI-H378, NCI-H526, NCI-H889, NCI-H1672, NCI-H1836, NCI-H2227, NCI-N417 and SHP-77; human non-small cell lung cancer cell lines A549, NCI-H23, NCI-H226, NCI-H358, NCI-H460, NCI-H522, NCI-H661, NCI-H810, NCI-H1155, NCI-H1299, NCI-H1395, NCI-H1417, NCI-H1435, NCI-H1581, NCI-H1651, NCI-H1703, NCI-H1793, NCI-H1963, NCI-H2073, NCI-H2085, NCI-H2106, NCI-H2228, NCI-H2342 and NCI-H2347; human ovary cancer cell lines ES-2, Caov-3, MDAH2774, NIH:OVCAR3, OV-90, SK-OV-3, TOV-112D and TOV-21G; human pancreatic cancer cell lines PANC-1, MIA-PaCa-2, AsPC-1, BxPC-3, Capan-1 and Capan-2; human prostate cancer cell line DU145; human retinoblastoma cell lines WERI-Rb-1 and Y79; and human testicular cancer cell line Cates-1B used below were purchased from ATCC. Human normal small airway epithelial cells SAEC and human normal prostate epithelial cells HPrEC were purchased from Clonetics Corp. Human colon cancer cell line COCM1, human non-small lung cancer cell line VMRC-LCD and human prostate cancer cell line PC3 were purchased from JCRB. These cell lines are sometimes used in REFERENCE EXAMPLE 9 and subsequent REFERENCE EXAMPLES. Total RNA was prepared from the 91 cell lines described above using RNeasy Mini Total RNA Kit (QIAGEN). Reverse transcription was performed on this total RNA as a template using a random primer to prepare cDNA. Using this cDNA as a template, quantitative PCR was carried out to examine the expression levels of the SEMA4B gene (SEQ ID NO: 2), the SEMA4B-M1 gene (SEQ ID NO: 5), the SEMA4B-M2 gene (SEQ ID NO: 8) and the SEMA4B-M3 gene (SEQ ID NO: 11).

The PCR above was carried out under the same conditions as in REFERENCE EXAMPLE 3, using cDNA obtained from 3 to 4 ng of the total RNA described above as the template, and the copies of the SEMA4B, SEMA4B-M1, SEMA4B-M2 and SEMA4B-M3 genes expressed were calculated. In parallel, the copy number of the gene for β-actin contained in 1 ng of the total RNA above was calculated using TaqMan Human β-actin Control Reagents (Applied Biosystems) and used as an internal standard.

A relative expression rate obtained by normalizing the total gene expression level described above with the gene expression level of β-actin is shown in TABLES 4 and 5.

The cancer cell lines in which the total gene expression level described above exceeds 1% of the gene expression level of β-actin gene were found to be 17 strains, indicating that overexpression of the genes above was detected in the cancer cell lines.

**[TABLE 4]**

| Cell Line | % of β-actin | Cell Line | % of β-actin | Cell Line | % of β-actin |
|---|---|---|---|---|---|
| SK-N-MC | 0.02 | COLO 201 | 0.66 | NCI-H889 | 0.07 |
| SK-N-AS | 0.07 | COLO 205 | 0.40 | NCI-H1672 | 0.10 |
| SK-N-BE | 0.04 | COLO 320DM | 0.12 | NCI-H1836 | 0.08 |
| SK-N-DZ | 0.05 | HCT-8 | 0.36 | NCI-H2227 | 0.15 |
| SK-N-FI | 0.20 | HT-29 | 0.52 | NCI-N417 | 0.04 |
| SK-N-SH | 0.11 | LoVo | 0.58 | SHP-77 | 0.16 |
| D341 Med | 0.05 | LS123 | 0.04 | A549 | 0.35 |
| Daoy | 0.08 | SNU-C1 | 0.52 | NCI-H23 | 0.98 |
| DBTRG-05MG | 0.01 | SK-CO-1 | 0.45 | NCI-H226 | 0.04 |
| U-118 MG | 0.01 | SW 403 | 0.31 | NCI-H358 | 1.09 |
| U-87 MG | 0.20 | SW 48 | 0.06 | NCI-H460 | 0.08 |
| CCF-STTG1 | 0.23 | SW 480 | 0.03 | NCI-H522 | 0.05 |
| SW 1088 | 0.06 | SW 620 | 0.12 | NCI-H661 | 0.05 |
| HCC1937 | 0.17 | SW 837 | 0.59 | NCI-H810 | 0.03 |
| ZR-75-1 | 0.30 | SW 948 | 0.18 | NCI-H1155 | 0.07 |
| AU565 | 0.06 | HEK293 | 0.05 | NCI-H1299 | 0.10 |
| MCF-7 | 0.06 | SAEC | 1.73 | NCI-H1395 | 0.39 |
| MDA-MB-231 | 0.06 | NCI-H187 | 0.38 | NCI-H1417 | 0.21 |
| Caco-2 | 0.04 | NCI-H378 | 0.17 | NCI-H1435 | 0.26 |
| COCM1 | 0.10 | NCI-H526 | 0.14 | NCI-H1581 | 0.16 |

**[TABLE 5]**

| Cell Line | % of β-actin | Cell Line | % of β-actin | Cell Line | % of β-actin |
|---|---|---|---|---|---|
| NCI-H1651 | 1.03 | ES-2 | 0.02 | BxPC-3 | 0.17 |
| NCI-H1703 | 0.21 | Caov-3 | 0.13 | Capan-1 | 0.07 |
| NCI-H1793 | 0.29 | MDAH2774 | 0.37 | Capan-2 | 0.27 |
| NCI-H1963 | 0.12 | NIH:OVCAR3 | 0.14 | HPrEC | 2.87 |
| NCI-H2073 | 0.15 | OV-90 | 0.23 | DU 145 | 3.05 |
| NCI-H2085 | 0.02 | SK-OV-3 | 2.44 | PC3 | 0.43 |
| NCI-H2106 | 0.07 | TOV-112D | 0.06 | WERI-Rb-1 | 0.90 |
| NCI-H2228 | 1.89 | TOV-21G | 1.00 | Y79 | 0.06 |
| NCI-H2342 | 0.18 | PANC-1 | 1.88 | Cates-1B | 0.01 |
| NCI-H2347 | 0.24 | MIA-PaCa-2 | 0.02 | | |
| VMRC-LCD | 0.09 | AsPC-1 | 0.24 | | |

### REFERENCE EXAMPLE 6

### Construction of Animal Cell Expression Vectors for Recombinant Full-Length Protein

SEMA4B gene was amplified by PCR using the plasmid the SEMA4B/pCR4-TOPO obtained in REFERENCE EXAMPLE 4 as a template. In the reaction solution for the reaction, 2 ng of the SEMA4B/pCR4-TOPO was used as a template and 2.5 U of Pfu Turbo Hotstart DNA Polymerase (STRATAGENE), 1 µM each of 2 primers (SEQ ID NO: 19 and SEQ ID NO: 21), 200 µM of dNTPs and 5 µl of 10 x Pfu Buffer were added to make the solution volume 50 µl. PCR was carried out by reacting at 95°C for 1 minute and then repeating 25 times the cycle set to include 95°C for 1 minute, 60°C for 1 minute and 72°C for 4 minutes. Next, the PCR product was purified using PCR Purification Kit (QIAGEN) and then treated with restriction enzymes XbaI and Eco RI. The plasmid p3xFLAG-CMV 14 (Sigma) was also treated with XbaI and Eco RI. Each DNA fragment was purified on PCR Purification Kit, followed by ligation using DNA Ligation Kit ver.2 (Takara Bio, Inc.). After the ligation solution was transfected to *Escherichia coli* TOP10, the transformed *Escherichia coli* was selected in ampicillin-containing LB agar medium. As a result of the analysis of individual clones, the plasmid pCMV-14-SEMA4B bearing the cDNA fragment corresponding to the SEMA4B gene (SEQ ID NO: 2) was acquired.

### REFERENCE EXAMPLE 7

### Construction of Animal Cell Expression Vector for Recombinant Full-Length Protein with Recombinant Tag

Animal cell expression vector capable of expressing the SEMA4B protein fused with 3xFLAG tag at the C terminus of the protein was constructed. A pair of primers used to amplify the SEMA4B gene by PCR were changed to another pair of primers (SEQ ID NO: 19 and SEQ ID NO: 20) and otherwise under the same conditions as in the method described in REFERENCE EXAMPLE 6, the transformed *Escherichia coli* was selected. As a result, the plasmid pCMV-14-SEMA4B-3xFLAG bearing the cDNA fragment encoding a fusion protein consisting of the SEMA4B protein (SEQ ID NO: 1) fused with 3xFLAG tag at the C terminus of the protein was acquired.

### REFERENCE EXAMPLE 8

### Production and Purification of Peptide Antibodies

Based on the amino acid sequences of SEMA4B protein (SEQ ID NO: 1), SEMA4B-M1 protein (SEQ ID NO: 4), SEMA4B-M2 protein (SEQ ID NO: 7) and SEMA4B-M3 protein (SEQ ID NO: 10), the following 4 peptides (Peptides 1 to 4) composed of 12 to 15 amino acids were synthesized by the Fmoc solid phase synthesis.

The amino acid sequence of Peptide 1
[Asn-Ser-Ala-Arg-Glu-Arg-Lys-Ile-Asn-Ser-Ser-Cys (SEQ ID NO: 22)] is a sequence which has the 402-412 amino acid sequence of the SEMA4B protein (SEQ ID NO: 1) with Cys being added to the C terminus.

The amino acid sequence of Peptide 2
[Ser-Val-Val-Ser-Pro-Ser-Phe-Val-Pro-Thr-Gly-Glu-Lys-Pro-Cys (SEQ ID NO: 23)] is a sequence which has the 582-596 amino acid sequence of the SEMA4B protein (SEQ ID NO: 1).

The amino acid sequence of Peptide 3
[Pro-Leu-Asp-His-Arg-Gly-Tyr-Gln-Ser-Leu-Ser-Asp-Ser-Pro-Cys (SEQ ID NO: 24)] is a sequence which has the 781-794 amino acid sequence of the SEMA4B protein (SEQ ID NO: 1) with Cys being added to the C terminus.

The amino acid sequence of Peptide 4
[Ser-Arg-Val-Phe-Thr-Glu-Ser-Glu-Lys-Arg-Pro-Leu-Ser-Cys (SEQ ID NO: 25)] is a sequence which has the 797-809 amino acid sequence of the SEMA4B protein (SEQ ID NO: 1) with Cys being added to the C terminus.

Keyhole limpet hemocyanin (KLH) as a carrier protein was coupled to the respective peptides of Peptides 1, 2, 3 and 4, which were used as antigens to produce rabbit polyclonal antibodies, as described below.

One male rabbit KBL: JW (11 weeks old, Oriental Yeast Co., Ltd.) was used as an immunized animal. A suspension of complete Freund's adjuvant (Difco) was used for primary sensitization and a suspension of incomplete adjuvant (Difco) for the second sensitization and thereafter. The sensitization was performed by subcutaneous injection at the back and 0.5 mg of each antigen was used per sensitization. After the primary sensitization, it was repeated 3 times every 14 days. On day 52 after the primary sensitization, blood was collected through the carotid artery under anesthesia to give about 50 ml of serum. The serum thus obtained was concentrated by means of ammonium sulfate salting out. The total amount of the crude IgG fractions obtained were purified on protein A-affinity column (Amersham-Bioscience) to give about 103 mg, about 76 mg, about 112 mg and about 122 mg of purified IgGs from Peptides 1, 2, 3 and 4, respectively. Furthermore, the IgG fractions bound to a column immobilized with the respective immunogen peptides were acquired. For the immobilization, the C-terminal Cys of each peptide was utilized and the peptide was coupled to Sepharose column (Amersham-Bioscience) using borate buffer. For elution from the column, 8M urea/phosphate buffered saline (PBS) was used. The eluate was dialyzed to PBS to remove urea, which was followed by ultraconcentration and sterilization by filtering. Thus, affinity-purified antibodies AS-2531, AS-2532, AS-2591 and AS-2592 to Peptides 1, 2, 3 and 4 were acquired in about 15 mg, about 126 mg, about 17 mg and about 35 mg, respectively.

### REFERENCE EXAMPLE 9

### Western Blotting Using Rabbit Peptide Antibodies

SEMA4B protein (SEQ ID NO: 1) was detected using the purified peptide antibodies prepared in REFERENCE EXAMPLE 8. Human non-small lung cancer-derived NCI-H358 cells were suspended in 10 ml ofRPMI-1640 medium (Invitrogen) containing 10% fetal calf serum (JRH) at a concentration of 1.5 x 10⁶ and plated on a Petri dish of 10 cm in diameter. After incubation at 37°C overnight in a 5% carbon dioxide flow, 6 µg of the plasmid pCMV-14-SEMA4B prepared in REFERENCE EXAMPLE 6 was mixed with Plus reagent (Invitrogen) and OPTI-MEM I (Invitrogen). After the mixture was allowed to stand at room temperature for 15 minutes, LipofectAMINE Transfection Reagent (Invitrogen) and OPTI-MEM I were added to the mixture, which was allowed to stand at room temperature for further 15 minutes. The resulting mixture was dropwise added to the medium and incubation was continued. Two days after the transfection of expression plasmid, the cells were washed with ice-cooled PBS and 1 ml of ice-cooled RIPA buffer [50 mM Tris-hydrochloride buffer, pH 7.5, 150 mM sodium chloride, 1% Triton X-100, 0.1% SDS, 1% deoxycholic acid, Complete tablet (Roche Diagnostics), Phosphatase Inhibitor Cocktail-2 (Sigma)] was added to the cells. The mixture was allowed to stand at 4°C for 30 minutes. This RIPA buffer was recovered and centrifuged at 15,000 rpm for 20 minutes. The supernatant obtained was used as the cell-free extract. This cell-free extract was mixed with a 2-fold concentration of SDS-PAGE sample buffer [125 mM Tris-hydrochloride buffer, pH 6.8, 40% glycerol, 4% SDS, 0.04% Bromophenol Blue and 5% 2-mercaptoethanol] in equal volumes. After heating at 95°C for 5 minutes, 10 µl of the mixture was provided for SDS-PAGE on 10% acrylamide gel. The protein separated by electrophoresis was transferred onto Clear Blotting P Membrane (ATTO) in a conventional manner, which was then allowed to stand in a blocking buffer [50 mM Tris-hydrochloride buffer, pH 7.5, 500 mM sodium chloride, 0.1% Tween 20, 5% skimmed milk] at room temperature for an hour. Next, the peptide antibody AS-2531, AS-2532, AS-2591 or AS-2592 produced in REFERENCE EXAMPLE 8 were diluted with the blocking buffer in a concentration of 3 µg/ml, followed by reacting at 4°C overnight. Subsequently, the reaction mixture was allowed to stand for an hour in a dilution of HRP-labeled anti-rabbit IgG antibody (Amersham-Bioscience) diluted in the blocking buffer to 50,000-fold or 100,000-fold. Detection was performed according to the protocol attached to ECL plus (Amersham-Bioscience). Thus, the SEMA4B protein was detected.

Even when any of AS-2532, AS-2591 and AS-2592 except AS-2531 was used, a specific band attributed to the SEMA4B protein was noted at the position near 100 kD molecular weight.

### REFERENCE EXAMPLE 10

### Immunoprecipitation Using the Rabbit Peptide Antibodies

Using the purified peptide antibodies produced in REFERENCE EXAMPLE 8, immunoprecipitation was performed on the SEMA4B protein under non-denaturing conditions.

Using the plasmid pCMV-14-SEMA4B-3xFLAG acquired in REFERENCE EXAMPLE 7, the cell-free extract was prepared by the same procedures as in REFERENCE EXAMPLE 9. The cell-free extract, 400 µl, was added to 50 µl of a suspension of Protein G-Sepharose 4FF (Amersham-Bioscience) prepared by suspending in an equal volume of RIPA buffer) and 5 µg of any one of the peptide antibodies AS-2531, AS-2532, AS-2591 and AS-2592 described in REFERENCE EXAMPLE 8 was further added thereto. The resulting mixture was agitated at 4°C overnight. After the Protein G-Sepharose 4FF co-precipitated fraction was washed with RIPA buffer, the fraction was suspended in 50 µl of SDS-PAGE sample buffer [62.5 mM Tris-hydrochloride buffer, pH 6.8, 20% glycerol, 2% SDS, 0.02% Bromophenol Blue and 2.5% 2-mercaptoethanol]. After heating at 95°C for 5 minutes, 5µl or 10 µl of the suspension was provided for SDS-PAGE on 10% acrylamide gel. Detection was performed by the same procedures as in REFERENCE EXAMPLE 9, except that mouse anti-FLAG M2 antibody (Sigma) diluted with the blocking buffer to 0.2 µg/ml or 0.1 µg/ml was used as a primary antibody and HRP-labeled anti-mouse IgG antibody (Amersham-Bioscience) diluted with the blocking buffer to 25,000-fold or 50,000-fold was used as a secondary antibody.

Even when immunoprecipitation was performed using any of the peptide antibodies AS-2531, AS-2532, AS-2591 and AS-2592, a specific band attributed to the SEMA4B protein was noted at the position near 100 kD molecular weight.

The results reveal that the peptide antibodies AS-2531, AS-2532. AS-2591 and AS-2592 bind to the non-denaturing SEMA4B protein.

### REFERENCE EXAMPLE 11

### Study of Expression of SEMA4B Protein in Cancer Cell Lines

Lung cancer cell lines NCI-H2228, NCI-H1651, NCI-H358, NCI-H23 and NCI-H1703; ovary cancer cell lines SKOV-3 and TOV-21G; prostate cancer cell line DU145; and pancreatic cancer cell line PANC-1 were plated, respectively, on two Petri dishes of 10 cm in diameter. For each of the cells, the cells for one Petri dish were dispersed in Trypsin-EDTA (Invitrogen) and the number of cells was counted. Based on the cells counted, ice-cooled RIPA buffer (described in REFERENCE EXAMPLE 9) was added to the remaining one Petri dish in 1 ml/5 x 10⁶ cells, followed by allowing to stand at 4°C for 30 minutes. This RIPA buffer was recovered and centrifuged at 15,000 rpm for 20 minutes. The supernatant obtained was used as the cell-free extract. Meanwhile, a resin obtained by crosslinking the peptide antibody AS-2531 described in REFERENCE EXAMPLE 8 with Protein G-Sepharose 4FF (Amersham-Bioscience) according to the protocol attached to Size X Protein G Immunoprecipitation Kit (Pierce Chemical) was prepared and suspended in an equal volume of RIPA buffer. The aforesaid cell-free extract, 400 µl, was added to 30 µl of this suspension, followed by agitation overnight at 4°C. After washing the Protein G-Sepharose 4FF co-precipitated fraction with RIPA buffer, the fraction was suspended in 30 µl of SDS-PAGE sample buffer described in REFERENCE EXAMPLE 10 and the suspension was heated at 95°C for 5 minutes. Then, 20 µl of the suspension was provided for SDS-PAGE on 10% acrylamide gel. Using the peptide antibody AS-2532, detection was performed by a modification of the procedure described in REFERENCE EXAMPLE 9.

In the 9 cell lines described above, a specific band attributed to the SEMA4B protein was observed at the position near 100 kD molecular weight in each cell line of NCI-H2228, NCI-H358, NCI-H23, SKOV-3, DU145 and PANC-1. The results revealed that the SEMA4B protein was overexpressed in the 6 cancer cell lines described above.

### REFERENCE EXAMPLE 12

### Establishment of the Cell Line Stably Expressing the Full-Length Recombinant Protein

Human non-small cell lung cancer-derived NCI-H358 was suspended in 2 ml ofRPMI-1640 medium (Invitrogen) containing 10% fetal calf serum (JRH), 1 mM sodium pyruvate and 25 mM HEPES. The suspension was plated on a 6-well plate, followed by incubation overnight at 37°C in a 5% carbon dioxide gas. On the other hand, 1 µg of plasmid pCMV-14-SEMA4B described in REFERENCE EXAMPLE 6, which was diluted with the OPTI-MEM I (Invitrogen), was mixed with 6 µl of Plus reagent (Invitrogen) and the mixture was allowed to stand at room temperature for 15 minutes. Then, 4 µl of LipofectAMINE transfection reagent (Invitrogen) diluted in OPTI-MEM I was added to the mixture, which was allowed to stand at room temperature for further 15 minutes. The mixture was dropwise added to the medium and incubation was further continued for a day. The cells were then dispersed in trypsin-EDTA (Invitrogen) and diluted to 10-fold in the above medium added with G418 (Promega) in 400 µg/ml, followed by plating which was plated on a 24-well plate. While the medium was exchanged with the G418-containing medium (G418 selection medium) every 3 or 4 other days, incubation was continued at 37°C in a 5% carbon dioxide gas flow. From colonies formed when one to three cells proliferated, the cells were recovered and plated equally on two wells of a 48-well plate. After incubation was continued until the cell density reached 50% or more, 50 µl of the SDS-PAGE sample buffer described in REFERENCE EXAMPLE 10 was added to the cells for one well to prepare the cell lysate. After heat treatment at 95°C for 5 minutes, 5 µl was provided on for SDS-PAGE on 10% acrylamide gel. Using the peptide antibody AS-2532, western blotting was performed by a modification of the procedures described in REFERENCE EXAMPLE 9 to explore a stable cell line constitutively expressing the SEMA4B-A protein (SEQ ID NO: 1). The cells recovered from the other well were diluted in 0.7 cell/well and then plated on a 96-well plate. While exchanging the G418 selection medium every 3 or 4 other days, incubation was continued at 37°C in a 5% carbon dioxide gas flow until the cell density reached about 50%. Again, the cells were plated equally on 2 wells of a 48-well plate, and incubation was continued until the cell density reached 50% or more. Using the cell lysate prepared from the cells for one well, western blotting was performed as described above. A clone with the highest expression of SEMA4B protein (SEQ ID NO: 1) was selected to acquire SEMA4B/H358 as the cell line stably expressing SEMA4B.

### REFERENCE EXAMPLE 13

### Study of Localization of SEM4A4B Protein (Biotin Labeling)

Using non-small cell lung cancer cell lines NCI-H2228 and NCI-H358 and the cell line (SEMA4B/H358) stably expressing the full-length recombinant protein prepared in REFERENCE EXAMPLE 12, the proteins exposed on the cell surfaces were biotinylated with Cellular Labeling and Immunoprecipitation Kit (Roche Diagnostics). Subsequently, the cell-free extract was prepared by the procedures of REFERENCE EXAMPLE 9. Using 1 ml of the cell-free extract thus prepared and 5 µg of the peptide antibody AS-2591 prepared in REFERENCE EXAMPLE 8, immunoprecipitation was performed in accordance with the process of REFERENCE EXAMPLE 10, followed by SDS-PAGE. By detection with HRP-labeled streptoavidin (Amersham-Bioscience), bands attributed to the SEMA4B protein were noted near 100 kD molecular weight. This reveals that the SEMA4B protein, SEMA4B-M1 protein, SEMA4B-M2 protein and SEMA4B-M3 protein are localized on the cell surface.

### REFERENCE EXAMPLE 14

### Study of Localization of SEMA4B Protein (FACS Analysis)

Human non-small cell lung cancer cell lines NCI-H2228 and NCI-H358 and SEMA4B/H358 described in REFERENCE EXAMPLE 12 were plated, respectively, on a Petri dish of 10 cm in diameter and incubated to become subconfluent. After the respective cells were washed with PBS, PBS containing 0.5% BSA and 5 mM EDTA were added thereto. The mixture was allowed to stand at room temperature for 15 minutes to disperse the cells. Next, the cells were suspended in Buffer A [HBSS (Hanks' Balanced Salt Solutions, Invitrogen) containing 2% fetal calf serum (JRH) and 0.1% sodium azide] in a concentration of 4 x 10⁶/ml, and AS-2532 or non-immunized rabbit IgG (Jackson) was added to the suspension in a final concentration of 10 µg/ml. The mixture was allowed to stand in ice for 3 hours. The cells were then washed with Buffer A and suspended in Buffer A containing 10 µg/ml of Alexa488-labeled anti-rabbit IgG antibody (Molecular Probes), followed by allowing to stand on ice for 2 hours. After washing again with Buffer A, the cells were analyzed by FACScan (BD Biosciences). The results revealed that all cells were stained specifically to rabbit peptide antibody AS-2532, indicating that the SEMA4B protein, SEMA4B-M1 protein, SEMA4B-M2 protein and SEMA4B-M3 protein are localized on the cell surface.

### REFERENCE EXAMPLE 15

### Apoptosis Induction of Human Non-Small Cell Lung Cancer Cell Line NCI-H358 by Transfection with an Antisense Oligonucleotide

It was examined if apoptosis could be induced also in human non-small cell lung cancer cell line other than NCI-H1703 described in REFERENCE EXAMPLE 2 by transfection of the antisense oligonucleotide.

NCI-H358 was suspended in RPMI-1640 medium (Invitrogen) containing 10% fetal calf serum (JRH), 1 mM sodium pyruvate and 25 mM HEPES. NCI-H358 was plated on a 96-well flat bottom tissue culture plate (BD Falcon) at a cell density of 8 x 10³/well (80 µl of medium volume), followed by incubation at 37°C overnight in a 5% carbon dioxide gas flow. On the other hand, 0.06 µg each of the antisense oligonucleotide (SEQ ID NO: 13) described in REFERENCE EXAMPLE 2 and control oligonucleotide (SEQ ID NO: 14) were diluted in OPTI-MEM I (Invitrogen). The dilution was mixed with 0.5 µl of Plus reagent (Invitrogen) and the mixture was allowed to stand at room temperature for 15 minutes. To the mixture, 0.4 µl of LipofectAMINE transfection reagent (Invitrogen) diluted in OPTI-MEM I was added. The mixture was allowed to stand at room temperature for further 15 minutes. The whole volume of the mixture was added to the medium for NCI-H358, and incubation was continued for further 3 hours. Following the protocols attached to Cell Death Detection ELISA^{PLUS} (Roche Diagnostics) and Caspase-Glo 3/7 assay (Promega), the oligonucleotide described above was assayed for its apoptosis induction activity.

As a result, the oligonucleotide showed apoptosis induction activity in NCI-H358, which was higher by 1.42 times and 1.77 times, respectively, than the control antisense oligonucleotide used as a negative control for both Cell Death Detection ELISA^{PLUS} and Caspase-Glo 3/7 assay, indicating that there was a statistically significant difference (P≦0.01) (TABLES 6 and 7).

**[TABLE 6]**

| | Apoptosis Induction Activity (A₄₀₅-A₄₉₂) | |
|---|---|---|
| | Mean Value | Standard Deviation |
| Blank | 0.217 | 0.007 |
| Control oligonucleotide (SEQ ID NO: 14) | 0.330 | 0.041 |
| Antisense oligonucleotide (SEQ ID NO: 13) | 0.467 | 0.029 |

**[TABLE 7]**

| | Apoptosis Induction Activity (CPS) | |
|---|---|---|
| | Mean Value | Standard Deviation |
| Blank | 7625 | 235 |
| Control oligonucleotide (SEQ ID NO: 14) | 8727 | 188 |
| Antisense oligonucleotide (SEQ ID NO: 13) | 15452 | 570 |

### REFERENCE EXAMPLE 16

### Apoptosis Induction of Human Non-Small Cell Cancer Cell Lines NCI-H2228, NCI-H1651 and NCI-H23 by Transfection with an Antisense Oligonucleotide

It was examined if apoptosis could also be induced in human non-small cell lung cancer cell lines other than NCI-H1703 (REFERENCE EXAMPLE 2) and NCI-H358 (REFERENCE EXAMPLE 15) by transfection of the antisense oligonucleotide.

For NCI-H2228, RPMI-1640 medium (Invitrogen) containing 10% fetal calf serum (JRH), 1 mM sodium pyruvate and 25 mM HEPES was used. For NCI-H1651, ACL-4 medium (ATCC) containing 10% FBS was used. For NCI-H23, RPMI-1640 medium (Invitrogen) containing 10% fetal calf serum (JRH) and 25 mM HEPES was used. The respective cells were suspended in the corresponding media and plated on a 96-well flat bottom tissue culture plate (BD Falcon) at cell densities of 7.5 x 10³/well (NCI-H2228), 7.5 x 10³/well (NCI-H1651) and 5 x 10³/well (NCI-H23), respectively (125 µl of medium volume), followed by incubation overnight at 37°C in a 5% carbon dioxide gas flow. On the other hand, 0.135 µg each of the antisense oligonucleotide (SEQ ID NO: 13) described in REFERENCE EXAMPLE 2 and the control oligonucleotide (SEQ ID NO: 14) were diluted in OPTI-MEM I (Invitrogen), respectively. After each dilution was mixed with 0.75 µl of Plus reagent (Invitrogen), the mixture was allowed to stand at room temperature for 15 minutes. Then, 0.4 µl of LipofectAMINE reagent (Invitrogen) diluted in OPTI-MEM I was added to the mixture, which was allowed to stand at room temperature for further 15 minutes. The whole volume of the mixture was added to the medium and incubation was further continued for 3 days. Following the protocol attached to Cell Death Detection ELISA^{PLUS} (Roche Diagnostics), the oligonucleotide described above was assayed for its apoptosis induction activity.

As a result, the oligonucleotide showed the apoptosis induction activity in any cell line as higher by 1.58 times (NCI-H2228), 1.21 times (NCI-H1651) and 1.25 times (NCI-H23), respectively, than the control antisense oligonucleotide used as a negative control, wherein P-values were calculated to be P≦0.05 (NCI-H2228), P≦0.05 (NCI-H1651) and P≦0.01 (NCI-H23), showing statistically significant differences (TABLES 8, 9 and 10).

**[TABLE 8]**

| | Apoptosis Induction Activity (A₄₀₅-A₄₉₂) | |
|---|---|---|
| | Mean Value | Standard Deviation |
| Blank | 0.312 | 0.009 |
| Control oligonucleotide (SEQ ID NO: 14) | 0.526 | 0.043 |
| Antisense oligonucleotide (SEQ ID NO: 13) | 0.829 | 0.123 |

**[TABLE 9]**

| | Apoptosis Induction Activity (A₄₀₅-A₄₉₂) | |
|---|---|---|
| | Mean Value | Standard Deviation |
| Blank | 0.523 | 0.091 |
| Control oligonucleotide (SEQ ID NO: 14) | 1.152 | 0.101 |
| Antisense oligonucleotide (SEQ ID NO: 13) | 1.390 | 0.104 |

**[TABLE 10]**

| | Apoptosis Induction Activity (A₄₀₅-A₄₉₂) | |
|---|---|---|
| | Mean Value | Standard Deviation |
| Blank | 0.678 | 0.028 |
| Control oligonucleotide (SEQ ID NO: 14) | 1.081 | 0.050 |
| Antisense oligonucleotide (SEQ ID NO: 13) | 1.351 | 0.058 |

### REFERENCE EXAMPLE 17

### Apoptosis Induction Using Rabbit Peptide Antibodies

Human non-small lung cancer cell line NCI-H2228 was treated with rabbit peptide antibodies AS-2531 and AS-2532 acquired in REFERENCE EXAMPLE 8 and the apoptosis induction activities of these rabbit peptide antibodies were assayed.

NCI-H2228 was suspended in RPMI-1640 medium (Invitrogen) containing 10% fetal calf serum (JRH), 1 mM sodium pyruvate and 25 mM HEPES. The suspension was plated on a 96-well flat bottom tissue culture plate (BD Falcon) coated with type I collagen to reach a cell density of 4 x 10³/well, followed by incubation overnight at 37°C in a 5% carbon dioxide gas. The rabbit peptide antibodies AS-2531 and AS-2532 acquired in REFERENCE EXAMPLE 8 and non-immunized rabbit IgG (Jackson) were diluted in PBS. The suspensions were added to the media, whereby the final concentrations of the antibodies reached 15 µg/ml, 45 µg/ml and 150 µg/ml, respectively. After incubation was continued for further 5 days, the rabbit peptide antibodies described above were assayed for their apoptosis induction activities, following the protocol attached to Cell Death Detection ELISA^{PLUS} (Roche Diagnostics).

As a result, the antibodies showed the apoptosis induction activity in the presence of 45 µg/ml and 15 µg/ml of AS-2531 as higher by 1.26 times and 1.31 times, respectively, than the non-immunized rabbit IgG of the same concentration (P≦0.05 and P≦0.01). Also, in the presence of 150 µg/ml of AS-2532, the antibodies showed the apoptosis induction activity as higher by 1.27 times than the non-immunized rabbit IgG of the same concentration (P≦0.01).

As such, it became clear that the SEMA4B protein, SEMA4B-M1 protein, SEMA4B-M2 protein and SEMA4B-M3 protein play an important role in sustaining the survival of human lung cancer cells.

### EXAMPLE 1

The following procedures were carried out by a modification of the yeast two-hybrid system publicly known (The Yeast Two-Hybrid System, Oxford University Press, Bartel and Fields, 1997, etc.).

The cDNA encoding the bait protein consisting of the amino acid sequence represented by SEQ ID NO: 26 was amplified by PCR from the plasmid pCMV-14-SEMA4B acquired in REFERENCE EXAMPLE 6 above. The resulting cDNA was then introduced through recombination into the yeast expression vector pGBT.Q, which is a close derivative of pGBT.C (Nat Genet., 12, 72-77 (1996)) in which the polylinker site has been modified to include the M13 primer for sequencing. The new construct was selected directly in the yeast strain PNY200 for its ability to synthesize tryptophan (genotype of this strain: MATα trpl-901 leu2-3, 112 ura3-52 his3-200 ade2 gal4Δ gal80Δ). In these yeast cells, the bait protein was produced as a fusion protein in which it is fused to the C-terminal of the DNA binding domain (the 1^{st} to 147^{th} amino acids) of the transcription factor Gal4 protein (GenBank NP_015076). Prey libraries were transformed into the yeast strain BK100 (genotype of this strain: MATa trp1-901 leu2-3,112 ura3-52 his3-200 gal4Δ gal80Δ LYS2::GAL-HIS3 GAL2-ADE2 met2::GAL7-lacZ), and selected for the presence or absence of the ability to synthesize leucine. In these yeast cells, the protein encoded by each cDNA was produced as a fusion protein in which it is fused to the C-terminal of the transcription activation domain (the 768^{th} to 881^{st} amino acids) of the transcription factor Ga14 protein (GenBank NP_015076) Then, PNY200 cells (MATα mating type) expressing the bait protein, were mated with BK100 cells (MATa mating type) expressing the prey protein from the prey libraries. The resulting diploid yeast cells expressing the prey protein interacting with the bait protein were selected for the presence or absence of the ability to synthesize tryptophan, leucine, histidine and adenine. DNA was prepared from each clone, transformed by electroporation into *Escherichia coli* strain KC8, and the cells were selected on ampicillin-containing media in the absence of either tryptophan (selection for the bait plasmid) or leucine (selection for the plasmid in the prey libraries). DNA for both plasmids was prepared and subjected to sequencing by the dideoxynucleotide chain termination method. The identity of the bait cDNA insert was confirmed and the cDNA insert obtained from plasmid in the prey libraries was identified using the BLAST program to search against the nucleotide and protein databases publicly known.

The results are described below.

The cDNA fragment encoding the 142-310 amino acid sequence of DLG1 (SEQ ID NO: 27) was acquired from mixed cDNA libraries of the human breast cancer cell line and the human prostate cancer cell line.

The cDNA fragment encoding the 81-353 amino acid sequence ofDLG3 (SEQ ID NO: 29) was acquired from a human brain-derived cDNA library.

### INDUSTRIAL APPLICABILITY

The substance that inhibits the binding of (a) a protein comprising the same or substantially the same amino acid sequence as the amino acid sequence represented by SEQ ID NO: 26, a partial peptide thereof, or a salt thereof and (b) a protein comprising the same or substantially the same amino acid sequence as the amino acid sequence represented by SEQ ID NO: 27 or/and SEQ ID NO: 29, a partial peptide thereof, or a salt thereof is useful as an agent for the prevention/treatment of, for example, cancer (e.g., colorectal cancer, breast cancer, lung cancer, prostate cancer, esophageal cancer, gastric cancer, liver cancer, biliary tract cancer, spleen cancer, renal cancer, bladder cancer, uterine cancer, testicular cancer, ovary cancer, thyroid cancer, pancreatic cancer, brain tumor, blood tumor, etc.) or the like. The substance that promotes said binding is useful as an agent for the prevention/treatment of, for example, neurodegenerative diseases (e.g., Alzheimer's diseases (familial Alzheimer's disease, juvenile Alzheimer's disease, sporadic Alzheimer's disease, etc.), etc.) or the like.

The protein comprising the same or substantially the same amino acid sequence as the amino acid sequence represented by SEQ ID NO: 27 or SEQ ID NO: 29, a partial peptide thereof, or a salt thereof is useful for screening an agent for the prevention/treatment of cancer, neurodegenerative diseases, etc.

## Claims

1. A complex comprising:
(a) a protein comprising the amino acid sequence of SEQ ID NO: 26, or a salt thereof, and
(b) (i) a protein comprising the amino acid sequence of SEQ ID NO: 27 or SEQ ID NO: 29, or (ii) a protein comprising an amino acid sequence having at least 95% homology to the amino acid sequence of SEQ ID NO: 27 or SEQ ID NO: 29 and which binds to the protein defined in (a) above, or a salt thereof.

2. The complex according to claim 1, wherein the protein comprising the amino acid sequence of SEQ ID NO: 26 is a protein comprising the amino acid sequence of SEQ ID NO: 1, SEQ ID NO: 4, SEQ ID NO: 7 or SEQ ID NO: 10.

3. An antibody which is capable of specifically recognizing the complex according to claim 1.

4. A medicament comprising the antibody according to claim 3.

5. A diagnostic agent comprising the antibody according to claim 3.

6. The diagnostic agent according to claim 5, which is a diagnostic agent for cancer or neurodegenerative disease.

7. A method of screening a compound or its salt that inhibits or promotes the binding of the protein according to claim 1(a) to the protein according to claim 1(b) wherein said method comprises using the protein defined in claim 1(a) and the protein defined in claim 1(b).

8. A kit for screening a compound or its salt that inhibits or promotes the binding of the protein according to claim 1(a) to the protein according to claim 1(b) wherein said kit comprises the protein defined in claim 1(a) and the protein defined in claim 1(b).

9. A method of screening a compound or its salt that promotes or inhibits the dissociation of a complex according to claim 1, wherein said method comprises using the protein defined in claim 1(a) and the protein defined in claim 1(b).

10. A kit for screening a compound or its salt that promotes or inhibits the dissociation of a complex according to claim 1, wherein said kit comprises the protein defined in claim 1(a) and the protein defined in claim 1(b).

11. A method of screening a compound or its salt having a preventive/therapeutic effect on cancer or neurodegenerative disease, which comprises using the protein according to claim 1(a) and the protein according to claim 1(b).

## Patentansprüche

1. Komplex, umfassend:
(a) ein Protein, umfassend die Aminosäuresequenz von SEQ ID NR.: 26, oder ein Salz davon und
(b) (i) ein Protein, umfassend die Aminosäuresequenz von SEQ ID NR.: 27 oder SEQ ID NR.: 29, oder (ii) ein Protein, umfassend eine Aminosäuresequenz, die mindestens 95%ige Homologie zu der Aminosäuresequenz von SEQ ID NR.: 27 oder SEQ ID NR.: 29 aufweist und die an das in (a) oben definierte Protein oder ein Salz davon bindet.

2. Komplex nach Anspruch 1, wobei das Protein, umfassend die Aminosäuresequenz von SEQ ID NR.: 26, ein Protein ist, welches die Aminosäuresequenz von SEQ ID NR.: 1, SEQ ID NR.: 4, SEQ ID NR.: 7 oder SEQ ID NR.: 10 umfasst.

3. Antikörper, der spezifisch den Komplex nach Anspruch 1 erkennt.

4. Medikament, umfassend den Antikörper nach Anspruch 3.

5. Diagnostisches Mittel, umfassend den Antikörper nach Anspruch 3.

6. Diagnostisches Mittel nach Anspruch 5, welches ein diagnostisches Mittel für Krebs oder eine neurodegenerative Erkrankung ist.

7. Verfahren zum Screenen einer Verbindung oder ihres Salzes, welche(s) das Binden des Proteins nach Anspruch 1(a) an das Protein nach Anspruch 1(b) inhibiert oder fördert, wobei das Verfahren die Verwendung des in Anspruch 1(a) definierten Proteins und des in Anspruch 1(b) definierten Proteins umfasst.

8. Kit zum Screenen einer Verbindung oder ihres Salzes, welche(s) das Binden des Proteins nach Anspruch 1(a) an das Protein nach Anspruch 1(b) inhibiert oder fördert, wobei das Kit das in Anspruch 1(a) definierte Protein und das in Anspruch 1(b) definierte Protein umfasst.

9. Verfahren zum Screenen einer Verbindung oder ihres Salzes, welche(s) die Dissoziation eines Komplexes nach Anspruch 1 inhibiert oder fördert, wobei das Verfahren die Verwendung des in Anspruch 1 (a) definieren Proteins und des in Anspruch 1(b) definierten Proteins umfasst.

10. Kit zum Screenen einer Verbindung oder ihres Salzes, welche(s) die Dissoziation eines Komplexes nach Anspruch 1 inhibiert oder fördert, wobei das Kit das in Anspruch 1(a) definierte Protein und das in Anspruch 1 (b) definierte Protein umfasst.

11. Verfahren zum Screenen einer Verbindung oder ihres Salzes mit einer präventiven/therapeutischen Wirkung gegen Krebs oder eine neurodegenerative Erkrankung, wobei das Verfahren die Verwendung des in Anspruch 1(a) definierten Proteins und des in Anspruch 1(b) definierten Proteins umfasst.

## Revendications

1. Complexe comprenant :
(a) une protéine comprenant la séquence d'acides aminés de SEQ ID NO : 26, ou un sel de celle-ci, et
(b) (i) une protéine comprenant la séquence d'acides aminés de SEQ ID NO : 27 ou SEQ ID NO : 29, ou (ii) une protéine comprenant une séquence d'acides aminés présentant une homologie d'au moins 95 % avec la séquence d'acides aminés de SEQ ID NO : 27 ou SEQ ID NO : 29 et qui se lie à la protéine définie dans le point (a) ci-dessus, ou un sel de celle-ci.

2. Complexe selon la revendication 1, dans lequel la protéine comprenant la séquence d'acides aminés de SEQ ID NO : 26 est une protéine comprenant la séquence d' acides aminés de SEQ ID NO : 1, SEQ ID NO : 4, SEQ ID NO : 7 ou SEQ ID NO : 10.

3. Anticorps qui est capable de spécifiquement reconnaître le complexe selon la revendication 1.

4. Médicament comprenant l'anticorps selon la revendication 3.

5. Agent de diagnostic comprenant l'anticorps selon la revendication 3.

6. Agent de diagnostic selon la revendication 5, qui est un agent de diagnostic pour le cancer ou une maladie neurodégénérative.

7. Procédé de criblage d'un composé ou de son sel qui inhibe ou promeut la liaison de la protéine selon la revendication 1(a) à la protéine selon la revendication 1(b), ledit procédé comprenant l'utilisation de la protéine définie dans la revendication 1(a) et de la protéine définie dans la revendication 1(b).

8. Kit de criblage d'un composé ou de son sel qui inhibe ou promeut la liaison de la protéine selon la revendication 1(a) à la protéine selon la revendication 1(b), ledit kit comprenant la protéine définie dans la revendication 1(a) et la protéine définie dans la revendication 1(b).

9. Procédé de criblage d'un composé ou de son sel qui promeut ou inhibe la dissociation d'un complexe selon la revendication 1, ledit procédé comprenant l'utilisation de la protéine définie dans la revendication 1(a) et de la protéine définie dans la revendication 1(b).

10. Kit de criblage d'un composé ou de son sel qui promeut ou inhibe la dissociation d'un complexe selon la revendication 1, ledit kit comprenant la protéine définie dans la revendication 1(a) et la protéine définie dans la revendication 1(b).

11. Procédé de criblage d'un composé ou de son sel ayant un effet préventif/thérapeutique sur le cancer ou une maladie neurodégénérative, qui comprend l'utilisation de la protéine selon la revendication 1(a) et de la protéine selon la revendication 1(b).
